(19) Europäisches Patentamt
European Patent Office
Office européen des brevets

(11) EP 2 257 545 B1

(12) # EUROPEAN PATENT SPECIFICATION

(45) Date of publication and mention of the grant of the patent:
**31.08.2011 Bulletin 2011/35**

(21) Application number: **09722963.7**

(22) Date of filing: **10.03.2009**

(51) Int Cl.:
***C07D 401/14*** (2006.01) ***C07D 403/04*** (2006.01)
***C07D 409/04*** (2006.01) ***C07D 471/04*** (2006.01)
***A61K 31/404*** (2006.01) ***A61P 25/24*** (2006.01)

(86) International application number:
**PCT/EP2009/052748**

(87) International publication number:
**WO 2009/115427 (24.09.2009 Gazette 2009/39)**

(54) **PYRROLIDINYL DERIVATIVES AND USES THEREOF**

PYRROLIDINYLDERIVATE UND ANWENDUNGEN DAVON

DERIVES DE PYRROLIDINYLE ET LEURS UTILISATIONS

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **20.03.2008 US 38160 P**

(43) Date of publication of application:
**08.12.2010 Bulletin 2010/49**

(73) Proprietor: **F. Hoffmann-La Roche AG**
**4070 Basel (CH)**

(72) Inventors:
- **LUCAS, Matthew C.**
**Sunnyvale**
**California 94089 (US)**
- **SCHOENFELD, Ryan Craig**
**San Jose**
**California 95117 (US)**
- **WEIKERT, Robert James**
**Boulder Creek**
**California 95006 (US)**

(74) Representative: **Küng, Peter**
**F. Hoffmann-La Roche AG**
**Patent Department**
**Grenzacherstrasse 124**
**4070 Basel (CH)**

(56) References cited:
**EP-A- 1 304 324** **WO-A-2006/121218**
**WO-A-2008/074703**

- **BEER B ET AL: "DOV 216,303, A TRIPLE REUPTAKE INHIBITOR: SAFETY, TOLERABILITY, AND PHARMACOKINETIC PROFILE" JOURNAL OF CLINICAL PHARMACOLOGY, LIPPINCOTT CO, HAGERSTOWN, MD, US, vol. 44, no. 12, 1 January 2004 (2004-01-01), pages 1360-1367, XP008055430 ISSN: 0091-2700**
- **BANNWART L M ET AL: "Novel 3,3-disubstituted pyrrolidines as selective triple serotonin/ norepinephrine/dopamine reuptake inhibitors" BIOORGANIC & MEDICINAL CHEMISTRY LETTERS, PERGAMON, ELSEVIER SCIENCE, GB, vol. 18, no. 23, 1 December 2008 (2008-12-01), pages 6062-6066, XP025646275 ISSN: 0960-894X [retrieved on 2008-10-11]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention pertains to pyrrolidinyl compounds and methods for using the same. In particular, compounds of the present invention are useful for treatment of diseases associated with monoamine reuptake inhibitors.

Monoamine deficiency has been long been linked to depressive, anxiolytic and other disorders (see, e.g.: Charney et al., J. Clin. Psychiatry (1998) 59, 1-14; Delgado et al., J. Clin. Psychiatry (2000) 67, 7-11; Resser et al., Depress. Anxiety (2000) 12 (Suppl 1) 2-19; and Hirschfeld et al., J. Clin. Psychiatry (2000) 61, 4-6). In particular, serotonin (5-hydroxytryptamine) and norepinephrine are recognized as key modulatory neurotransmitters that play an important role in mood regulation. Selective serotonin reuptake inhibitors (SSRIs) such as fluoxetine, sertraline, paroxetine, fluvoxamine, citalopram and escitalopram have provided treatments for depressive disorders (Mas and et al., Harv. Rev. Psychiatry (1999) 7, 69-84). Noradrenaline or norepinephrine reuptake inhibitors such as reboxetine, atomoxetine, desipramine and nortryptyline have provided effective treatments for depressive, attention deficit and hyperactivity disorders (Scates et al., Ann. Pharmacother. (2000) 34, 1302-1312; Tatsumi et al., Eur-. J. Pharmacol. (1997) 340, 249-258).

Enhancement of serotonin and norepinephrine neurotransmission is recognized to be synergistic in the pharmacotherapy of depressive and anxiolytic disorders, in comparison with enhancement of only serotonin or norepinephrine neurotransmission alone (Thase et al., Br. J. Psychiatry (2001) 178, 234, 241; Tran et al., J. Clin. Psychopharmacology (2003) 23, 78-86). Dual reuptake inhibitors of both serotonin and norepinephrine, such as duloxetine, milnacipran and venlafaxine are currently marketed for treatment of depressive and anxiolytic disorders (Mallinckrodt et al., J. Clin. Psychiatry (2003) 5(1) 19-28; Bymaster et al., Expert Opin. Investig. Drugs (2003) 12(4) 531-543). Dual reuptake inhibitors of serotonin and norepinephrine also offer potential treatments for schizophrenia and other psychoses, dyskinesias, drug addition, cognitive disorders, Alzheimer's disease, obsessive-compulsive behaviour, attention deficit disorders, panic attacks, social phobias, eating disorders such as obesity, anorexia, bulimia and "binge-eating", stress, hyperglycaemia, hyperlipidemia, non-insulin-dependent diabetes, seizure disorders such as epilepsy, and treatment of conditions associated with neurological damage resulting from stroke, brain trauma, cerebral ischaemia, head injury and hemorrhage. Dual reuptake inhibitors of serotonin and norepinephrine also offer potential treatments for disorders and disease states of the urinary tract, and for pain and inflammation.

More recently, "triple reuptake" inhibitors *("broad-spectrum* antidepressants") which inhibit the reuptake of norepinephrine, serotonin, and dopamine, have been recognized as useful for the treatment of depression and other CNS indications (Beer et al., J. Clinical Pharmacology (2004) 44:1360-1367; Skolnick et al., Eur J Pharmacol. (2003) Feb 14;461(2-3):99-104).

Monamine reuptake inhibitors also have use in pain treatment. Serotonin has been found to have a role in pain processing in the peripheral nervous system and to contribute to peripheral sensitization and hyperalgesia in inflammation and nerve injury (Sommer et al., Molecular Neurobiology (2004) 30(2), 117-125). The serotonin-norepinephrine reuptake inhibitor duloxetine has been shown effective in treatment of pain in animal models (Iyengar et al., J. Pharm. Exper. Ther-apeutics (2004), 311, 576-584).

Monoamine rempstake inhibitors are disclosed also in WO 2006/121218 and EP1304324.

There is accordingly a need for compounds that are effective as serotonin reuptake inhibitors, norepinephrine reuptake inhibitors, dopamine reuptake inhibitors, and/or dual reuptake inhibitors of serotonin, norepinephrine and/or dopamine, or triple reuptake inhibitors of norepinephrine, serotonin, and dopamine, as well as methods of making and using such compounds in the treatment of depressive, anxiolytic, genitourinary, pain, and other disorders. The present invention satisfies these needs.

The application provides a compound of Formula **I**:

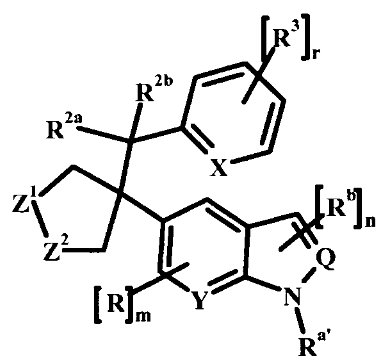

**I**

or an enantiomer, diastereomer, or pharmaceutically acceptable salt thereof, wherein:

either $Z^1$ or $Z^2$ is N($R^a$) and the other is $CH_2$;
X is CH or N;
Y is CH or N;
m is 0 or 1;
R is hydroxy, halogen, lower alkyl, or lower alkoxy;
Q is CH, C($R^b$), or N;
$R^a$ is H, lower alkyl, or benzyl;
$R^{a'}$ is H, lower alkyl, cycloalkyl alkyl, -S(=O)$_2R^c$, -C(=O)N($R^C$)$_2$, or S(=O)$_2$N($R^c$)$_2$;
n is 0 or 1;
each $R^b$ is independently $R^{b'}$ or $R^{b''}$ ;
$R^{b''}$ is hydroxy, halogen, -C(=O)($R^c$), -C(=O)O($R^c$), -OC(=O)($R^c$), - N($R^c$)$_2$, -C(=O)N($R^C$)$_2$, -NHC(=O)($R^c$), -CN, -S(=O)$_2R^c$, or- S(=O)$_2$($R^c$)$_2$;
$R^{b''}$ is lower alkyl, lower alkenyl, lower alkynyl, lower alkoxy, lower haloalkyl, phenyl, cycloalkyl, cycloalkyl alkyl, heterocycloalkyl, heterocycloalkyl alkyl, optionally substituted with one or more $R^c$;
each $R^C$ is independently $R^d$ or $R^e$;
$R^d$ is H;
$R^e$ is lower alkyl, lower haloalkyl, cycloalkyl, phenyl, heterocycloalkyl, or heteroaryl, optionally substituted with one or more $R^{e'}$;
each $R^{e'}$ is independently hydroxy, halogen, amino, lower alkyl, lower alkenyl, lower alkynyl, lower alkoxy, lower haloalkyl, or -CN;
$R^{2a}$ and $R^{2b}$ are each independently H, hydroxy, lower alkyl, lower haloalkyl, or lower alkoxy;
r is 0, 1, or 2; and
$R^3$ is halogen, hydroxy, lower alkyl, lower haloalkyl, or lower alkoxy.

In one embodiment, $Z^1$ is N($R^a$) and $Z^2$ is $CH_2$.
In another embodiment, $Z^1$ is $CH_2$ and $Z^2$ is N($R^a$).
In one embodiment, $Z^1$ is N($R^a$) $Z^2$ is $CH_2$, $R^a$, $R^{2a}$, and $R^{2b}$ are H, m is 0, r is 0, X is CH, Y is CH, $R^a$ is H, and n is 1.
In another embodiment, $Z^1$ is $CH_2$, $Z^2$ is N($R^a$), $R^a$, $R^{2a}$, and $R^{2b}$ are H, m is 0, r is 0, X is CH, Y is CH, $R^{a'}$ is H, and n is 1.
In one variation of the above embodiments, Q is C($R^b$).
In one variation of the above embodiment, $R^b$ is $R^{b'}$.
In another variation of the above embodiment, $R^b$ is $R^{b''}$.
In certain embodiments, Q is C($R^b$) , $R^b$ is $R^{b'}$, and $R^{b'}$ is -CN or halogen.
In certain embodiments, Q is C($R^b$), $R^b$ is $R^{b'}$, and $R^{b'}$ is -C(=O)N($R^{2c}$)$_2$ or -NHC(=O)($R^{2c}$).
In certain embodiments, Q is C($R^b$), $R^b$ is $R^{b'}$, and R' is -S(=O)$_2R^{2c}$ or-S(=O)$_2$N($R^{2c}$)$_2$.
In certain embodiments, Q is C($R^b$), $R^b$ is $R^{b'}$, and $R^b$ is -C(=O)($R^{2c}$) or -C(=O)O($R^{2c}$).
In certain embodiments, Q is C($R^b$), $R^b$ is $R^{b''}$ , and $R^{b''}$ is lower alkyl or cycloalkyl alkyl.
The application provides a compound of Formula II:

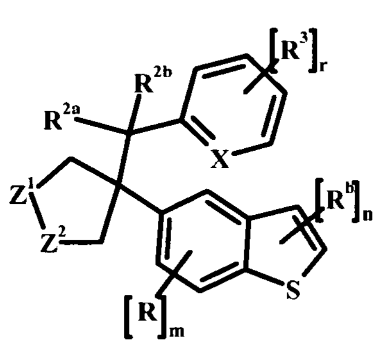

**II**

or an enantiomer, diastereomer, or pharmaceutically acceptable salt thereof, wherein:

either $Z^1$ or $Z^2$ is N($R^a$) and the other is $CH_2$;

| | |
|---|---|
| X is | CH or N; |
| m is | 0 or 1; |
| R is | hydroxy, halogen, lower alkyl, or lower alkoxy; |
| $R^a$ is | H, lower alkyl, or benzyl; |
| n is | 0 or 1; |
| each $R^b$ is | independently $R^{b'}$ or $R^{b''}$; |
| $R^{b'}$ is | hydroxy, halogen, -C(=O)($R^c$), -C(=O)O($R^c$), -OC(=O)($R^c$), -N($R^c$)$_2$, -C(=O)N($R^c$)$_2$, -NHC(=O)($R^c$), -CN, -S(=O)$_2$$R^c$, or -S(=O)$_2$N($R^c$)$_2$; |
| Rb" is | lower alkyl, lower alkenyl, lower alkynyl, lower alkoxy, lower haloalkyl, phenyl, cycloalkyl, cycloalkyl alkyl, heterocycloalkyl, heterocycloalkyl alkyl, optionally substituted with one or more $R^c$; |
| each $R^c$ is | independently $R^d$ or $R^e$; |
| $R^d$ a is | H; |
| $R^e$ is | lower alkyl, lower haloalkyl, cycloalkyl, phenyl, heterocycloalkyl, or heteroaryl, optionally substituted with one or more $R^{e'}$; |
| each $R^{e'}$ is | independently hydroxy, halogen, amino, lower alkyl, lower alkenyl, lower alkynyl, lower alkoxy, lower haloalkyl, or -CN; |
| $R^{2a}$ and $R^{2b}$ are | each independently H, hydroxy, lower alkyl, lower haloalkyl, or lower alkoxy; |
| r is | 0, 1, or 2; and |
| $R^3$ is | halogen, hydroxy, lower alkyl, lower haloalkyl, or lower alkoxy. |

In one embodiment, $R^{2a}$ and $R^{2b}$ are H, m is 0, and r is 0.

In one variation of the above embodimenr, $Z^1$ is $CH_2$, $Z^2$ is N($R^a$), $R^a$ is H, and n is 1.

The application provides a compound of Formula III:

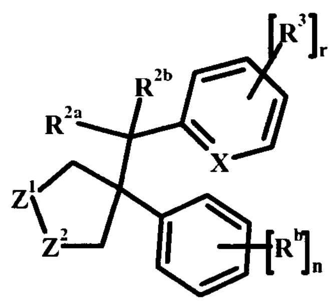

III

or an enantiomer, diastereomer, or pharmaceutically acceptable salt thereof, wherein:

| | |
|---|---|
| either $Z^1$ or $Z^2$ is | N($R^a$) and the other is $CH_2$; |
| X is | CH or N; |
| $R^a$ is | H, lower alkyl, or benzyl; |
| n is | 0, 1, or 2; |
| each $R^b$ is | independently $R^{b'}$ or $R^{b''}$; |
| $R^{b'}$ is | independently hydroxy, halogen, -C(=O)($R^c$), -C(=O)O($R^c$), -OC(=O)($R^c$), -N($R^c$)$_2$, -C(=O)N($R^c$)$_2$, -NHC(=O)($R^c$), -CN,- S(=O)$_2$$R^c$, or -S(=O)$_2$N($R^c$)$_2$; |
| $R^{b''}$ is | lower alkyl, lower alkenyl, lower alkynyl, lower alkoxy, lower haloalkyl, phenyl, cycloalkyl, cycloalkyl alkyl, heterocycloalkyl, heterocycloalkyl alkyl, optionally substituted with one or more $R^c$; |
| each $R^c$ is | independently $R^d$ or $R^e$; |
| $R^d$ is | H; |
| $R^e$ is | lower alkyl, lower haloalkyl, cycloalkyl, phenyl, heterocycloalkyl, or heteroaryl, optionally substituted with one or more $R^{e'}$; and |
| each $R^{e'}$ is | independently hydroxy, halogen, amino, lower alkyl, lower alkenyl, lower alkynyl, lower alkoxy, lower haloalkyl, or -CN; |
| $R^{2a}$ and $R^{2b}$ are | each independently H, hydroxy, lower alkyl, lower haloalkyl, or lower alkoxy; |
| r is | 0, 1, or 2; |
| $R^3$ is | halogen, hydroxy, lower alkyl, lower haloalkyl, or lower alkoxy; |

with the proviso that when $Z^1$ is $N(R^a)$, $Z^2$ is $CH_2$, $R^a$ is H, and X is CH, then n is not 0; and with the proviso that when $Z^1$ is $N(R^a)$, $Z^2$ is $CH_2$, $R^a$ is ethyl, X is CH, and either $R^{2a}$ or $R^{2b}$ is hydroxy, then n is not 0.

In one embodiment, $R^{2a}$ and $R^{2b}$ are H and r is 0.

In one variation of the above embodiment, $Z^1$ is $CH_2$, $Z^2$ is $N(R^a)$ and $R^a$ is H.

The application provides a compound of Formula IV

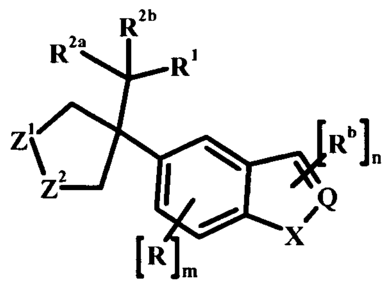

IV

or an enantiomer, diastereomer, or pharmaceutically acceptable salt thereof, wherein:

| | |
|---|---|
| either $Z^1$ or $Z^2$ is | $N(R^a)$ and the other is $CH_2$; |
| X is | S or $N(R^{a'})$; |
| m is | 0 or 1; |
| R is | hydroxy, halogen, lower alkyl, or lower alkoxy; |
| Q is | CH, $C(R^b)$, or N; |
| $R^a$ is | H, lower alkyl, or benzyl; |
| $R^{a'}$ is | H, lower alkyl, cycloalkyl alkyl, $-S(=O)_2R^c$, $-C(=O)N(R^C)_2$, or $S(=O)_2N(R^c)2$; |
| $R^1$ is | $R^{1a}$ or $R^{1b}$; |
| $R^{1a}$ is | H; |
| $R^{1b}$ is | lower alkyl, lower alkenyl, lower alkynyl, lower alkoxy, or lower haloalkyl, optionally substituted with one or more $R^{1b'}$; |
| each $R^{1b}$ is | independently hydroxy, halogen, amino, lower alkyl, lower alkoxy, lower haloalkyl, or -CN; |
| n is | 0 or 1; |
| each $R^b$ is | independently $R^{b'}$ or $R^{b''}$; |
| $R^{b'}$ is | hydroxy, halogen, $-C(=O)(R^c)$, $-C(=O)O(R^c)$, $-OC(=O)(R^c)$, $-N(R^C)_2$, $-C(=O)N(R^c)_2$, $-NHC(=O)(R^c)$, -CN, $-S(=O)_2R^c$, or $-S(=O)_2N(R^c)_2$; |
| $R^{b''}$ is | lower alkyl, lower alkenyl, lower alkynyl, lower alkoxy, lower haloalkyl, phenyl, cycloalkyl, cycloalkyl alkyl, heterocycloalkyl, heterocycloalkyl alkyl, optionally substituted with one or more $R^c$; |
| each $R^c$ is | independently $R^d$ or $R^e$; |
| $R^d$ is | H; |
| $R^e$ is | lower alkyl, lower haloalkyl, cycloalkyl, phenyl, heterocycloalkyl, or heteroaryl, optionally substituted with one or more $R^{e'}$; |
| each $R^{e'}$ is | independently hydroxy, halogen, amino, lower alkyl, lower alkenyl, lower alkynyl, lower alkoxy, lower haloalkyl, or -CN; and |
| $R^{2a}$ and $R^{2b}$ are | each independently H, hydroxy, lower alkyl, lower haloalkyl, or lower alkoxy. |

In one embodiment, $R^{2a}$ and $R^{2b}$ are H and m is 0.

In one variation of the above embodiment, $Z^1$ is $CH_2$, $Z^2$ is $N(R^a)$, $R^a$ is H, and n is 1.

The application provides a compound of Formula V:

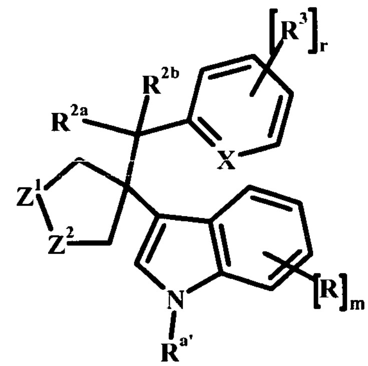

**V**

or an enantiomer, diastereomer, or pharmaceutically acceptable salt thereof, wherein:

| | |
|---|---|
| either $Z^1$ or $Z^2$ is | $N(R^a)$ and the other is $CH_2$; |
| $R^a$ is | H, lower alkyl, or benzyl; |
| X is | CH or N; |
| m is | 0 or 1; |
| R is | hydroxy, halogen, lower alkyl, or lower alkoxy; |
| $R^a$ is | H, lower alkyl, cycloalkyl alkyl, $-S(=O)_2R^c$, $-C(=O)N(R^c)_2$, or $S(=O)_2N(R^c)_2$; |
| each $R^c$ is | independently $R^d$ or $R^e$; |
| Rd is | H; |
| $R^e$ is | lower alkyl, lower haloalkyl, cycloalkyl, phenyl, heterocycloalkyl, or heteroaryl, optionally substituted with one or more $R^{e'}$; |
| each $R^e$ is | independently hydroxy, halogen, amino, lower alkyl, lower alkenyl, lower alkynyl, lower alkoxy, lower haloalkyl, or -CN; |
| $R^{2a}$ and $R^{2b}$ are | each independently H, hydroxy, lower alkyl, lower haloalkyl, or lower alkoxy; |
| r is | 0, 1, or 2; and |
| $R^3$ is | halogen, hydroxy, lower alkyl, lower haloalkyl, or lower alkoxy. |

The application provides a compound of Formula **VI**:

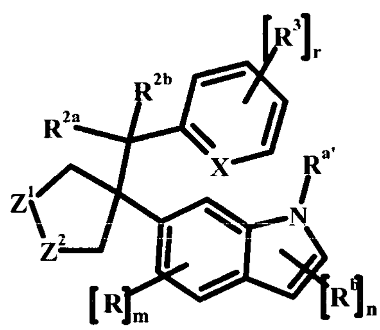

**VI**

or an enantiomer, diastereomer, or pharmaceutically acceptable salt thereof, wherein:

| | |
|---|---|
| either $Z^1$ or $Z^2$ is | $N(R^a)$ and the other is $CH_2$; |
| X is | CH or N; |
| mis | 0 or 1; |
| R is | hydroxy, halogen, lower alkyl, or lower alkoxy; |
| $R^a$ is | H, lower alkyl, or benzyl; |
| $R^{a'}$ is | H, lower alkyl, cycloalkyl alkyl, $-S(=O)_2R^c$, $-C(=O)N(R^c)_2$, or $S(=O)_2N(R^c)_2$; |
| n is | 0 or 1; |

| | |
|---|---|
| each $R^b$ is | independently $R^{b'}$ or $R^{b''}$; |
| $R^{b'}$ is | hydroxy, halogen, -C(=O)($R^c$), -C(=O)O($R^c$), -OC(=O)($R^c$), -N(R')$_2$, -C(=O)N($R^c$)$_2$, -NHC(=O)($R^c$), -CN, -S(=O)$_2$$R^c$, or -S(=O)$_2$N($R^c$)$_2$; |
| $R^{b''}$ is | lower alkyl, lower alkenyl, lower alkynyl, lower alkoxy, lower haloalkyl, phenyl, cycloalkyl, cycloalkyl alkyl, heterocycloalkyl, heterocycloalkyl alkyl, optionally substituted with one or more $R^c$; |
| each $R^c$ is | independently $R^d$ or $R^e$; |
| $R^d$ is | H; |
| $R^e$ is | lower alkyl, lower haloalkyl, cycloalkyl, phenyl, heterocycloalkyl, or heteroaryl, optionally substituted with one or more $R^{e'}$; |
| each $R^{e'}$ is | independently hydroxy, halogen, amino, lower alkyl, lower alkenyl, lower alkynyl, lower alkoxy, lower haloalkyl, or -CN; |
| $R^{2a}$ and $R^{2b}$ are | each independently H, hydroxy, lower alkyl, lower haloalkyl, or lower alkoxy; |
| r is | 0, 1, or 2; and |
| $R^3$ is | halogen, hydroxy, lower alkyl, lower haloalkyl, or lower alkoxy. |

In one aspect, the application provides a compound selected from the group consisting of:

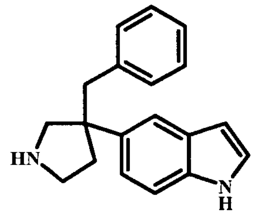
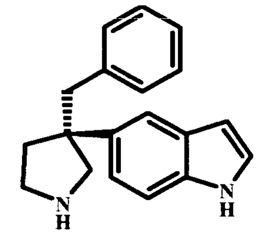
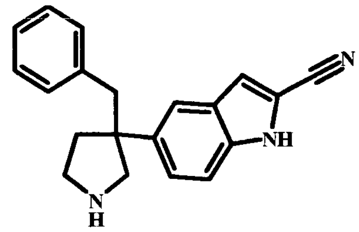
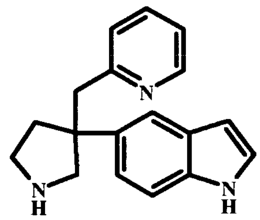
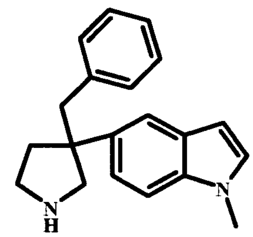
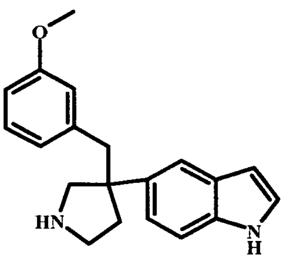
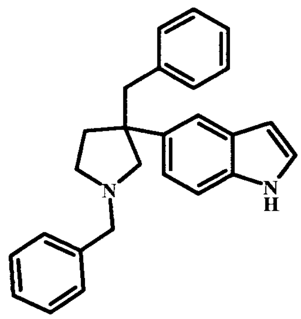
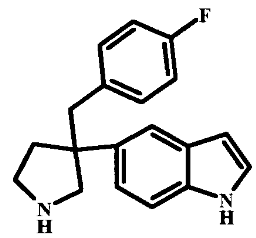
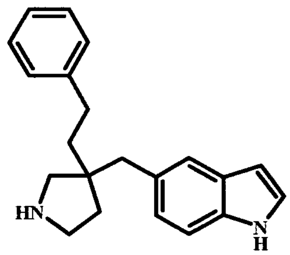
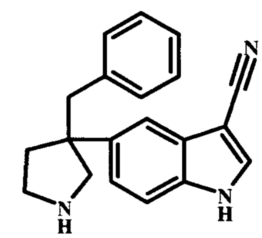
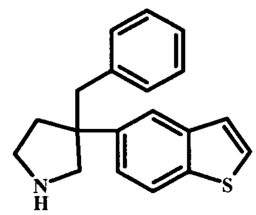
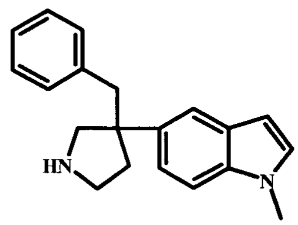
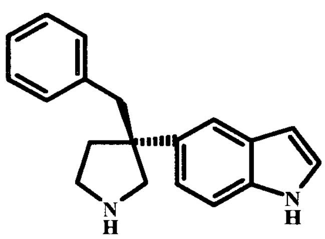
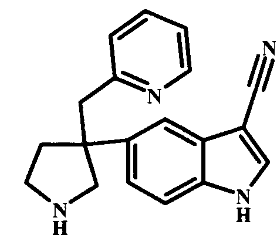
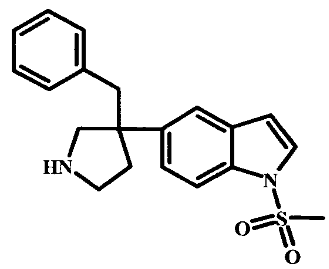
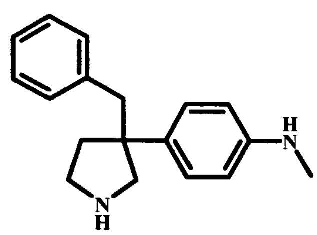

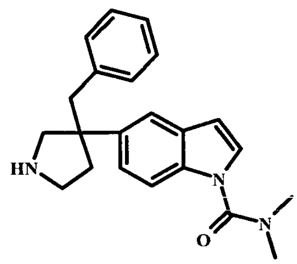

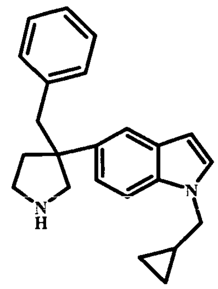

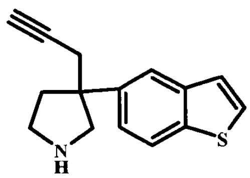

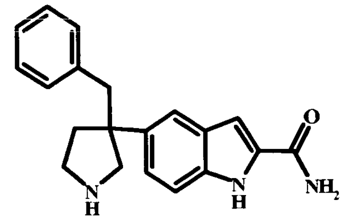

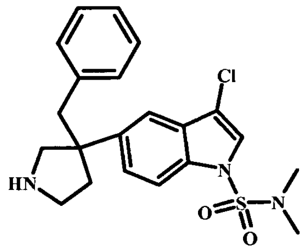

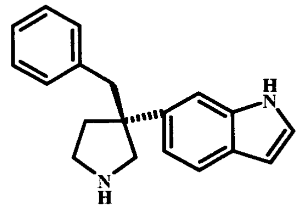

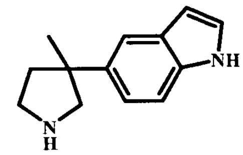

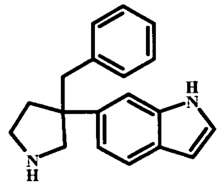

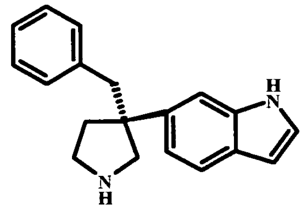

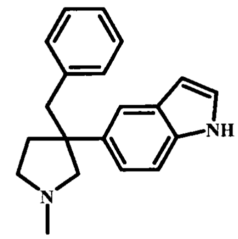

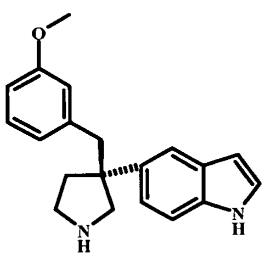

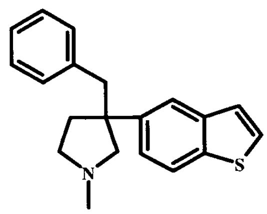

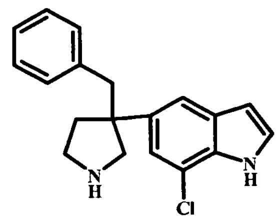

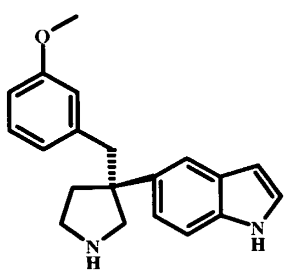

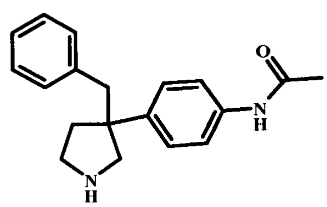

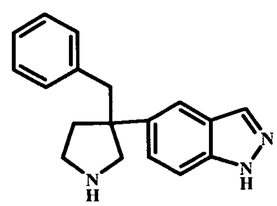

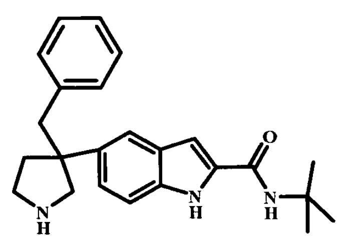

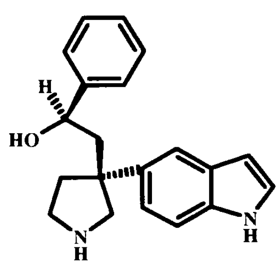

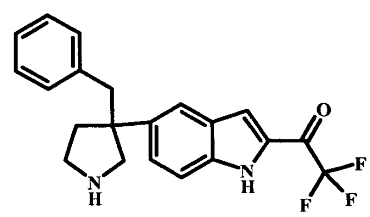

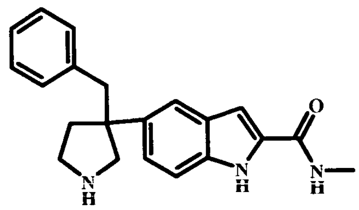
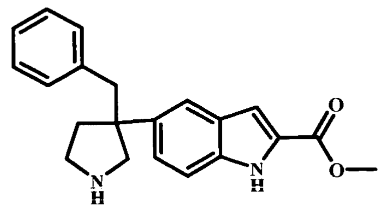
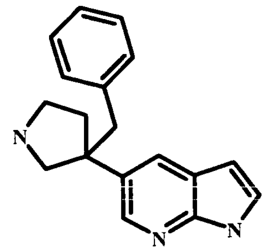
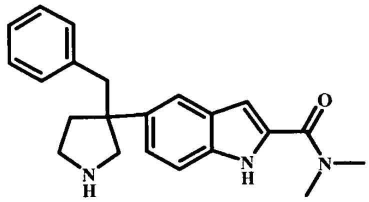
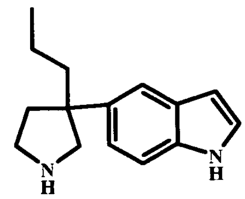
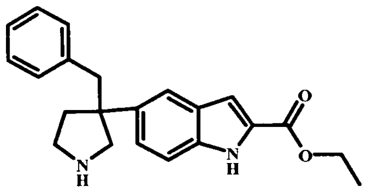
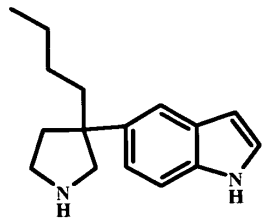

In one aspect, the application provides a pharmaceutical composition comprising any one of the compounds described herein and a pharmaceutically acceptable carrier.

In one aspect, the application provides a method for treating diseases associated with monoamine reuptake inhibitors, comprising administering to a subject in need thereof a pharmaceutically effective amount of any one of the compounds described herein.

In one aspect, the application provides a method for treating anxiety, depression, or both, said method comprising administering to a subject in need thereof a pharmaceutically effective amount of any one of the compounds described herein.

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

Agonist" refers to a compound that enhances the activity of another compound or receptor site.

"Alkyl" means the monovalent linear or branched saturated hydrocarbon moiety, consisting solely of carbon and hydrogen atoms, having from one to twelve carbon atoms.

"Lower alkyl" refers to a linear or branched alkyl group of one to six carbon atoms, i.e. $C_1$-$C_6$alkyl. Examples of alkyl groups include, but are not limited to, methyl, ethyl, propyl, isopropyl, isobutyl, sec-butyl, tert-butyl, pentyl, n-hexyl, octyl, dodecyl, and the like.

"alkylene" means a linear saturated divalent hydrocarbon radical of one to six carbon atoms or a branched saturated divalent hydrocarbon radical of three to six carbon atoms, e.g., methylene, ethylene, 2,2-dimethylethylene, propylene, 2-methylpropylene, butylene, pentylene, and the like.

"Alkoxy" means a moiety of the formula -OR, wherein R is an alkyl moiety as defined herein. Examples of alkoxy moieties include, but are not limited to, methoxy, ethoxy, isopropoxy, tert-butoxy and the like.

"Alkoxyalkyl" means a moiety of the formula -R'-R", where R' is alkylene and R" is alkoxy as defined herein. Exemplary alkoxyalkyl groups include, by way of example, 2-methoxyethyl, 3-methoxypropyl, 1-methyl-2-methoxyethyl, 1-(2-methoxyethyl)-3-methoxypropyl, and 1-(2-methoxyethyl)-3-methoxypropyl.

"Alkylcarbonyl" means a moiety of the formula -C(O)-R, where R' is alkyl as defined herein.

"Alkylsulfonyl" means a moiety of the formula $-SO_2$-R' where R' is alkyl as defined herein.

"Alkylsulfanyl" means a moiety of the formula -S-R' where R' is alkyl as defined herein.

"Alkylsulfonylalkyl" means a moiety of the formula $-R^b-S0_2-R^a$, where $R^a$ is alkyl and $R^b$ is alkylene as defined herein. Exemplary alkylsulfonylalkyl groups include, by way of example, 3-methanesulfonylpropyl, 2-methanesulfonylethyl, 2-methanesulfonylpropy, and the like.

"Alkylsulfanylalkyl" means a moiety of the formula $-R^b-S-R^a$, where $R^a$ is alkyl and $R^b$ is alkylene as defined herein.

"Alkylsulfonyloxy" means a moiety of the formula $R^a-SO_2-O-$, where $R^a$ is alkyl as defined herein.

"Amino" means a moiety of the formula -NRR' wherein R and R' each independently is hydrogen or alkyl as

defined herein. Amino thus includes "alkylamino" (where one of R and R' is alkyl and the other is hydrogen) and "dialkylamino" (where R and R' are both alkyl.

"Alkylcarbonylamino" means a group of the formula -NR-C(O)-R' wherein R is hydrogen or alkyl and R' is alkyl as defined herein.

"Antagonist" refers to a compound that diminishes or prevents the action of another compound or receptor site.

"Aryl" means a monovalent cyclic aromatic hydrocarbon moiety consisting of a mono-, bi- or tricyclic aromatic ring. The aryl group can be optionally substituted as defined herein. Examples of aryl moieties include, but are not limited to, optionally substituted phenyl, naphthyl, phenanthryl, fluorenyl, indenyl, azulenyl, oxydiphenyl, biphenyl, methylenediphenyl, aminodiphenyl, diphenylsulfidyl, diphenylsulfonyl, diphenylisopropylidenyl, benzodioxanyl, benzodioxylyl, benzoxazinyl, benzoxazinonyl, benzopiperadinyl, benzopiperazinyl, benzopyrrolidinyl, benzomorpholinyl, methylenedioxyphenyl, ethylenedioxyphenyl, and the like. Preferred aryl include optionally substituted phenyl and optionally substituted naphthyl.

"Aryloxy" means a moiety of the formula -OR, wherein R is an aryl moiety as defined herein.

"Arylalkyl" and "Aralkyl", which may be used interchangeably, mean a radical-$R^aR^b$ where $R^a$ is an alkylene group and $R^b$ is an aryl group as defmed herein; e.g., phenylalkyls such as benzyl, phenylethyl, 3-(3-chlorophenyl)-2-methylpentyl, and the like are examples of arylalkyl.

"Aralkoxy" means a moiety of the formula -OR, wherein R is an aralkyl moiety as defined herein.

"Azaindolyl" means a group of the formula

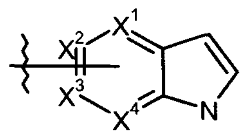

wherein one or two of any of $X^1$, $X^2$, $X^3$ and $X^4$ is N (aza), and the others are carbon. "Azaindoles" may be optionally substituted, as defined herein for heteroaryls, at position 1, 2 and 3, and at any of positions 4- through seven that are not nitrogen. "Azaindolyl" thus includes: "pyrrolopyrimidines" of the above formula wherein $X^2$ and $X^4$ are N; "pyrrolopyrimidines" of the above formula wherein $X^1$ and $X^3$ are N; "pyrollopyrazines" of the above formula wherein $X^1$ and $X^4$ are N; "pyrrolopyridines" of the above formula wherein $X^1$ is N; "pyrrolopyridines" of the above formula wherein $X^2$ is N; "pyrrolopyridines" of the above formula wherein $X^3$ is N; and "pyrrolopyridines" of the above formula wherein $X^4$ is N. One preferred azaindolyl is 7-azaindolyl ($X^1$, $X^2$, $X^3$ = C and $X^4$ = N) or pyrrolo[2,3-b]pyridinyl. Another preferred azaindole is 4-azaindolyl or pyrrolo[3,2-b]pyridinyl.

"Azaindazolyl" means a group of the formula

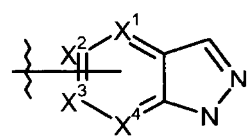

wherein one or two of any of $X^1$, $X^2$, $X^3$ and $X^4$ is N (aza), and the others are carbon. "Azaindazoles" may be optionally substituted, as defined herein for heteroaryls, at position 1, 2 and 3, and at any of positions 4- through seven that are not nitrogen. "Azaindaolyl" thus includes: "pyrazolopyrimidines" of the above formula wherein $X^2$ and $X^4$ are N; "pyrazolopyrimidines" of the above formula wherein $X^1$ and $X^3$ are N; "pyrazolopyrazines" of the above formula wherein $X^1$ and $X^4$ are N; "pyrazolopyridines" of the above formula wherein $X^1$ is N; "pyrazolopyridines" of the above formula wherein $X^2$ is N; "pyrazolopyridines" of the above formula wherein $X^3$ is N; and "pyrazolopyridines" of the above formula wherein $X^4$ is N.

"Cyanoalkyl" means a moiety of the formula -R'-R", where R' is alkylene as defined herein and R" is cyano or nitrile.

"Cycloalkyl" means a monovalent saturated carbocyclic moiety consisting of mono- or bicyclic rings. Cycloalkyl can optionally be substituted with one or more substituents, wherein each substituent is independently hydroxy, alkyl, alkoxy, halo, haloalkyl, amino, monoalkylamino, or dialkylamino, unless otherwise specifically indicated. Examples of cycloalkyl moieties include, but are not limited to, cyclopropyl, cyclobutyl, cyclopentyl, cyclohexyl, cycloheptyl, and the like, including partially unsaturated derivatives thereof.

"Cycloalkyloxy" and "cycloalkoxy", which may be used interchangeably, mean a group of the formula -OR

wherein R is cycloalkyl as defined herein. Exemplary cycloalkyloxy include cyclopropyloxy, cyclobutyloxy, cyclopentyloxy, cyclohexyloxy and the like.

"Cycloalkylalkyl" or "cycloalkyl alkyl"means a moiety of the formula -R'-R", where R' is alkylene and R" is cycloalkyl as defined herein.

"Alkylcycloalkylalkyl" means a moiety of the formula

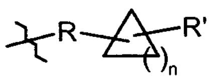

wherein n is from 1 to 4, R is alkylene and R' is alkyl as defined herein. Exemplary alkylcycloalkylalkyl include 2-(1-methyl-cyclopropyl)-ethyl and 3-(1-methyl-cyclopropyl)-methyl and the like.

"Cycloalkylalkyloxy" and "cycloalkylalkoxy", which may be used interchangeably, mean a group of the formula -OR wherein R is cycloalkylalkyl as defined herein. Exemplary cycloalkyloxy include cyclopropylmethoxy, cyclobutylmethoxy, cyclopentylmethoxy, cyclohexylmethoxy and the like.

"Heteroalkyl" means an alkyl radical as defined herein, including a branched $C_4$-$C_7$-alkyl, wherein one, two or three hydrogen atoms have been replaced with a substituent independently selected from the group consisting of $-OR^a$, $-NR^bR^c$, and $-S(O)_nR^d$ (where n is an integer from 0 to 2), with the understanding that the point of attachment of the heteroalkyl radical is through a carbon atom, wherein $R^a$ is hydrogen, acyl, alkyl, cycloalkyl, or cycloalkylalkyl; $R^b$ and $R^c$ are independently of each other hydrogen, acyl, alkyl, cycloalkyl, or cycloalkylalkyl; and when n is 0, $R^d$ is hydrogen, alkyl, cycloalkyl, or cycloalkylalkyl, and when n is 1 or 2, $R^d$ is alkyl, cycloalkyl, cycloalkylalkyl, amino, acylamino, monoalkylamino, or dialkylamino. Representative examples include, but are not limited to, 2-hydroxyethyl, 3-hydroxypropyl, 2-hydroxy-1-hydroxymethylethyl, 2,3-dihydroxypropyl, 1-hydroxymethylethyl, 3-hydroxybutyl, 2,3-dihydroxybutyl, 2-hydroxy-1-methylpropyl, 2-aminoethyl, 3-aminopropyl, 2-methylsulfonylethyl, aminosulfonylmethyl, aminosulfonylethyl, aminosulfonylpropyl, methylaminosulfonylmethyl, methylaminosulfonylethyl, methylaminosulfonylpropyl, and the like.

"Heteroaryl" means a monocyclic, bicyclic or tricyclic radical of 5 to 12 ring atoms having at least one aromatic ring containing one, two, or three ring heteroatoms selected from N, O, or S, the remaining ring atoms being C, with the understanding that the attachment point of the heteroaryl radical will be on an aromatic ring. The heteroaryl ring may be optionally substituted as defmed herein. Examples of heteroaryl moieties include, but are not limited to, optionally substituted imidazolyl, oxazolyl, isoxazolyl, thiazolyl, isothiazolyl, oxadiazolyl, thiadiazolyl, pyrazinyl, pyridazinyl, thiophenyl, furanyl, pyranyl, pyridinyl, pyrrolyl, pyrazolyl, pyrimidyl, quinolinyl, isoquinolinyl, quinazolinyl, benzofuranyl, benzothiophenyl, benzothiopyranyl, benzimidazolyl, benzoxazolyl, benzooxadiazolyl, benzothiazolyl, benzothiadiazolyl, benzopyranyl, indolyl, isoindolyl, indazolyl, triazolyl, triazinyl, quinoxalinyl, purinyl, quinazolinyl, quinolizinyl, naphthyridinyl, pteridinyl, carbazolyl, azepinyl, diazepinyl, acridinyl and the like.

"Heteroarylalkyl" and "heteroaralkyl", which may be used interchangeably, mean a radical-$R^aR^b$ where $R^a$ is an alkylene group and $R^b$ is a heteroaryl group as defined herein.

The terms "halo" and "halogen", which may be used interchangeably, refer to a substituent fluoro, chloro, bromo, or iodo.

"Haloalkyl" means alkyl as defined herein in which one or more hydrogen has been replaced with same or different halogen. Exemplary haloalkyls include $-CH_2Cl$, $-CH_2CF_3$, $-CH_2CCl_3$, perfluoroalkyl (e.g., $-CF_3$), and the like.

"Haloalkoxy" means a moiety of the formula -OR, wherein R is a haloalkyl moiety as defined herein. Examples of haloalkoxy moieties include, but are not limited to, trifluoromethoxy, difluoromethoxy, 2,2,2-trifluoroethoxy, and the like.

"Hydroxyalkyl" refers to a subset of heteroalkyl and refers in particular to an alkyl moiety as defined herein that is substituted with one or more, preferably one, two or three hydroxy groups, provided that the same carbon atom does not carry more than one hydroxy group. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 2-hydroxy-1-hydroxymethylethyl, 2,3-dihydroxybutyl, 3,4-dihydroxybutyl and 2-(hydroxymethyl)-3-hydroxypropyl.

"Heterocycloamino" means a saturated ring wherein at least one ring atom is N, NH or N-alkyl and the remaining ring atoms form an alkylene group.

"Heterocyclyl" means a monovalent saturated moiety, consisting of one to three rings, incorporating one, two, three, or four heteroatoms (chosen from nitrogen, oxygen or sulfur). The heterocyclyl ring may be optionally substituted as defined herein. Examples of heterocyclyl moieties include, but are not limited to, optionally substituted piperidinyl, piperazinyl, homopiperazinyl, azepanyl, pyrrolidinyl, pyrazolidinyl, imidazolinyl, imidazolidinyl, oxazolidinyl, isoxazolidinyl, morpholinyl, thiazolidinyl, isothiazolidinyl, thiadiazolylidinyl, benzothiazolidinyl, benzoazolylidinyl, dihydrofuranyl, tetrahydrofuryl, dihydropyranyl, tetrahydropyranyl, thiamorpholinyl, thiamorpholinylsulfoxide, thiamorpholinylsulfone, dihydroquinolinyl, dihydrisoquinolinyl, tetrahydroquinolinyl, tetrahydrisoquinolinyl, and the like. Preferred heterocyclyl include tetrahydropyranyl, tetrahydrofuranyl, piperidinyl, piperazinyl and pyrrolidinyl.

"Optionally substituted", when used in association with "aryl", "phenyl", "heteroaryl" (including indolyl such as indol-1-yl, indol-2-yl and indol-3-yl, 2,3-dihydroindolyl such as 2,3-dihydroindol-1-yl, 2,3-dihydroindol-2-yl and 2,3-dihydroindol-3-yl, indazolyl such as indazol-1-yl, indazol-2-yl and indazol-3-yl, benzimidazolyl such as benzimidazol-1-yl and benzimidazol-2-yl, benzothiophenyl such as benzothiophen-2-yl and benzothiophen-3-yl, benzoxazol-2-yl, benzothiazol-2-yl, thienyl, furanyl, pyridinyl, pyrimidinyl, pyridazinyl, pyrazinyl, oxazolyl, thiazolyl, isoxazolyl, isothiazolyl, imidazolyl, pyrazolyl and quinolinyl) or "heterocyclyl", means an aryl, phenyl, heteroaryl or heterocyclyl which is optionally substituted independently with one to four substituents, preferably one or two substituents selected from alkyl, cycloalkyl, alkoxy, halo, haloalkyl, haloalkoxy, cyano, nitro, heteroalkyl, amino, acylamino, mono-alkylamino, dialkylamino, hydroxyalkyl, alkoxyalkyl, benzyloxy, cycloalkylalkyl, cycloalkoxy, cycloalkylalkoxy, alkylsulfonyloxy, optionally substituted thiophenyl, optionally substituted pyrazolyl, optionally substituted pyridinyl, morpholinocarbonyl,$-(CH_2)q-S(O)_rR^f$; $-(CH_2)_q-NR^gR^h$; $-(CH_2)_q-C(=O)-NR^gR^h$; $-(CH_2)_q-C(=O)-C(=O)-NR^gR^h$; $-(CH_2)_q-SO_2-NR^gR^h$; $-(CH_2)_q-N(R^f)-C(=O)-R^i$; $-(CH_2)_q-C(=O)-R^i$; or $-(CH_2)_q-N(R^f)-SO_2-R^g$; where q is 0 or 1, r is from 0 to 2, $R^f$, $R^g$, and $R^h$ each independently is hydrogen or alkyl, and each $R^i$ is independently hydrogen, alkyl, hydroxy, or alkoxy. Certain preferred optional substituents for "aryl", "phenyl", "heteroaryl" "cycloalkyl" or "heterocyclyl" include alkyl, halo, haloalkyl, alkoxy, cyano, amino and alkylsulfonyl. More preferred substituents are methyl, fluoro, chloro, trifluoromethyl, methoxy, amino and methanesulfonyl.

"Leaving group" means the group with the meaning conventionally associated with it in synthetic organic chemistry, i.e., an atom or group displaceable under substitution reaction conditions. Examples of leaving groups include, but are not limited to, halogen, alkane- or arylenesulfonyloxy, such as methanesulfonyloxy, ethanesulfonyloxy, thiomethyl, benzenesulfonyloxy, tosyloxy, and thienyloxy, dihalophosphinoyloxy, optionally substituted benzyloxy, isopropyloxy, acyloxy, and the like.

"Modulator" means a molecule that interacts with a target. The interactions include, but are not limited to, agonist, antagonist, and the like, as defined herein.

"Optional" or "optionally" means that the subsequently described event or circumstance may but need not occur, and that the description includes instances where the event or circumstance occurs and instances in which it does not.

"Disease" and "Disease state" means any disease, condition, symptom, disorder or indication.

"Inert organic solvent" or "inert solvent" means the solvent is inert under the conditions of the reaction being described in conjunction therewith, including for example, benzene, toluene, acetonitrile, tetrahydrofuran, N,N-dimethylformamide, chloroform, methylene chloride or dichloromethane, dichloroethane, diethyl ether, ethyl acetate, acetone, methyl ethyl ketone, methanol, ethanol, propanol, isopropanol, *tert*-butanol, dioxane, pyridine, and the like. Unless specified to the contrary, the solvents used in the reactions of the present invention are inert solvents.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

"Pharmaceutically acceptable salts" of a compound means salts that are pharmaceutically acceptable, as defined herein, and that possess the desired pharmacological activity of the parent compound.

Such salts include:

acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid, trimethylacetic acid, and the like; or

salts formed when an acidic proton present in the parent compound either is replaced by a metal ion, e.g., an alkali metal ion, an alkaline earth ion, or an aluminum ion; or coordinates with an organic or inorganic base. Acceptable organic bases include diethanolamine, ethanolamine, N-methylglucamine, triethanolamine, tromethamine, and the like. Acceptable inorganic bases include aluminum hydroxide, calcium hydroxide, potassium hydroxide, sodium carbonate and sodium hydroxide.

The preferred pharmaceutically acceptable salts are the salts formed from acetic acid, hydrochloric acid, sulphuric acid, methanesulfonic acid, maleic acid, phosphoric acid, tartaric acid, citric acid, sodium, potassium, calcium, zinc, and magnesium.

It should be understood that all references to pharmaceutically acceptable salts include solvent addition forms (solvates) or crystal forms (polymorphs) as defined herein, of the same acid addition salt.

"Protective group" or "protecting group" means the group which selectively blocks one reactive site in a multifunctional compound such that a chemical reaction can be carried out selectively at another unprotected reactive site in the meaning conventionally associated with it in synthetic chemistry. Certain processes of this invention rely upon the protective groups to block reactive nitrogen and/or oxygen atoms present in the reactants. For example, the terms "amino-protecting group" and "nitrogen protecting group" are used interchangeably herein and refer to those organic groups intended to protect the nitrogen atom against undesirable reactions during synthetic procedures. Exemplary nitrogen protecting groups include, but are not limited to, trifluoroacetyl, acetamido, benzyl (Bn), benzyloxycarbonyl (carbobenzyloxy, CBZ), p-methoxybenzyloxycarbonyl, p-nitrobenzyloxycarbonyl, *tert*-butoxycarbonyl (BOC), and the like. Skilled persons will know how to choose a group for the ease of removal and for the ability to withstand the following reactions.

"Solvates" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as $H_2O$, such combination being able to form one or more hydrate.

"Subject" means mammals and non-mammals. Mammals means any member of the mammalia class including, but not limited to, humans; non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex.

"Disease states" associated with serotonin, norepinephrine and/or dopamine neurotransmission include depressive and anxiolytic disorders, as well as schizophrenia and other psychoses, dyskinesias, drug addition, cognitive disorders, Alzheimer's disease, attention deficit disorders such as ADHD, obsessive-compulsive behaviour, panic attacks, social phobias, eating disorders such as obesity, anorexia, bulimia and "binge-eating", stress, hyperglycaemia, hyperlipidaemia, non-insulin-dependent diabetes, seizure disorders such as epilepsy, and treatment of conditions associated with neurological damage resulting from stroke, brain trauma, cerebral ischaemia, head injury, haemorrhage, and disorders and disease states of the urinary tract. "Disease states" associated with serotonin, norepinephrine and/or dopamine neurotransmission also include inflammation conditions in a subject. Compounds of the invention would be useful to treat arthritis, including but not limited to, rheumatoid arthritis, spondyloarthropathies, gouty arthritis, osteoarthritis, systemic lupus erythematosus and juvenile arthritis, osteoarthritis, gouty arthritis and other arthritic conditions.

"Depression" as used herein includes, but is not limited to, major depression, long-term depression, treatment resistant depression, dysthymia, mental states of depressed mood characterised by feelings of sadness, despair, discouragement, "blues", melancholy, feelings of low self esteem, guilt and self reproach, withdrawal from interpersonal contact, and somatic symptoms such as eating and sleep disturbances.

"Anxiety" as used herein includes, but is not limited to, unpleasant or undesirable emotional states associated with psychophysiological responses to anticipation of unreal, imagined or exaggerated danger or harm, and physical concomitants such as increased heart rate, altered respiration rate, sweating, trembling, weakness and fatigue, feelings of impending danger, powerlessness, apprehension and tension.

"Disorders of the urinary tract" or "uropathy" used interchangeably with "symptoms of the urinary tract" means the pathologic changes in the urinary tract. Examples of urinary tract disorders include, but are not limited to, stress incontinence, urge incontence, benign prostatic hypertrophy (BPH), prostatitis, detrusor hyperreflexia, outlet obstruction, urinary frequency, nocturia, urinary urgency, overactive bladder, pelvic hypersensitivity, urethritis, prostatodynia, cystitis, idiophatic bladder hypersensitivity, and the like.

"Disease states associated with the urinary tract" or "urinary tract disease states" or "uropathy" used interchangeably with "symptoms of the urinary tract" mean the pathologic changes in the urinary tract, or dysfunction of urinary bladder smooth muscle or its innervation causing disordered urinary storage or voiding. Symptoms of the urinary tract include, but are not limited to, overactive bladder (also known as detrusor hyperactivity), outlet obstruction, outlet insufficiency, and pelvic hypersensitivity.

"Overactive bladder" or "detrusor hyperactivity" includes, but is not limited to, the changes symptomatically manifested as urgency, frequency, altered bladder capacity, incontinence, micturition threshold, unstable bladder contractions, sphincteric spasticity, detrusor hyperreflexia (neurogenic bladder), detrusor instability, and the like.

"Outlet obstruction" includes, but is not limited to, benign prostatic hypertrophy (BPH), urethral stricture disease, tumors, low flow rates, difficulty in initiating urination, urgency, suprapubic pain, and the like.

"Outlet insufficiency" includes, but is not limited to, urethral hypermobility, intrinsic sphincteric deficiency, mixed incontinence, stress incontinence, and the like.

"Pelvic hypersensitivity" includes, but is not limited to, pelvic pain, interstitial (cell) cystitis, prostatodynia, prostatitis, vulvadynia, urethritis, orchidalgia, overactive bladder, and the like.

"Pain" means the more or less localized sensation of discomfort, distress, or agony, resulting from the stimulation

of specialized nerve endings. There are many types of pain, including, but not limited to, lightning pains, phantom pains, shooting pains, acute pain, inflammatory pain, neuropathic pain, complex regional pain, neuralgia, neuropathy, and the like *(*Dorland's Illustrated Medical Dictionary, 28th Edition, W. B. Saunders Company, Philadelphia, PA). The goal of treatment of pain is to reduce the degree of severity of pain perceived by a treatment subject.

"Neuropathic pain" means the pain resulting from functional disturbances and /or pathological changes as well as noninflammatory lesions in the peripheral nervous system. Examples of neuropathic pain include, but are not limited to, thermal or mechanical hyperalgesia, thermal or mechanical allodynia, diabetic pain, entrapment pain, and the like.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The terms "those defined above" and "those defined herein" when referring to a variable incorporates by reference the broad definition of the variable as well as preferred, more preferred and most preferred definitions, if any.

"Treating" or "treatment" of a disease state includes:

(i) preventing the disease state, i.e. causing the clinical symptoms of the disease state not to develop in a subject that may be exposed to or predisposed to the disease state, but does not yet experience or display symptoms of the disease state.
(ii) inhibiting the disease state, i.e., arresting the development of the disease state or its clinical symptoms, or
(iii) relieving the disease state , i.e., causing temporary or permanent regression of the disease state or its clinical symptoms.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

In general, the nomenclature used in this Application is based on AUTONOM™ v.4.0, a Beilstein Institute computerized system for the generation of IUPAC systematic nomenclature. Chemical structures shown herein were prepared using ISIS® version 2.2. Any open valency appearing on a carbon, oxygen, sulfur or nitrogen atom in the structures herein indicates the presence of a hydrogen atom.

Whenever a chiral carbon is present in a chemical structure, it is intended that all stereoisomers associated with that chiral carbon are encompassed by the structure, so as to include specific enantiomers.

Representative compounds in accordance with the methods of the invention are shown in Table 1

Table 1

| # | Structure | Name |
|---|---|---|
| 1 | 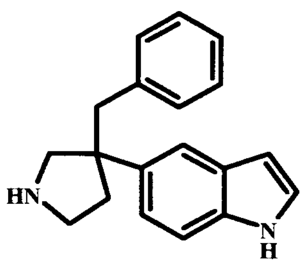 | 5-(3-Benzyl-pyrrolidin-3-yl)-1H-indole |
| 2 | 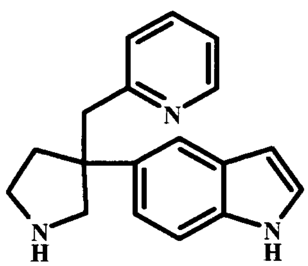 | 5-(3-Pyridin-2-ylmethyl-pyrrolidin-3-yl)-1H-indole |

(continued)

| # | Structure | Name |
|---|---|---|
| 3 | 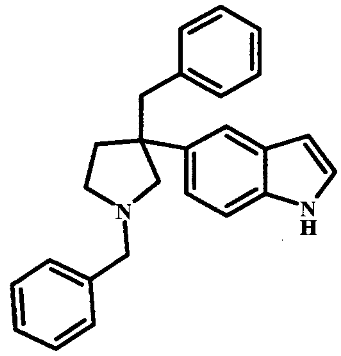 | 5-(1,3-Dibenzyl-pyrrolidin-3-yl)-1H-indole |
| 4 | 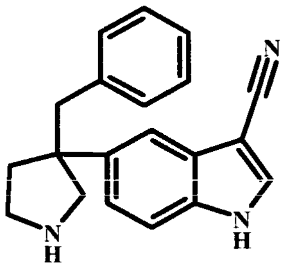 | 5-(3-Benzyl-pyrrolidin-3-yl)-1H-indole-3-earbonitrile |
| 5 | 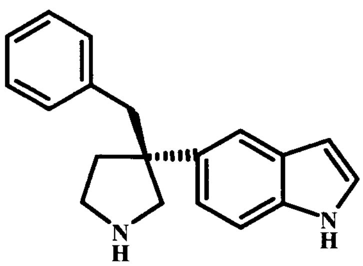 | 5-((R)-3-Benzyl-pyrrolidin-3-yl)-1H-indole |
| 6 | 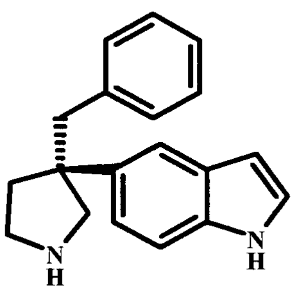 | 5-((S)-3-Benzyl-pyrrolidin-3-yl)-1H-indole |
| 7 | 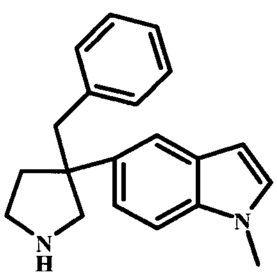 | 5-(3-Benzyl-pyrrolidin-3-yl)-1-methyl-1H-indole |

(continued)

| # | Structure | Name |
|---|---|---|
| 8 | 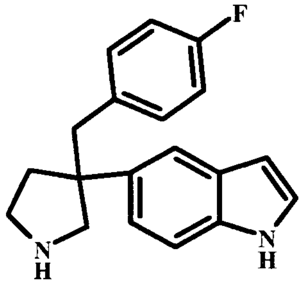 | 5-[3-(4-Fluoro-benzyl)-pyrrolidin-3-yl]-1H-indole |
| 9 | 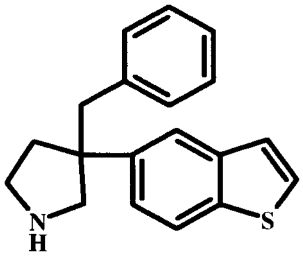 | 3-Benzo[b]thiophen-5-yl-3-benzyl-pyrro lid ine |
| 10 | 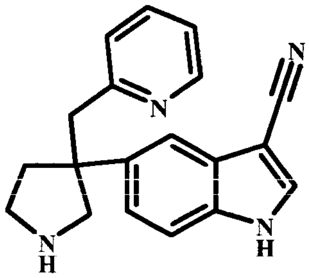 | 5-(3-Pyridin-2-ylmethyl-pyrrolidin-3-yl)-1H-indole-3-carbonitrile |
| 11 | 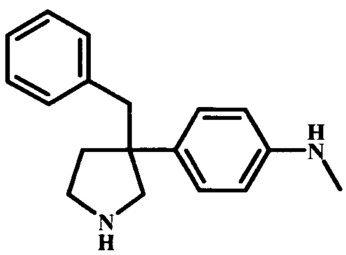 | [4-(3-Benzyl-pyrrolidin-3-yl)-phenyl]-methyl-amine. |
| 12 | 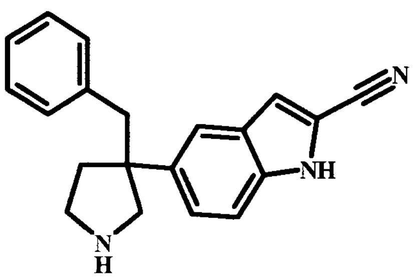 | 5-(3-Benzyl-pyrrolidin-3-yl)-1H-indole-2-carbonitrile |

(continued)

| # | Structure | Name |
|---|---|---|
| 13 | 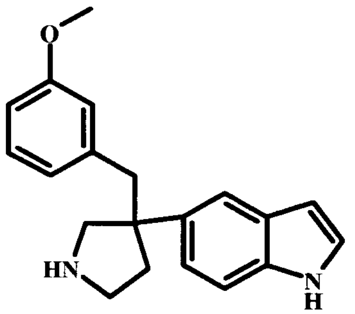 | 5-[3-(3-Methoxy-benzyl)-pyrrolidin-3-yl]-1H-indole |
| 14 | 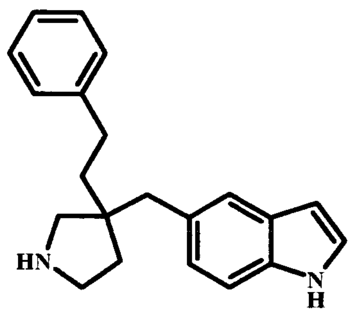 | 5-(3-Phenethyl-pyrrolidin-3-yl)-1H-indole |
| 15 | 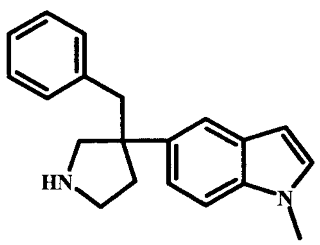 | 1-Methyl-5-(3-pyridin-2-ylmethyl-pyrrolidin-3-yl)-1H-indo le |
| 16 | 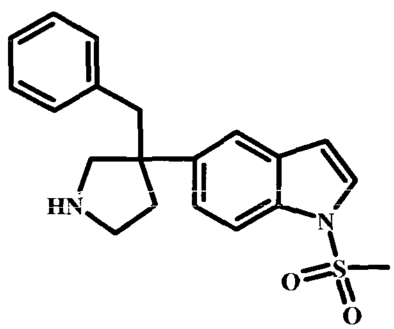 | 5-(3-Benzyl-pyrrolidin-3-yl)-1-methanesulfonyl-1H-indole |
| 17 | 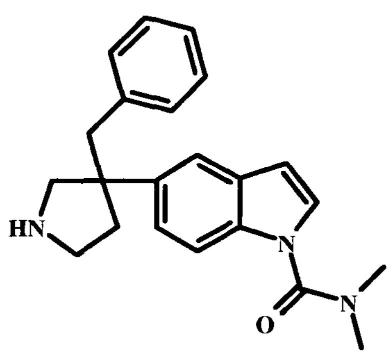 | 5-(3-Benzyl-pyrrolidin-3-yl)-indole-1-carboxylic acid dimethylamide |

(continued)

| # | Structure | Name |
|---|---|---|
| 18 | 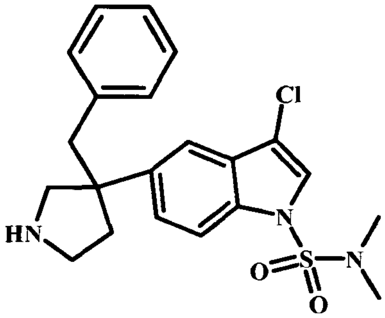 | 5-(3-Benzyl-pyrrolidin-3-yl)-3-chloro-indole-1-sulfonic acid dimethylamide |
| 19 | 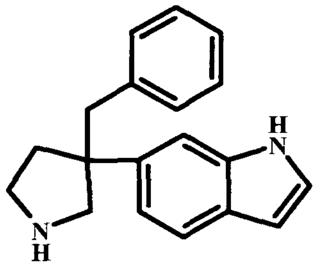 | 6-(3-Benzyl-pyrrolidin-3-yl)-1H-indole |
| 20 | 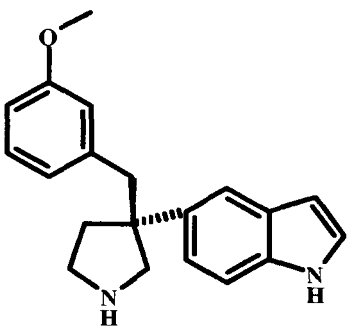 | 5-[(R)-3-(3-Methoxy-benzyl)-pyrrolidin-3-yl]-1H-indole |
| 21 | 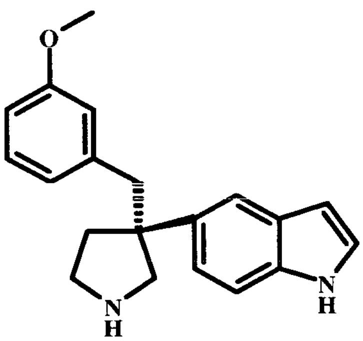 | 5-[(S)-3-(3-Methoxy-benzyl)-pyrrolidin-3-yl]-1H-indole |
| 22 | 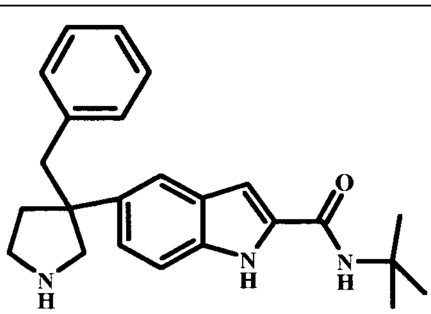 | 5-(3-Benzyl-pyrrolidin-3-yl)-1H-indole-2-carboxylic acid tert-butylamide |

(continued)

| # | Structure | Name |
|---|---|---|
| 23 | 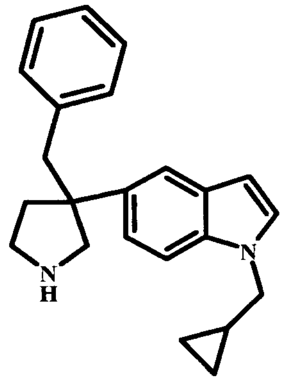 | 5-(3-Benzyl-pyrrolidin-3-yl)-1 -cyclopropylmethyl-1H-indole |
| 24 | 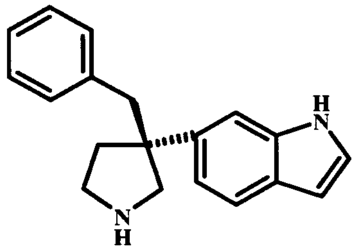 | 6-((R)-3-Benzyl-pyrrolidin-3-yl)-1H-indole |
| 25 | 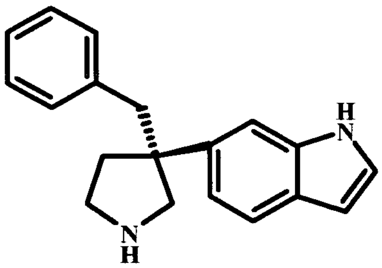 | 6-((S)-3-Benzyl-pyrrolidin-3-yl)-1H-indole |
| 26 | 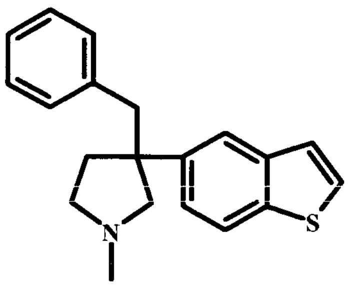 | 3-Benzo[b]thiophen-5-yl-3-benzyl-1-methyl-pyrrolidine |
| 27 | 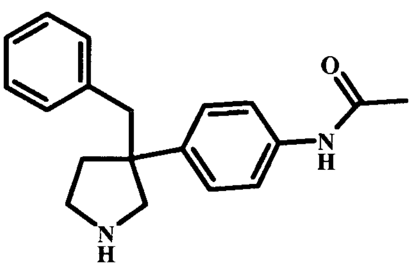 | N-[4-(3-Benzyl-pyrrolidin-3-yl)-phenyl]-acetamide |

(continued)

| # | Structure | Name |
|---|---|---|
| 28 | 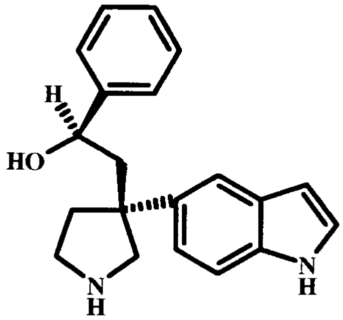 | (R)-2-[(S)-3-(1H-Indol-5-yl)-pyrrolidin-3-yl]-1-phenyl-ethanol |
| 29 | 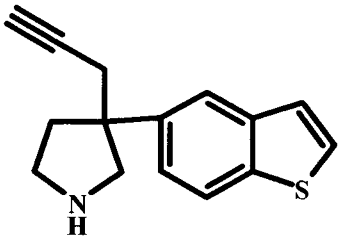 | 3-Benzo[b]thiophen-5-yl-3-prop-2-ynyl-pyrro lid ine |
| 30 | 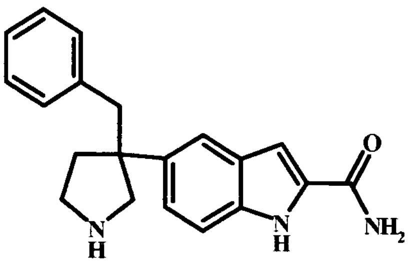 | 5-(3-Benzyl-pyrrolidin-3-yl)-1H-indole-2-carboxylic acid amide |
| 31 | 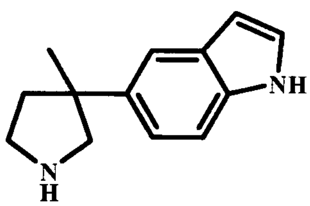 | 5-(3-Methyl-pyrrolidin-3-yl)-1H-indole |
| 32 | 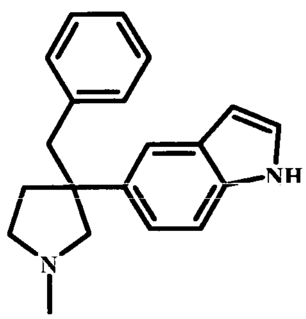 | 5-(3-Benzyl- 1-methyl-Pyrrolidin-3-yl)1H-indole |

(continued)

| # | Structure | Name |
|---|---|---|
| 33 | 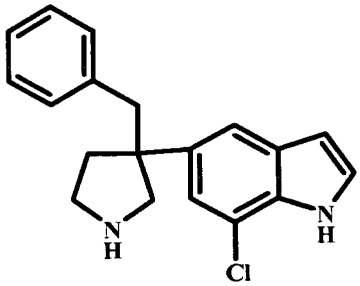 | 5-(3-Benzyl-pyrrolidin-3-yl)-7-chloro-1H-indole |
| 34 | 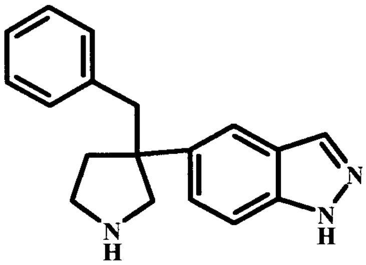 | 5-(3-Benzyl-pyrrolidin-3-yl)-1H-indazole |
| 35 | 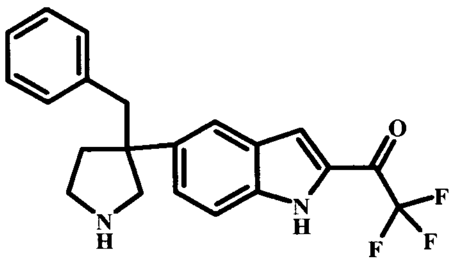 | 1-[5-(3-Benzyl-pyrrolidin-3-yl)-1H-indo1-2-yl]-2,2,2-trifluoro-ethanone |
| 36 | 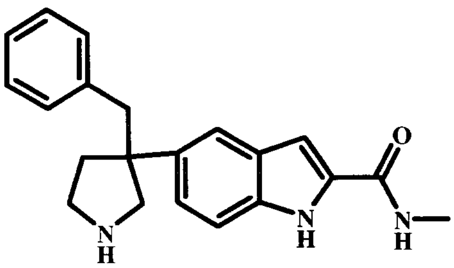 | 5-(3-Benzyl-pyrrolidin-3-yl)-1H-indole-2-carboxylic acid methylamide |
| 37 | 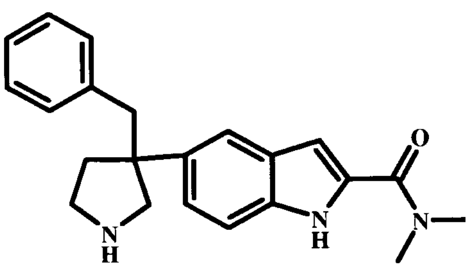 | 5-(3-Benzyl-pyrrolidin-3-yl)-1H-indole-2-carboxylic acid dimethylamide |

(continued)

| # | Structure | Name |
|---|---|---|
| 38 | 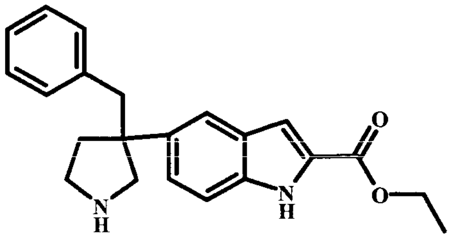 | 5-(3-Benzyl-pyrrolidin-3-yl)-1H-indole-2-carboxyclic acid ethyl ester |
| 39 | 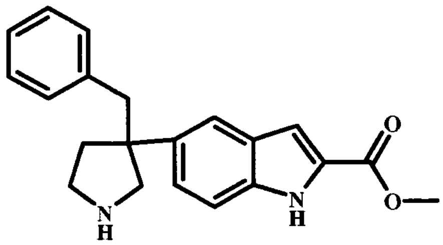 | 5-(3-Benzyl-pyrrolidin-3-yl)-1H-indole-2-carboxylic acid methyl ester |
| 40 | 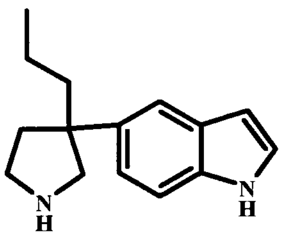 | 5-(3-Propyl-pyrrolidin-3-yl)-1H-indole |
| 41 | 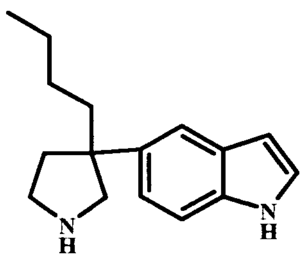 | 5-(3-Butyl-pyrrolidin-3-yl)-1H-indole |
| 42 | 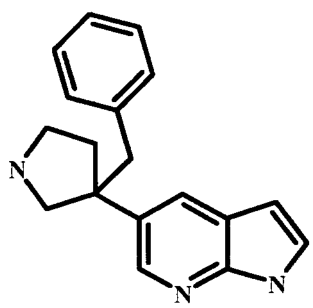 | 5-(3-Benzyl-pyrrolidin-3-yl)-1H-pyrrolo[2,3-b]pyridine |

Compounds of the present invention can be made by a variety of methods depicted in the illustrative synthetic reaction schemes shown and described below.

The starting materials and reagents used in preparing these compounds generally are either available from commercial suppliers, such as Aldrich Chemical Co., or are prepared by methods known to those skilled in the art following procedures set forth in references such as Fieser and Fieser's Reagents for Organic Synthesis; Wiley & Sons: New York, 1991, Volumes 1-15; Rodd's Chemistry of Carbon Compounds, Elsevier Science Publishers, 1989, Volumes 1-5 and Supplementals; and Organic Reactions, Wiley & Sons: New York, 1991, Volumes 1-40. The following synthetic reaction schemes are merely illustrative of some methods by which the compounds of the present invention can be synthesized, and various modifications to these synthetic reaction schemes can be made and will be suggested to one

skilled in the art having referred to the disclosure contained in this Application.

The starting materials and the intermediates of the synthetic reaction schemes can be isolated and purified if desired using conventional techniques, including but not limited to, filtration, distillation, crystallization, chromatography, and the like. Such materials can be characterized using conventional means, including physical constants and spectral data.

Unless specified to the contrary, the reactions described herein preferably are conducted under an inert atmosphere at atmospheric pressure at a reaction temperature range of from about -78 ˚C to about 150 ˚C, more preferably from about 0 ˚C to about 125 ˚C, and most preferably and conveniently at about room (or ambient) temperature, e.g., about 20 ˚C.

The compounds of the invention are usable for the treatment of diseases or conditions associated with serotonin neurotransmission, norepinephrine neuortransmission and/or dopamine neurotransmission. Such diseases and conditions include depressive and anxiolytic disorders, as well as schizophrenia and other psychoses, dyskinesias, drug addition, cognitive disorders, Alzheimer's disease, attention deficit disorders such as ADHD, obsessive-compulsive behaviour, panic attacks, social phobias, eating disorders such as obesity, anorexia, bulimia and "binge-eating", stress, hyperglycaemia, hyperlipidaemia, non-insulin-dependent diabetes, seizure disorders such as epilepsy, and treatment of conditions associated with neurological damage resulting from stroke, brain trauma, cerebral ischaemia, head injury, and haemorrhage.

The compounds of the invention are also usable for treatment of disorders and disease states of the urinary tract such as stress incontinence, urge incontinence, benign prostatic hypertrophy (BPH), prostatitis, detrusor hyperreflexia, outlet obstruction, urinary frequency, nocturia, urinary urgency, overactive bladder, pelvic hypersensitivity, urethritis, prostatodynia, cystitis, idiophatic bladder hypersensitivity.

The compounds of the invention also possess anti-inflammatory and/or analgesic properties *in vivo,* and accordingly, are expected to find utility in the treatment of disease states associated with pain conditions from a wide variety of causes, including, but not limited to, neuropathic pain, inflammatory pain, surgical pain, visceral pain, dental pain, premenstrual pain, central pain, pain due to burns, migraine or cluster headaches, nerve injury, neuritis, neuralgias, poisoning, ischemic injury, interstitial cystitis, cancer pain, viral, parasitic or bacterial infection, post-traumatic injuries (including fractures and sports injuries), and pain associated with functional bowel disorders such as irritable bowel syndrome.

Compounds of the invention are also useful for treatment of arthritis, including but not limited to, rheumatoid arthritis, spondyloarthropathies, gouty arthritis, osteoarthritis, systemic lupus erythematosus and juvenile arthritis, osteoarthritis, gouty arthritis and other arthritic conditions.

The invention includes pharmaceutical compositions comprising at least one compound of the present invention, or an individual isomer, racemic or non-racemic mixture of isomers or a pharmaceutically acceptable salt or solvate thereof, together with at least one pharmaceutically acceptable carrier, and optionally other therapeutic and/or prophylactic ingredients.

In general, the compounds of the invention will be administered in a therapeutically effective amount by any of the accepted modes of administration for agents that serve similar utilities. Suitable dosage ranges are typically 1-500 mg daily, preferably 1-100 mg daily, and most preferably 1-30 mg daily, depending upon numerous factors such as the severity of the disease to be treated, the age and relative health of the subject, the potency of the compound used, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this Application, to ascertain a therapeutically effective amount of the compounds of the present invention for a given disease.

Compounds of the invention may be administered as pharmaceutical formulations including those suitable for oral (including buccal and sub-lingual), rectal, nasal, topical, pulmonary, vaginal, or parenteral (including intramuscular, intraarterial, intrathecal, subcutaneous and intravenous) administration or in a form suitable for administration by inhalation or insufflation. The preferred manner of administration is generally oral using a convenient daily dosage regimen which can be adjusted according to the degree of affliction.

A compound or compounds of the invention, together with one or more conventional adjuvants, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. Formulations containing about one (1) milligram of active ingredient or, more broadly, about 0.01 to about one hundred (100) milligrams, per tablet, are accordingly suitable representative unit dosage forms.

The compounds of the invention may be formulated in a wide variety of oral administration dosage forms. The pharmaceutical compositions and dosage forms may comprise a compound or compounds of the present invention or pharmaceutically acceptable salts thereof as the active component. The pharmaceutically acceptable carriers may be either solid or liquid. Solid form preparations include powders, tablets, pills, capsules, cachets, suppositories, and dispersible granules. A solid carrier may be one or more substances which may also act as diluents, flavouring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. The powders and tablets preferably contain from about one (1) to about seventy (70) percent of the active compound. Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting wax, cocoa butter, and the like. The term "preparation" is intended to include the formulation of the active compound with encapsulating material as carrier, providing a capsule in which the active component, with or without carriers, is surrounded by a carrier, which is in association with it. Similarly, cachets and lozenges are included. Tablets, powders, capsules, pills, cachets, and lozenges may be as solid forms suitable for oral administration.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents. Aqueous suspensions can be prepared by dispersing the finely divided active component in water with viscous material, such as natural or synthetic gums, resins, methylcellulose, sodium carboxymethylcellulose, and other well known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The compounds of the invention may be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, for example solutions in aqueous polyethylene glycol. Examples of oily or nonaqueous carriers, diluents, solvents or vehicles include propylene glycol, polyethylene glycol, vegetable oils (e.g., olive oil), and injectable organic esters (e.g., ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilization from solution for constitution before use with a suitable vehicle, e.g., sterile, pyrogen-free water.

The compounds of the invention may be formulated for topical administration to the epidermis as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatine and glycerine or sucrose and acacia; and mouthwashes comprising the active ingredient in a suitable liquid carrier.

The compounds of the invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The compounds of the invention may be formulated for vaginal administration. Pessaries, tampons, creams, gels, pastes, foams or sprays containing in addition to the active ingredient such carriers as are known in the art to be appropriate.

The subject compounds may be formulated for nasal administration. The solutions or suspensions are applied directly to the nasal cavity by conventional means, for example, with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved for example by means of a metering atomizing spray pump.

The compounds of the invention may be formulated for aerosol administration, particularly to the respiratory tract and including intranasal administration. The compound will generally have a small particle size for example of the order of five (5) microns or less. Such a particle size may be obtained by means known in the art, for example by

micronization. The active ingredient is provided in a pressurized pack with a suitable propellant such as a chlorofluorocarbon (CFC), for example, dichlorodifluoromethane, trichlorofluoromethane, or chlorofluorocarbon (CFC), for example, dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form for example in capsules or cartridges of e.g., gelatine or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to a skin-adhesive solid support. The compound of interest can also be combined with a penetration enhancer, e.g., Azone (1-dodecylazacycloheptan-2-one). Sustained release delivery systems are inserted subcutaneously into the subdermal layer by surgery or injection. The subdermal implants encapsulate the compound in a lipid soluble membrane, e.g., silicone rubber, or a biodegradable polymer, e.g., polylactic acid.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Other suitable pharmaceutical carriers and their formulations are described in Remington: The Science and Practice of Pharmacy 1995, edited by E. W. Martin, Mack Publishing Company, 19the edition, Easton, Pennsylvania. Representative pharmaceutical formulations containing a compound of the present invention are described below.

Examples

The following preparations and examples are given to enable those skilled in the art to more clearly understand and to practice the present invention. They should not be considered as limiting the scope of the invention, but merely as being illustrative and representative thereof

Whenever a chiral carbon is present in a chemical structure, it is intended that all stereoisomers associated with that chiral carbon are encompassed by the structure, so as to include specific enantiomers.

The following abbreviations may be used in the Examples.

Abbreviations

| | |
|---|---|
| ACE-Cl | α-Chloroethyl chloroformate |
| AcOH | Acetic acid |
| Bn | Benzyl |
| $(BOC)_2O$ | di-ter-t-Butyl dicarbonate |
| t-BuLi | ter-t-Butyllithium |
| t-BuOH | ter-t-Butyl alcohol |
| m-CPBA | 3-Chloroperoxybenzoic acid |
| DCE | 1,2-Dichloroethane |
| DCM | Dichloromethane/Methylene chloride |
| DEA | Diethylamine |
| DIPEA | Diisopropylethylamine |
| DIBALH | Diisobutylaluminum hydride |
| DMAP | 4-Dimethylaminopyridine |
| DMF | N,N-Dimethylformamide |
| DMP | Dess Martin Periodinane (acetic acid 1,1-diacetoxy-3-oxo-1lambda*5*-ioda- 2-oxa-indan-1-yl ester) |
| DMSO | Dimethyl sulphoxide |
| Dppf | 1,1'-Bis(diphenylphosphino)ferrocene |
| EDC | 1-(3-Dimethylaminopropyl)-3-ethylcarbodiimide hydrochloride |
| EtOAc | Ethyl acetate |

| | |
|---|---|
| HPLC | High pressure liquid chromatography |
| HOBt | 1-Hydroxybenzotriazole |
| LAH | Lithium aluminum hydride |
| LHMDS | Lithium bis(trimethylsilyl)amide |
| MeOH | Methanol |
| MsCl | Methanesulfonyl chloride |
| NMP | 1-Methyl-2-pyrrolidinone |
| NBS | N-bromosuccinimide |
| PFBSF | Perfluorobutanesulfonyl fluoride |
| PPTS | Pyridinium p-toluenesulfonate |
| TBAF | Tetrabutylammonium fluoride |
| TBAHS | Tetrabutyl ammonium hydrogen sulfate |
| TBDMS | ter-t-Butyldimethylsilyl |
| TMSI | Iodotrimethylsilane |
| TEA | Triethylamine |
| TIPS | Triisopropylsilyl |
| TFA | Trifluoroacetic acid |
| THF | Tetrahydrofuran |
| TLC | Thin layer chromatography |
| TMAF | Tetramethylammonium fluoride |
| TMS | Trimethylsilyl |
| p-TSOH | p-Toluenesulfonic acid |

Procedure 1 1-(Tetrahydro-pyran-2-yl)-5-(4,4,5,5-tetramethyl-[1,3,2]dioxaborolan-2-yl)-1*H*-indazole

The synthetic procedure described in this Procedure was carried out according to the process shown in Scheme A.

**SCHEME A**

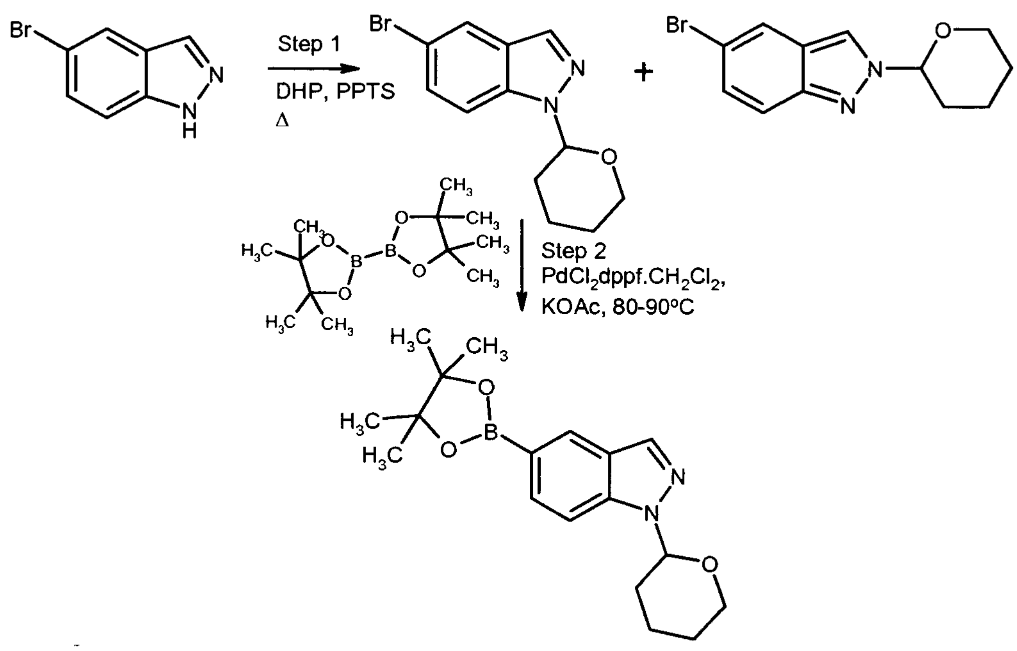


Step 1 5-Bromo-1-(tetrahydro-pyran-2-yl)-1*H*-indazole

A mixture of 5-bromoindazole (2.48 g, 12.6 mmol), 3,4-dihydro-2H-pyran (2.61 mL, 28.9 mmol) and pyridinium p-toluenesulfonate (158 mg, 0.629 mmol) in DCM (15 mL) was heated at reflux for 5 hours. The reaction mixture was then cooled and poured onto a saturated aqueous solution of $NaHCO_3$ (30 mL). The organic layer was separated and the aqueous layer was extracted twice with DCM (30 mL). The combined organic extracts were washed with a solution

of citric acid (1 M in water, ca. 40 ml), with brine (30 mL), dried over $MgSO_4$, filtered and evaporated under reduced pressure to give an orange oil (4 g). This crude material was purified via flash chromatography (10% to 30% EtOAc in hexane) to give 2.46 g (70% yield) of 5-bromo-1-(tetrahydro-pyran-2-yl)-1*H*-indazole as an orange gum and 0.86 g (24% yield) of 5-bromo-2-(tetrahydro-pyran-2-yl)-2*H*-indazole as an orange oil.

Step 2 1-(Tetrahydro-pyran-2-yl)-5-(4,4,5,5-tetramethyl-[1,3,2]dioxaborolan-2-yl-1*H*-indazole.

A mixture of 5-bromo-1-(tetrahydro-pyran-2-yl)-1*H*-indazole (2.40 g, 8.54 mmol), bis(pinacolato)diboron (3.25 g, 12.8 mmol), KOAc (2.51 g, 2.6 mmol), [1,1'-bis(diphenylphosphino)-ferrocene]dichloro palladium(II).$CH_2Cl_2$ (0.348 g, 0.427 mmol) and DMSO (50 mL) was heated at 90° C under nitrogen atmosphere. After stirring for 5 hours at 90° C the dark mixture was cooled and poured into a mixture of water (100 mL) and EtOAc (200 mL). The organic phase was separated and the aqueous phase was extracted 3 times with EtOAc (100 mL). The combined organic extracts were washed once with brine (150 mL), 3 times with water (100 mL) and with brine again (100 mL) and then dried over $MgSO_4$, filtered and evaporated under reduced pressure to give a dark brown oil that solidified upon standing. This crude material was purified via flash chromatography (10% to 30% EtOAc in hexane) to give 1.66 g (59% yield) of 1-(tetrahydro-pyran-2-yl)-5-(4,4,5,5-tetramethyl-[1,3,2]dioxaborolan-2-yl)-1*H*-indazole as a yellow gum.

Similarly prepared using the appropriate starting material, were: 1-(*tert*-Butyl-dimethyl-silanyl)-5-(4,4,5,5-tetramethyl-[1,3,2]dioxaborolan-2-yl)-1*H*-pyrrolo[2,3-*b*]pyridine (271 mg, 47% yield, low melting point solid), using 5-bromo-1-(*tert*-butyl-dimethyl-silanyl)-1*H*-pyrrolo[2,3-b]pyridine synthesized as described in W02004/078757 A2; 7-Chloro-5-(4,4,5,5-tetramethyl-[1,3,2]dioxaborolan-2-yl)-1*H*-indole (2.31 g, 96% yield, white solid).

Example 1 5-(3-Benzyl-pyrrolidin-3-yl-1*H*-indole

The synthetic procedure described in this Example was carried out according to the process shown in Scheme B.

**SCHEME B**

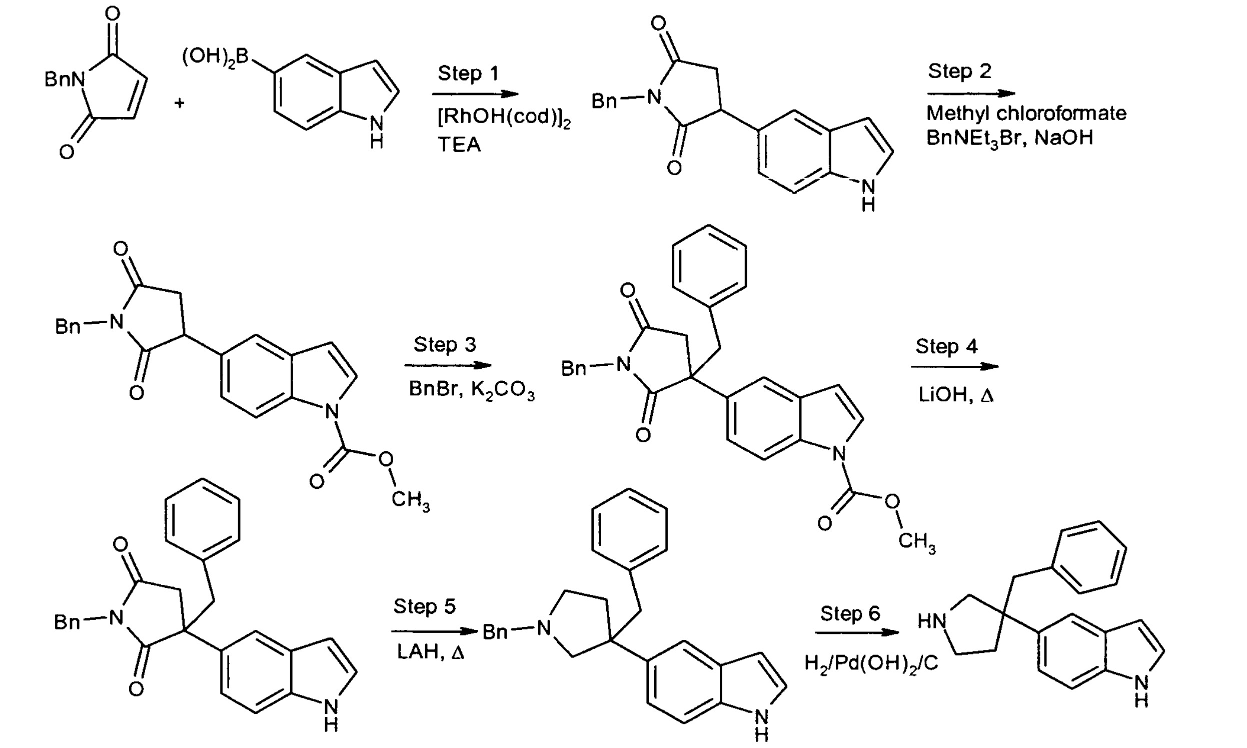


Step 1 1-Benzyl-3-(1*H*-indol-5-yl)-pyrrolidine-2,5-dione

Triethylamine (0.433 mL, 3.11 mmol) and N-benzylmaleimide (0.582 mg, 3.11 mmol) were added to a solution of 5-indolylboronic acid (0.750 mg, 4.66 mmol) and $[RhOH(cod)]_2$ (70.9 mg, 0.155 mmol) in a mixture of 1,4-dioxane/water (9/1, 10 mL). The dark brown mixture was heated at 50° C for 2.5 hours; it was then cooled and filtered through a silica plug. The filter cake was washed with EtOAc (100 mL) and the filtrate was concentrated under reduced pressure

to a brown oil (1.5 g). This crude material was purified via flash chromatography (10% to 100% of EtOAc in hexane) to give 932 mg (99% yield) of 1-benzyl-3-(1*H*-indol-5-yl)-pyrrolidine-2,5-dione as a pale yellow foam.

Similarly prepared, using the appropriate boronic acid, were: 3-Benzo[b]thiophen-5-yl-1-benzyl-pyrrolidine-2,5-dione (yellow foam, 93% yield) using 2-(1-benzothiophene-5-yl)-4,4,5,5-tetramethyl-1,3,2-dioxaborolane; 1-Benzyl-3-[1-(tetrahydro-pyran-2-yl)-1*H*-indazo-5-yl]-pyrrolidine-2,5-dione (yellow gum, 80% yield) using 1-(tetrahydro-pyran-2-yl)-5-(4,4,5,5-tetramethyl-[1,3,2]dioxaborolan-2-yl)-1*H*-indazole (see Preparation 1); [4-(1-Benzyl-2,5-dioxo-pyrrolidin-3-yl)-phenyl]-carbamic acid *tert*-butyl ester (brown solid, 91% yield) using 4-(N-BOC-amino)phenyl boronic acid; MS = 761 $[2M+H]^+$; 1-Benzyl-3-(1*H*-indol-6-yl)-pyrrolidine-2,5-dione (colorless crisp solid, 1.39 g, 92% yield); 1-Benzyl-3-[1-(*tert*-butyl-dimethyl-silanyl)-1*H*-pyrrolo[2,3-*b*]pyridin-5-yl]-pyrrolidine-2,5-dione (white solid, 52% yield); MS = 420 $[M+H]^+$; 3-(1-Benzenesulfonyl-1*H*-indol-3-yl)-1-benzyl-pyrrolidine-2,5-dione (colorless foam, 66% yield); MS = 445 $[M+H]^+$, 486 $[M+H+CH_3CN]^+$; 1-Benzyl-3-(7-chloro-1*H*-indol-5-yl)-pyrrolidine-2,5-dione (yellow gum, 2.80 g, quantitative yield).

Similarly prepared using N-methylmaleimide, were: 3-Benzo[b]thiophen-5-yl-1-methyl-pyrrolidine-2,5-dione (25% yield); and 3-(1*H*-Indol-5-yl)-1-methyl-pyrrolidine-2,5-dione (yellow solid, 71% yield).

Step 2 5-(1-Benzyl-2,5-dioxo-pyrrolidin-3-yl)-indole-1-carboxylic acid methyl ester

Methyl chloroformate (0.26 mL, 3.31 mmol) was added dropwise over a 15 minutes period to a rapidly stirred mixture of 1-benzyl-3-(1*H*-indol-5-yl)-pyrrolidine-2,5-dione (0.606 mg, 1.99 mmol) and benzyltriethylammoniumbromide (5.4 mg, 19.9 μmol) in a mixture of DCM (8 mL) and NaOH (30% in water, 8 mL) at 0° C. The reaction mixture was stirred for 30 minutes; it was then extracted 3 times with DCM (10 mL). The combined organic extracts were washed with water (15 mL) and brine (15 mL), dried over $MgSO_4$, filtered and evaporated under reduced pressure to give an orange-brown foam (590 mg). This crude material was purified via flash chromatography (5% to 60% of EtOAc in hexane) to give 400 mg (56% yield) of 5-(1-benzyl-2,5-dioxo-pyrrolidin-3-yl)-indole-1-carboxylic acid methyl ester as a yellow gum. MS = 363 $[M+H]^+$, 404 $[M+H+CH_3CN]^+$.

Similarly prepared using the appropriate starting material, were: 6-(1-Benzyl-2,5-dioxo-pyrrolidin-3-yl)-indole-1-carboxylic acid methyl ester (yellow solid, 1.04 g, 64% yield); 5-(1-Benzyl-2,5-dioxo-pyrrolidin-3-yl)-7-chloro-indole-1-carboxylic acid methyl ester (yellow gum, 1.0 g, 33% yield); and 5-(1-Methyl-2,5-dioxo-pyrrolidin-3-yl)-indole-1-carboxylic acid methyl ester.

Step 3 5-(1,3-Dibenzyl-2,5-dioxo-pyrrolidin-3-yl)-indole-1-carboxylic acid methyl ester

To a stirring mixture of 5-(1-benzyl-2,5-dioxo-pyrrolidin-3-yl)-indole-1-carboxylic acid methyl ester (177 mg, 0.489 mmol) in DMF (0.5 mL) was added, at room temperature, under nitrogen atmosphere, benzyl bromide (0.166 mL, 0.978 mmol) followed by anhydrous (freshly dried under high vacuum) $K_2CO_3$ (350 mg). After stirring for 5 hours at room temperature EtOAc (20 mL) and water (20 mL) were added. The organic layer was separated and the aqueous layer was extracted twice with EtOAc (15 mL). The combined organic extracts were washed with a saturated aqueous solution of $NaHCO_3$ (20 mL) and brine (20 mL); and then dried over $MgSO_4$, filtered and evaporated under reduced pressure. The crude residue was purified via flash chromatography (5% to 40% of EtOAc in hexane) to give 170 mg (77% yield) of 5-(1,3-dibenzyl-2,5-dioxo-pyrrolidin-3-yl)-indole-1-carboxylic acid methyl ester as a white powder. MS = 453 $[M+H]^+$, 494 $[M+H+CH_3CN]^+$, 905 $[2M+H]^+$.

Similarly prepared using the appropriate starting material, were: 1,3-Dibenzyl-3-[1-(tetrahydropyran-2-yl)-1*H*-indazol-5-yl]-pyrrolidine-2,5-dione (pale yellow foam, 57% yield); MS = 480 $[M+H]^+$; [4-(1,3-Dibenzyl-2,5-dioxo-pyrrolidin-3-yl)-phenyl]-carbamic acid ter-t-butyl ester (pale yellow foam, 47% yield); MH = 469 $[M-H]^-$; 493 $[M+Na]^+$; 6-(1,3-Dibenzyl-2,5-dioxo-pyrrolidin-3-yl)-indole-1-carboxylic acid methyl ester (fluorescent yellow gum, 531 mg, 85% yield); 1,3-Dibenzyl-3-[1-(*tert*-butyl-dimethyl-silanyl)-1*H*-pyrrolo[2,3-*b*]pyridin-5-yl]-pyrrolidine-2,5-dione (colorless foam, 67% yield); 3-(1-Benzenesulfonyl-1*H*-indol-3-yl)-1,3-dibenzyl-pyrrolidine-2,5-dione (white solid, 63% yield); MS = 535 $[M+H]^+$; 7-Chloro-5-(1,3-dibenzyl-2,5-dioxo-pyrrolidin-3-yl)-indole-1-carboxylic acid methyl ester (colorless gum, 447 mg, 36% yield); 5-[1-Benzyl-3-(4-fluoro-benzyl)-2,5-dioxo-pyrrolidin-3-yl]-indole-1-carboxylic acid methyl ester (yellow foam, 529 mg, 93% yield); 5-[1-Benzyl-3-(3-methoxy-benzyl)-2,5-dioxo-pyrrolidin-3-yl]-indole-1-carboxylic acid methyl ester (viscous gum, 519 mg, 78% yield); 5-(3-Benzyl-1-methyl-2,5-dioxo-pyrrolidin-3-yl)-indole-1-carboxylic acid methyl ester (off white solid, 527 mg, 80% yield); MS = 377 $[M+H]^+$; 5-(1-Benzyl-2,5-dioxo-3-pyridin-2-ylmethyl-pyrrolidin-3-yl)-indole-1-carboxylic acid methyl ester (glossy yellow solid, 1.23 g, 59% yield); 5-(1-Benzyl-2,5-dioxo-3-phenethyl-pyrrolidin-3-yl)-indole-1-carboxylic acid methyl ester (yellow gum, 381 mg, 59% yield); 5-[1-Benzyl-2,5-dioxo-3-(2-oxo-2-phenyl-ethyl)-pyrrolidin-3-yl]-indole-1-carboxylic acid methyl ester (yellow solid, 494 mg, 75% yield); and 5-(1-Benzyl-3-methyl-2,5-dioxo-pyrrolidin-3-yl)-indole-1-carboxylic acid methyl ester (324 mg, 62% yield).

Step 4 1 3-Dibenzyl-3-(1*H*-indol-5-)-pyrrolidine-2,5-dione

A solution of 5-(1,3-dibenzyl-2,5-dioxo-pyrrolidin-3-yl)-indole-1-carboxylic acid methyl ester (162 mg, 0.358 mmol) and LiOH (34 mg, 1.43 mmol) in a mixture of THF (6 mL) and water (2 mL) was heated at reflux for 1.5 hour. The reaction mixture was concentrated under reduced pressure; the residue was diluted with EtOAc (30 mL) and washed with water (10 mL), citric acid (10 mL), a saturated aqueous solution of $NaHCO_3$ (10 mL) and brine (10 mL). It was then dried over $MgSO_4$, filtered and evaporated under reduced pressure to give 142 mg (quantitative yield) of 1,3-dibenzyl-3-(1*H*-indol-5-yl)-pyrrolidine-2,5-dione as a colorless gum that was used without further purifications.

Similarly prepared using the appropriate indole, were: 1,3-Dibenzyl-3-(1*H*-indol-6-yl)-pyrrolidine-2,5-dione (colorless gum, 450 mg, 100% yield); 1-Benzyl-3-(4-fluoro-benzyl)-3-(1*H*-indol-5-yl)-pyrrolidine-2,5-dione (off-white foam, 412 mg, 91% yield); 1-Benzyl-3-(1H-indol-5-yl)-3-(3-methoxy-benzyl)-pyrrolidine-2,5-dione (colorless gum, 424 mg, 94% yield); 1-Benzyl-3-(1*H*-indol-5-yl)-3-pyridin-2-ylmethyl-pyrrolidine-2,5-dione (yellow gum, 192 mg, 85% yield); 1-Benzyl-3-(1*H*-indol-5-yl)-3-phenethyl-pyrrolidine-2,5-dione (277 mg, 92% yield); 1-Benzyl-3-(1*H*-indol-5-yl)-3-(2-oxo-2-phenyl-ethyl)-pyrrolidine-2,5-dione (yellow solid, 297 mg, 67% yield); and 1-Benzyl-3-(1*H*-indol-5-yl)-3-methyl-pyrrolidine-2,5-dione (yellow gum, 148 mg, 66% yield).

Step 5 5-(1,3-Dibenzyl-pyrrolidin-3-yl)-1*H*-indole

A solution of lithium aluminum hydride (1.0 M solution in THF, 1.63 mL) was added dropwise, under nitrogen atmosphere, at room temperature, to a stirred solution of 1,3-dibenzyl-3-(1*H*-indol-5-yl)-pyrrolidine-2,5-dione (129 mg, 0.327 mmol) in THF (4 mL). The reaction mixture was heated at 80° C for 4 hours; it was then cooled at room temperature and quenched by addition of $Na_2SO_4$. $10H_2O$ (ca. 1 g). The mixture was diluted with EtOAc (25 mL) and filtered; the inorganic salts were washed 3 times with EtOAc (5 mL). The filtrate was concentrated under reduced pressure to give 5-(1,3-dibenzyl-pyrrolidin-3-yl)-1*H*-indole (108 mg, 90% yield) that was used without further purifications. MS = 367 $[M+H]^+$. Similarly prepared used the appropriate starting material, were: 5-(1,3-Dibenzyl-pyrrolidin-3-yl)-1-(tetrahydro-pyran-2-yl)-1H-indazole, MS = 452 $[M+H]^+$; 6-(1,3-Dibenzyl-pyrrolidin-3-yl)-1*H*-indole (colorless foam, 260 mg, 66% yield), MS = 367 $[M+H]^+$; 5-[1-Benzyl-3-(4-fluoro-benzyl)-pyrrolidin-3-yl]-1*H*-indole (colorless film, 131 mg, 67% yield); 5-[1-Benzyl-3-(3-methoxy-benzyl)-pyrrolidin-3-yl]-1*H*-indole (white solid, 420 mg, quantitative yield); 5-(1-Benzyl-3-pyridin-2-ylmethyl-pyrrolidin-3-yl)-1*H*-indole (quantitative yield); 5-(1-Benzyl-3-phenethyl-pyrrolidin-3-yl)-1*H*-indole; and 5-(1-Benzyl-3-methyl-pyrrolidin-3-yl)-1*H*-indole.

Step 6 5-(3-Benzyl-pyrrolidin-3-yl)-1*H*-indole

A mixture of 5-(1,3-dibenzyl-pyrrolidin-3-yl)-1*H*-indole (108 mg, 0.295 mmol) in MeOH (20 mL) was shaken with $Pd(OH)_2/C$ (20%, 54 mg) under hydrogen atmosphere (60 PSI) in a Parr apparatus for 4.5 hours. The resulting mixture was filtered and concentrated under reduced pressure to a colorless residue (84 mg) that was purified via flash chromatography (2% to 20% of 9/1 $MeOH/NH_4OH$ in DCM) to give 44 mg (54% yield) of 5-(3-benzyl-pyrrolidin-3-yl)-1*H*-indole as white foam. MS = 277 $[M+H]^+$. A second batch of this material was prepared and was purified by preparative chiral HPLC (on Chiralpak AD preparative column 20 x 250 mm ID, using 80% Hexanes (0.1% DEA)/20% Ethanol at 10 ml/min flow rate) to give the 2 enantiomers: Enantiomer A off-white powder, $\alpha_D$ = +16° (c = 2.10 mg/ml MeOH); and Enantiomer B white powder, $\alpha_D$ = -34° (c = 7.28 mg/ml MeOH).

Similarly prepared using the appropriate starting material, were: 6-(3-Benzyl-pyrrolidin-3-yl)-1*H*-indole (colorless foam, 115 mg, 66% yield) MS = 277 $[M+H]^+$; and the correspondent: Enantiomer A: $\alpha_D$ = -21.1° (c = 4.8mg/mL $CHCl_3$); Enatiomer B: $\alpha_D$ = +21.0° (c = 4.2mg/mL $CHCl_3$); separated on Chiralpak IA preparative column (30 x 250 mm ID) using 70% Hexanes (0.1% DEA)/30% Ethanol at 20 ml/min.; 5-[3-(4-Fluoro-benzyl)-pyrrolidin-3-yl]-1*H*-indole (off-white foam, 50 mg, 50% yield); MS = 295 $[M+H]^+$; 5-[3-(3-Methoxy-benzyl)-pyrrolidin-3-yl]-1*H*-indole (white powder, 191 mg, 62% yield); MS = 307 $[M+H]^+$; and the correspondent: Enatiomer A: $\alpha_D$ = -17.4° (c = 4.3 mg/mL $CHCl_3$); Enatiomer B: $\alpha_D$ = +14.3 ° (c = 4.6 mg/mL $CHCl_3$); separated on Chiralpak IA preparative column (30 x 250 mm ID) using 90% Hexanes (0.1% DEA)/10% Ethanol at 20 ml/min; 5-(3-Pyridin-2-ylmethyl-pyrrolidin-3-yl)-1*H*-indole (off-white foam, 45 mg, 28% 3 steps yield); MS = 278 $[M+H]^+$; Mp = 169.9-171.1° C; 5-(3-Phenethyl-pyrrolidin-3-yl)-1*H*-indole (off-white powder, 63 mg, 32% 2 steps yield); MS = 291 $[M+H]^+$; and 5-(3-Methyl-pyrrolidin-3-yl)-1*H*-indole (white solid, 70 mg, 75% 2 steps yield), MS = 201 $[M+H]^+$.

Example 2 3-Benzo[*b*]thiophen-5-yl-3-benzyl-pyuolidine hydrochloride.

The synthetic procedure described in this Example was carried out according to the process shown in Scheme C.

## SCHEME C

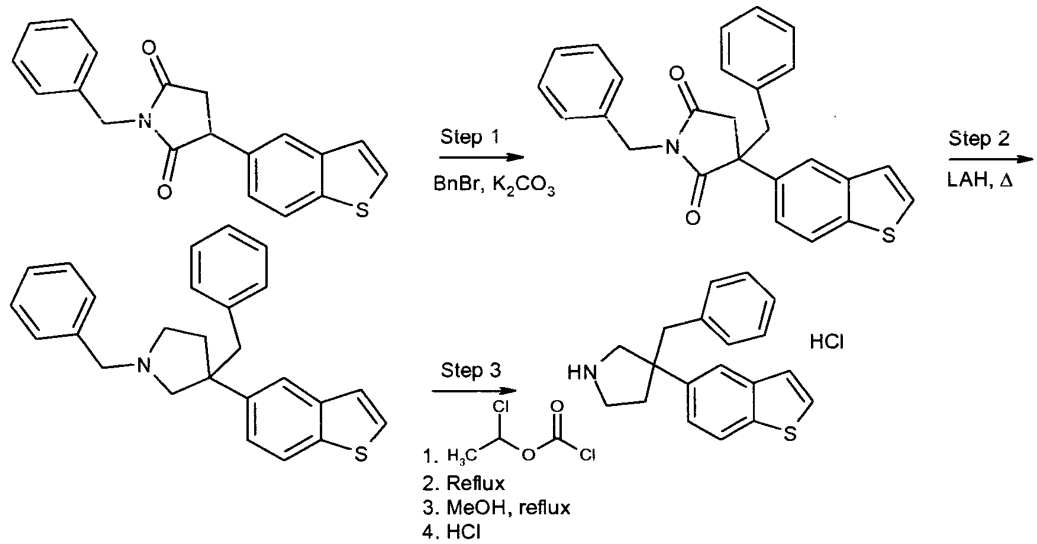


Step 1 3-Benzo[*b*]thiophen-5-yl-1,3-dibenzyl-pyrrolidine-2,5-dione.

To a stirring mixture of 3-benzo[*b*]thiophen-5-yl-1-benzyl-pyrrolidine-2,5-dione (462 mg, 1.44 mmol) and benzyl bromide (0.34 mL, 2.88 mmol) in DMF (1.5 mL) was added, at room temperature, under nitrogen atmosphere, anhydrous (freshly dried under high vacuum) $K_2CO_3$ (1.0 g). The reaction mixture was warmed at 45° C for 3 hours, it was then cooled, diluted with EtOAc (30 mL) and water (10 mL). The organic layer was separated and the aqueous was extracted twice with EtOAc (15 mL). The combined organic extracts were washed with brine (20 mL), dried over $MgSO_4$, filtered and evaporated under reduced pressure to give a yellow oil (1.0 g). This crude residue was purified via flash chromatography (0% to 30% of EtOAc in hexane) to give 444 mg (75% yield) of 3-benzo[*b*]thiophen-5-yl-1,3-dibenzyl-pyrrolidine-2,5-dione as colorless needles.

Similarly prepared using the appropriate starting material, were: 3-Benzo[*b*]thiophen-5-yl-3-benzyl-1-methyl-pyrrolidine-2,5-dione (white solid, 77% yield); 3-Benzo[*b*]thiophen-5-yl-1-benzyl-3-prop-2-ynyl-pyrrolidine-2,5-dione (yellow gum, 325 mg, 75% yield).

Step 2 3-Benzo[*b*]thiophen-5-yl-1,3-dibenzyl-pyrrolidine.

A solution of lithium aluminum hydride (1.0 M solution in THF, 4.2 mL) was added dropwise, under nitrogen atmosphere, at room temperature, to a stirred solution of 3-benzo[*b*]thiophen-5-yl-1,3-dibenzyl-pyrrolidine-2,5-dione (428 mg, 1.04 mmol) in THF (10 mL). The reaction mixture was heated at 80°C for 2 hours; it was then cooled at room temperature and quenched by addition of $Na_2SO_4$. $10H_2O$ (ca. 3 g). The mixture was filtered and the filtrate was concentrated under reduced pressure. The crude residue was purified via flash chromatography (5% to 50% of EtOAc in hexane) to give 3-benzo[*b*]thiophen-5-yl-1,3-dibenzyl-pyrrolidine (374 mg, 81% yield) as a colorless film.

3-Benzo[*b*]thiophen-5-yl-1-benzyl-3-prop-2-ynyl-pyrrolidine was prepared in a similar manner using the appropriate starting material (colorless gum, 232 mg, 79% yield).

Step 3 3-Benzo[*b*]thiophen-5-yl-3-benzyl-pyrrolidine hydrochloride.

1-Chloroethyl chloroformate (56 μL, 0.509 mmol) was added, at 0°C under nitrogen atmosphere, to a stirred solution of 3-benzo[*b*]thiophen-5-yl-1,3-dibenzyl-pyrrolidine (130 mg, 0.339 mmol) in 1,2-dichloroethane (3 mL). After stirring for 15 minutes the mixture was warmed to room temperature, it was then heated at 80° C for 3 hours. The resulting mixture was concentrated under reduced pressure, MeOH (2 mL) was then added to the residue and the mixture was refluxed for 1 hour. The solvent was then evaporated under reduced pressure and the crude residue was purified via flash chromatography (0% to 20% of 9/1 mixture of $MeOH/NH_4OH$ in DCM) to give 14 mg of 3-benzo[*b*]thiophen-5-yl-3-benzyl-pyrrolidine as a yellow oil. This material was dissolved in DCM (0.5 mL) and a solution HCl (1 M in $Et_2O$, 50 μL) was added. The mixture was concentrated under reduced pressure and the foamy, off-white residue was dried

under high vacuum to give 17 mg (15% yield) of 3-benzo[*b*]thiophen-5-yl-3-benzyl-pyrrolidine hydrochloride. Mp = 120.9 ˚ -123.0 ˚; MS = 294 $[M+H]^+$. 3-Benzo[*b*]thiophen-5-yl-3-prop-2-ynyl-pyrrolidine hydrochloride was prepared in a similar manner using the appropriate starting material (crisp off-white foam, 158 mg, 81% yield); MS = 242 $[M+H]^+$.

Example 3 5-(3-Benzyl-pyrrolidin-3-yl)-1*H*-indazole.

The synthetic procedure described in this Example was carried out according to the process shown in Scheme D.

## SCHEME D

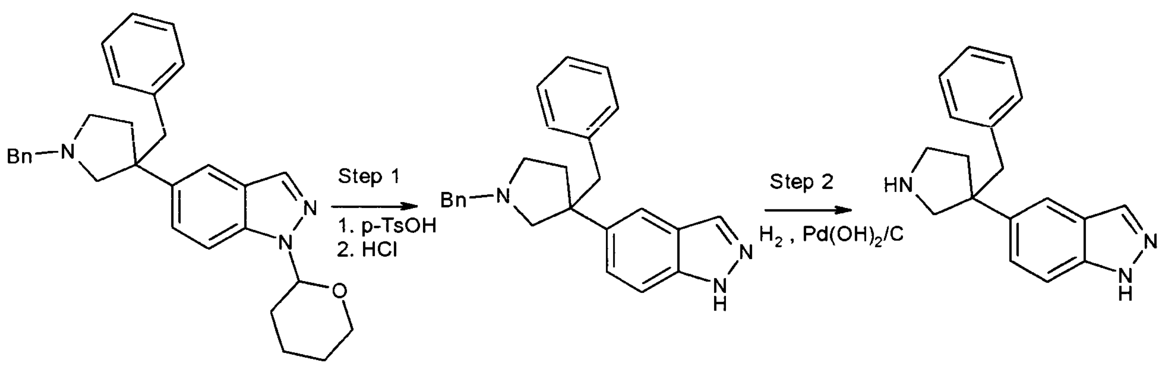


Step 1 5-(1,3-Dibenzyl-pyrrolidin-3-yl)-1*H*-indazole.

p-Toluenesulfonic acid (218 mg, 1.15 mmol) was added, at room temperature, to a stirred solution of 5-(1,3-dibenzyl-pyrrolidin-3-yl)-1-(tetrahydro-pyran-2-yl)-1*H*-indazole (prepared in a similar manner as described in Example 1) (259 mg, 0.574 mmol); and the clear, light yellow resulting solution was stirred for 2 hours. A solution of HCl (2 M in water, 4 mL) was then added and the mixture was warmed to 50˚ C for 2 hours; it was then cooled and quenched by addition of a solution of NaOH (10% in water, 20 mL). The resulting mixture was extracted 3 times with EtOAc (30 mL); the combined organic extracts were washed with brine (20 mL) dried over $MgSO_4$, filtered and evaporated under reduced pressure to give a yellow residue (190 mg). The crude material was purified via flash chromatography (2% to 20% ofMeOH in DCM) to give 133 mg (63% yield) of 5-(1,3-dibenzyl-pyrrolidin-3-yl)-1*H*-indazole as a light yellow oil.

Step 2 5-(3-Benzyl-pyrrolidin-3-yl)-1*H*-indazole.

5-(1,3-Dibenzyl-pyrrolidin-3-yl)-1*H*-indazole was hydrogenated following the procedure described in Example 1, Step 6; to give 53 mg (53% yield) of 5-(3-benzyl-pyrrolidin-3-yl)-1*H*-indazole as a white foam. MS = 278 $[M+H]^+$.

Example 4 [4-(3-Benzyl-pyrrolidin-3-yl)-phenyl]-methyl-amine hydrochloride

The synthetic procedure described in this Example was carried out according to the process shown in Scheme E.

## SCHEME E

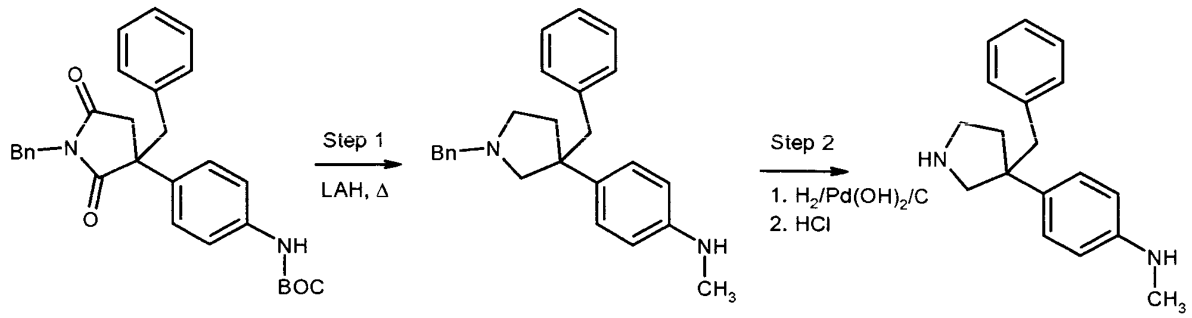


Step 1 [4-(1,3-Dibenzyl-pyrrolidin-3-yl)-phenyl]-methyl-amine

A solution of lithium aluminum hydride (1.0 M in THF, 1.70 mL) was added, at room temperature, under nitrogen atmosphere, to a stirred solution of [4-(1,3-dibenzyl-2,5-dioxo-pyrrolidin-3-yl)-phenyl]-carbamic acid *tert*-butyl ester (prepared following the procedure described in Example 1) (200 mg, 0.426 mmol) in THF (5 mL). The reaction mixture was heated at 80° C for 2 hours, it was cooled and stirred for 15 hours, and then it was quenched by addition of $Na_2SO_4$. $10H_2O$ (ca. 1 g). The mixture was filtered and the inorganic salts were washed twice with EtOAc (20 mL) and once with a mixture DCM/MeOH (9/1). The filtrate was concentrated under reduced pressure to give [4-(1,3-dibenzyl-pyrrolidin-3-yl)-phenyl]-methyl-amine (165 mg) that was used without any further purification.

Step 2 [4-(3-Benzyl-pyrrolidin-3-yl)-phenyl]-methyl-amine bishydrochloride

A mixture of [4-(1,3-dibenzyl-pyrrolidin-3-yl)-phenyl]-methyl-amine (ca. 0.426 mmol) in MeOH (10 mL) was shaken with $Pd(OH)_2/C$ (20%, 120 mg) under hydrogen atmosphere (60 PSI) in a Parr apparatus for 8 hours. The resulting mixture was filtered and concentrated under reduced pressure to a dark brown oil that was purified via flash chromatography (1% to 20% of 9/1 MeOH/$NH_4OH$ in DCM) to give 50 mg of [4-(3-benzyl-pyrrolidin-3-yl)-phenyl]-methylamine as a brown oil. This material was dissolved in a mixture of DCM/MeOH (9/1, 2 mL) and a solution of HCl (1 M in $Et_2O$, 1.0 mL) was added to it. The resulting mixture was concentrated under reduced pressure to give 64 mg (44% yield) of the bishydrochloride salt of the title compound as a light brown foam. MS = 267 $[M+H]^+$.

Example 5 *N*-[4-(3-Benzyl-pyrrolidin-3-yl)-phenyl]-acetamide

The synthetic procedure described in this Example was carried out according to the process shown in Scheme F.

## SCHEME F

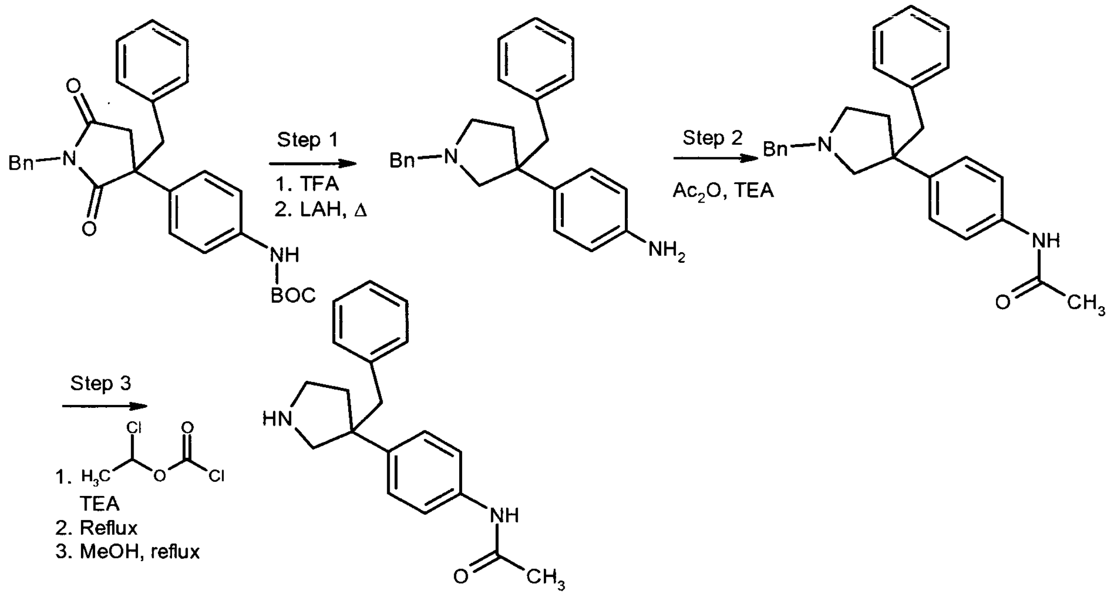


Step1 4-(1,3-Dibenzyl-pyrrolidin-3-yl)-phenylamine

Trifluoroacetic acid (5.2 mL, 67.4 mmol) was added, at room temperature, to a stirring solution of [4-(1,3-dibenzyl-2,5-dioxo-pyrrolidin-3-yl)-phenyl]-carbamic acid *tert*-butyl ester (prepared following the procedure described in Example 1) (3.17 g, 6.74 mmol) in DCM (10 mL). After stirring for 4 hours the reaction mixture was diluted with DCM (40 mL) and a solution ofLiOH (1 M in water, 100 mL) was added. The yellow organic phase was separated; the aqueous phase was extracted 3 times with DCM (40 mL). The combined organic extracts were washed with a solution ofLiOH (1 M in water, 50 mL), brine (40 mL), dried over $MgSO_4$, filtered and evaporated under reduced pressure to give 3-(4-amino-phenyl)-1,3-dibenzyl-pyrrolidine-2,5-dione as a yellow gum (2.53 g, quantitative yield). This crude material (2.49 g, 6.73 mmol) was dissolved in THF (16 mL) and, at room temperature, under nitrogen atmosphere, a solution of lithium aluminum hydride (1.0 M in THF, 16.1 mL) was added dropwise. The reaction mixture was heated at 80° C for 3 hours, it was then cooled and quenched by addition of $Na_2SO_4$. $10H_2O$ (ca. 2 g). The mixture was diluted with EtOAc (150 mL), filtered and concentrated under reduced pressure to give 2.1 g (91% yield) of 4-(1,3-dibenzyl-pyrrolidin-3-yl)-phenylamine as a yellow solid.

Step 2 *N*-[4-(1,3-Dibenzyl-pyrrolidin-3-yl)-phenyl]-acetamide

Acetic anhydride (0.32 mL, 3.36 mmol) and triethylamine (0.47 mL, 3.36 mmol) were added to a stirring solution of 4-(1,3-dibenzyl-pyrrolidin-3-yl)-phenylamine (0.50 g, 1.46 mmo) in chloroform (20 mL). After 1.5 hour the reaction mixture was diluted with chloroform (30 mL) and washed twice with water (10 mL), once with brine (20 mL), dried over $MgSO_4$, filtered and evaporated under reduced pressure to a colorless gum. This crude material was purified via flash chromatography (0% to 5% ofMeOH in DCM) to give 0.487 mg (87% yield) of *N*-[4-(1,3-dibenzyl-pyrrolidin-3-yl)-phenyl]-acetamide as a glassy solid.

Step 3 *N*-[4-(3-Benzyl-pyrrolidin-3-yl-phenyl]-acetamide

1-Chloroethyl chloroformate (57 μL, 0.520 mmol) and triethylamine (40 μL, 0.520 mmol) were added, at room temperature, under nitrogen atmosphere, to a stirred solution of *N*-[4-(1,3-dibenzyl-pyrrolidin-3-yl)-phenyl]-acetamide (100 mg, 0.260 mmol) in 1,2-dichloroethane (2 mL). After stirring for 30 minutes the reaction mixture was concentrated under reduced pressure. The yellow residue was dissolved in MeOH (2 mL) and the resulting mixture was warmed to 50° C for 45 minutes. The solvent was then evaporated under reduced pressure and the crude residue was purified via flash chromatography (0% to 20% of 9/1 mixture of $MeOH/NH_4OH$ in DCM) to give 58 mg of *N*-[4-(3-benzyl-pyrrolidin-3-yl)-phenyl]-acetamide as a colorless white foamy solid. MS = 295 $[M+H]^+$.

Example 6 5-(3-Benzyl-pymolidin-3-yl-1*H*-indole-2-carbonitrile trifluoroacetate and 5-(3-Benzyl-pyrrolidin-3-yl)-1*H*-indole-2-carboxylic acid *tert*-butylamide trifluoroacetate

The synthetic procedure described in this Example was carried out according to the process shown in Scheme G.

**SCHEME G**

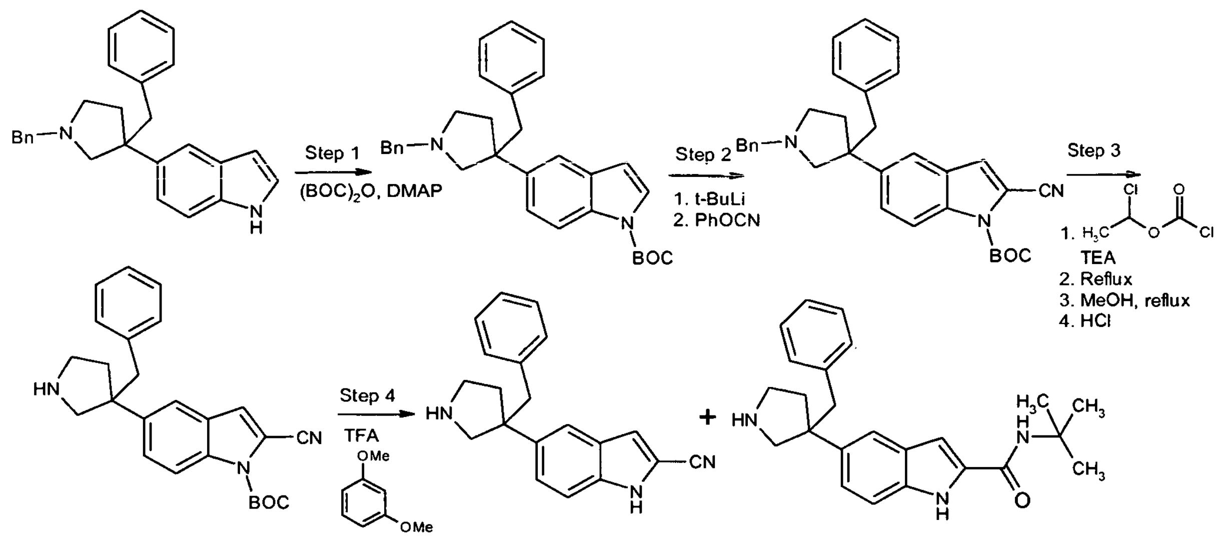


Step 1 5-(1,3-Dibenzyl-pyrrolidin-3-yl)-indole-1-carboxylic acid *tert*-butyl ester.

To a stirred solution of 5-(1,3-dibenzyl-pyrrolidin-3-yl)-1*H*-indole (1.50 g, 4.10 mmol) and 4-dimethylaminopyridine (25 mg, 0.205 mmol) in THF (41 mL) was added, at room temperature, under nitrogen atmosphere, di-*tert*-butyl dicarbonate (0.941 g, 4.31 mmol) followed by triethylamine (0.60 mL, 4.31 mmol). After stirring 2 hours at room temperature, under nitrogen atmosphere, a second aliquot of di-*tert*-butyl dicarbonate (0.298 g, 1.37 mmol) and triethylamine (0.191 mL, 1.37 mmol) was added. After stirring for a further hour a third aliquot of di-*tert*-butyl dicarbonate (0.47 g, 2.15 mmol) and triethylamine (0.30 mL, 2.15 mmol) was added and the resulting clear solution was stirred for additional 16 hours. The reaction mixture was evaporated under reduced pressure; the residue was diluted with water (40 mL) and extracted 3 times with EtOAc (40 mL). The combined organic extracts were washed with brine (40 mL), dried over $MgSO_4$, filtered and evaporated under reduced pressure to give a yellow oil. This crude material was purified via flash chromatography (0% to 30% of EtOAc in hexane) to give 1.45 g (76% yield) of 5-(1,3-dibenzyl-pyrrolidin-3-yl)-indole-1-carboxylic acid *tert*-butyl ester as a white solid.

Step 2 2-Cyano-5-(1,3-dibenzyl-pyrrolidin-3-yl)-indole-1-carboxylic acid *tert*-butyl ester

*tert*-Butyl lithium (1.55 M in pentane, 0.97 mL, 1.50 mmol) was added dropwise, under nitrogen atmosphere, at -75° C, to a stirred solution of 5-(1,3-dibenzyl-pyrrolidin-3-yl)-indole-1-carboxylic acid *tert*-butyl ester (500 mg, 1.07 mmol) in THF (20 mL). After stirring for 30 minutes a solution of phenyl cyanate (0.190 g, 1.61 mmol) in THF (2 mL) was added. After stirring for 30 minutes at -75° C the reaction mixture was warmed to 0° C over a period of 30 minutes; it was then warmed up to room temperature and quenched by addition of a saturated aqueous solution of $NH_4Cl$ (20 mL). The resulting mixture was extracted 3 times with EtOAc (20 mL). The combined organic extracts were washed with brine (15 mL), dried over $MgSO_4$, faltered and evaporated under reduced pressure to give a yellow oil (0.8 g). This crude material It was purified via flash chromatography (0% to 3% of MeOH in DCM) to give 212 mg (40% yield) of 2-cyano-5-(1,3-dibenzyl-pyrrolidin-3-yl)-indole-1-carboxylic acid *tert*-butyl ester. MS = 492 $[M+H]^+$.

Step 3 5-(3-Benzyl-pyrrolidin-3-yl-2-cyano-indole-1-carboxylic acid *tert*-butyl ester

1-Chloroethyl chloroformate (80 μL, 0.733 mmol) was added, at room temperature, under nitrogen atmosphere, to a stirred solution of 2-cyano-5-(1,3-dibenzyl-pyrrolidin-3-yl)-indole-1-carboxylic acid *tert*-butyl ester (200 mg, 0.407 mmol) in 1,2-dichloroethane (3 mL); followed by triethylamine (57 μL, 0.407 mmol). After stirring for 15 minutes the

cloudy yellow mixture was concentrated under reduced pressure, MeOH (2 mL) was then added to the yellow residue and the resulting mixture was heated at 60° C, under nitrogen atmosphere, for 30 minutes. The clear yellow solution was then evaporated under reduced pressure and the pale yellow resulting gum (310 mg) was purified via flash chromatography (0% to 10% of 9/1 mixture of MeOH/$NH_4OH$ in DCM) to give 98 mg (60% yield) of 5-(3-benzyl-pyrrolidin-3-yl)-2-cyano-indole-1-carboxylic acid *tert*-butyl ester as a colorless foamy gum.

Step 4 5-(3-Benzyl-pyrrolidin-3-yl)-1*H*-indole-2-carbonitrile

Trifluoroacetic acid (0.52 mL, 6.73 mmol) was added, at room temperature, under nitrogen atmosphere, to a stirred solution of 5-(3-benzyl-pyrrolidin-3-yl)-2-cyano-indole-1-carboxylic acid *tert*-butyl ester (135 mg, 0.337 mmol) and 1,3-dimethoxybenzene in DCM (0.5 mL) for 1 hour. The reaction mixture was concentrated under reduced pressure onto silica gel and purified via flash chromatography (0% to 20% of 9/1 mixture of MeOH/$NH_4OH$ in DCM) to give 68 mg of an off-white foam that was subsequently repurified via preparative HPLC (85% water + 0.1 % TFA/15% Acetonitrile to 5%/95% gradient over 12 minutes, on a Gemini Phenyl column 50 x 20 mm ID (Phenomenex Corp.)) to give 5-(3-benzyl-pyrrolidin-3-yl)-1*H*-indole-2-carbonitrile trifluoroacetate (crystalline white solid) and 5-(3-benzyl-pyrrolidin-3-yl)-1*H*-indole-2-carboxylic acid *tert*-butylamide trifluoroacetate (white solid).

Example 7 5-(3-Benzyl-pyrrolidin-3-yl)-1*H*-indole-2-carboxylic acid amide

The synthetic procedure described in this Example was carried out according to the process shown in Scheme H.

## SCHEME H

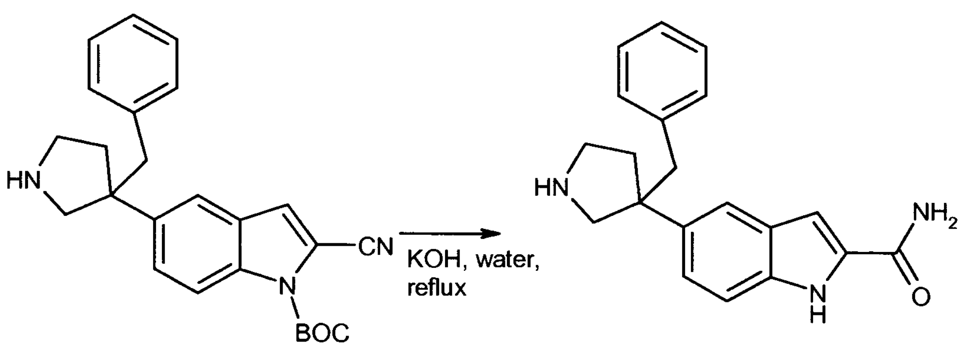


A solution of KOH (77 mg, 1.38 mmol) in water (0.2 mL) was added to a stirring solution of 5-(3-benzyl-pyrrolidin-3-yl)-2-cyano-indole-1-carboxylic acid *tert*-butyl ester (138 mg, 0.344 mmol) in EtOH (10 mL) and the resulting mixture was heated at reflux for 18 hours. The reaction mixture was concentrated under reduced pressure and the crude residue was purified via flash chromatography (0% to 20% of 9/1 mixture of MeOH/$NH_4OH$ in DCM) to give 50 mg (48% yield) of 5-(3-benzyl-pyrrolidin-3-yl)-1*H*-indole-2-carbonitrile and 7 mg (6% yield) of the desired primary amide. The nitrile was dissolved in EtOH (3 mL) and water (0.1 mL) and KOH (37 mg, 0.664 mmol) was added. The resulting mixture was heated at 80° C for 16 hours; a second aliquot of KOH (74 mg) and water (0.2 mL) was added and heating continued. After 8 hours the reaction mixture was concentrated under reduced pressure and the crude residue was purified via flash chromatography (0% to 20% of 9/1 mixture of MeOH/$NH_4OH$ in DCM) to give 18 mg (34% yield) of 5-(3-benzyl-pyrrolidin-3-yl)-1*H*-indole-2-carboxylic acid amide as an off-white powder. MS = 320 $[M+H]^+$.

Example 8 5-(3-Benzyl-pyrrolidin-3-yl)-1*H*-indole-2-carboxylic acid methylamide

The synthetic procedure described in this Example was carried out according to the process shown in Scheme I.

## SCHEME I

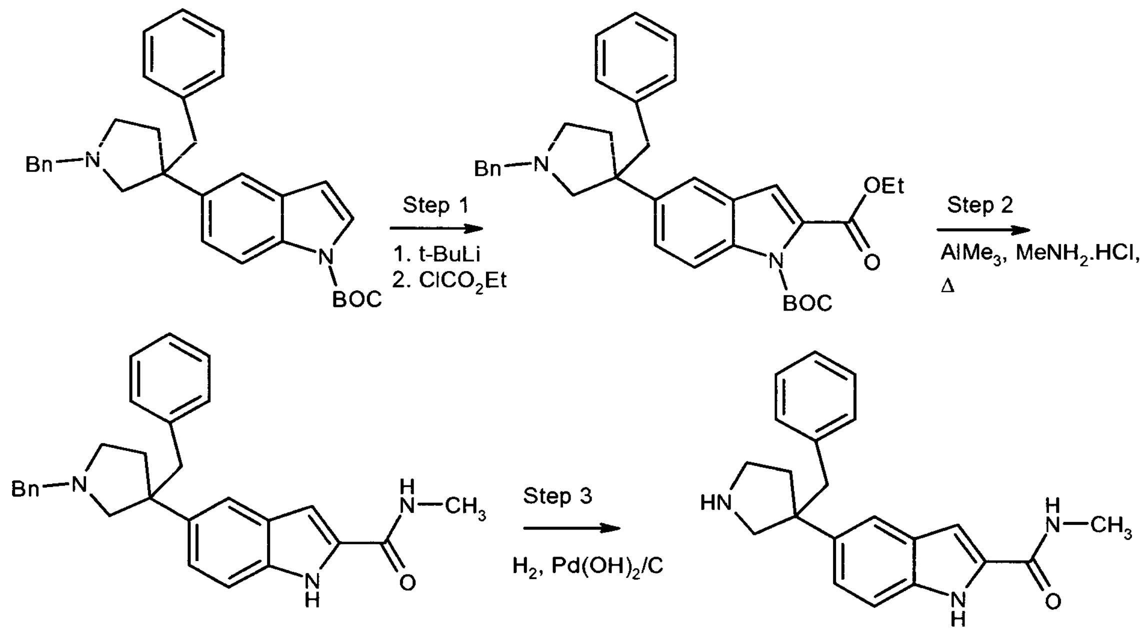


Step 1 5-(1,3-Dibenzyl-pyrrolidin-3-yl)-indole-1,2-dicarboxylic acid 1-*tert*-butyl ester 2-ethyl ester

A cooled (-78° C) solution of *tert*-butyllithium (1.33 M in pentane, 1.63 mL, 2.17 mmol) was added dropwise, under nitrogen atmosphere, at -78° C, to a stirred solution of 5-(1,3-dibenzyl-pyrrolidin-3-yl)-indole-1-carboxylic acid *tert*-butyl ester (674 mg, 1.45 mmol) in THF (25 mL) over a 5 minutes period. After stirring for 1.5 hours a solution of ethyl chloroformate (0.21 mL, 2.17 mmol) in THF (2 mL) was added dropwise over a period of 2 minutes. After stirring for 30 minutes the reaction was quenched by addition of a saturated aqueous solution of $NH_4Cl$ (20 mL) and the resulting mixture was warmed to room temperature. The organic layer was separated and the aqueous layer was extracted 3 times with EtOAc (20 mL). The combined organic extracts were washed with brine (20 mL), dried over $MgSO_4$, filtered and evaporated under reduced pressure to give a yellow oil (0.8 g). This crude material was purified via flash chromatography (5% to 55% of EtOAc in hexane) to give 587 mg (75% yield) of 5-(1,3-dibenzyl-pyrrolidin-3-yl)-indole-1,2-dicarboxylic acid 1-*tert*-butyl ester 2-ethyl ester as light yellow gum.

Step 2 5-(1,3-Dibenzyl-pyrrolidin-3-yl)-1*H*-indole-2-carboxylic acid methylamide

A solution oftrimethylaluminum (2.0 M in toluene, 0.377 mL, 0.753 mmol) was added dropwise at room temperature to a stirred suspension of methylamine hydrochloride (50.8 mg, 0.753 mmol) in toluene (1 mL). The mixture was stirred for 1 hour, then a solution of 5-(1,3-dibenzyl-pyrrolidin-3-yl)-indole-1,2-dicarboxylic acid 1-*tert*-butyl ester 2-ethyl ester (135 mg, 0.251 1 mmol) in toluene (1.5 mL) was added and the resulting mixture was heated at 80° C for 18 hours. The reaction mixture was quenched by the addition of water (0.5 mL) and it was diluted with EtOAc (15 mL). The organic layer was separated, dried over $MgSO_4$, filtered and evaporated under reduced pressure to give 152 mg of 5-(1,3-dibenzyl-pyrrolidin-3-yl)-1*H*-indole-2-carboxylic acid methylamide as a yellow solid without further purifications. MS = 424 $[M+H]^+$.

5-(1,3-Dibenzyl-pyrrolidin-3-yl)-1*H*-indole-2-carboxylic acid dimethylamide was prepared in a similar manner using dimethylamine hydrochloride (94 mg, 32% yield).

Step 3 5-(3-Benzyl-pyrrolidin-3-yl)-1*H*-indole-2-carboxylic acid methylamide

A solution of 5-(1,3-dibenzyl-pyrrolidin-3-yl)-1*H*-indole-2-carboxylic acid methylamide (ca. 0.251 mmol) in MeOH (20 mL) was shaken with $Pd(OH)_2/C$ (20%, 150 mg) under hydrogen atmosphere (60 PSI) in a Parr apparatus for 6 hours. The resulting mixture was filtered and concentrated under reduced pressure to give a yellow residue (105 mg) that was purified via flash chromatography (5% to 20% of 9/1 $MeOH/NH_4OH$ in DCM) to give 11 mg (13% 2 steps yield) of 5-(3-benzyl-pyrrolidin-3-yl)-1*H*-indole-2-carboxylic acid methylamide as an off-white powder. MS = 334 $[M+H]^+$.

5-(3-Benzyl-pyrrolidin-3-yl)-1*H*-indole-2-carboxylic acid dimethylamide was prepared in a similar manner using the appropriate starting material (38 mg, 69% yield, white foam).

Example 9 5-(3-Benzyl-pyrrolidin-3-yl)-1*H*-pyrrolo[2,3-*b*]pyridine

The synthetic procedure described in this Example was carried out according to the process shown in Scheme J.

**SCHEME J**

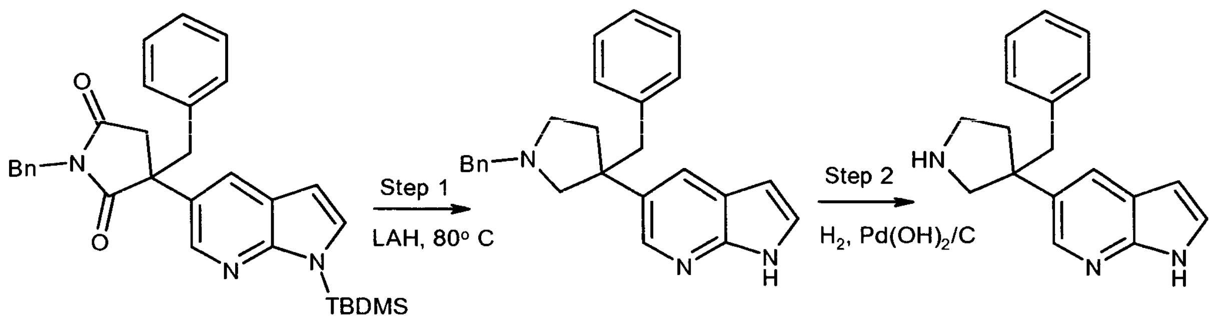


Step 1 5-(1,3-Dibenzyl-pyrrolidin-3-yl)-1*H*-pyrrolo[2,3-*b*]pyridine

A solution of lithium aluminum hydride (1.0 M in THF, 1.27 mL) was added dropwise, at room temperature under nitrogen atmosphere, to a stirred solution of 1,3-dibenzyl-3-[1-(*tert-b*utyldimethyl-silanyl)-1*H*-pyrrolo[2,3-*b*]pyridin-5-yl]-pyrrolidine-2,5-dione (prepared following the procedure described in Example 1) (162 mg, 0.318 mmol) in THF (5 mL). The reaction mixture was heated at 80° C for 1.5 hour; it was then cooled and quenched by addition of $Na_2SO_4$. $10H_2O$ (ca. 1 g). The mixture was filtered and the inorganic salts were washed 3 times with a mixture DCM/MeOH/$NH_4OH$ (9/1/0/1, 10 mL). The filtrate was concentrated under reduced pressure to give 160 mg of an off-white residue, which was purified by flash chromatography (0% to 10% of 9/1 MeOH/$NH_4OH$ in DCM) to give 78 mg (67% yield) of 5-(1,3-dibenzyl-pyrrolidin-3-yl)-1*H*-pyrrolo[2,3-*b*]pyridine as a colorless gum.

Step 2 5-(3-Benzyl-pyrrolidin-3-yl)-1*H*-pyrrolo[2,3-*b*]pyridine

A mixture of 5-(1,3-dibenzyl-pyrrolidin-3-yl)-1*H*-pyrrolo[2,3-*b*]pyridine (78 mg, 0.213 mmol) in MeOH (10 mL) was shaken with $Pd(OH)_2$/C (20%, 78 mg) under hydrogen atmosphere (60 PSI) in a Parr apparatus for 18 hours. The resulting mixture was filtered and concentrated under reduced pressure to give a yellow film (58 mg) that was purified via flash chromatography (10 to 20% of 9/1 MeOH/$NH_4OH$ in DCM) to give 45 mg (76% yield) of 5-(3-benzyl-pyrrolidin-3-yl)-1*H*-pyrrolo[2,3-*b*]pyridine as a white foam. MS = 278 $[M+H]^+$.

Example 10 3-(3-Benzyl-pyrrolidin-3-yl)-1*H*-indole hydrochloride

The synthetic procedure described in this Example was carried out according to the process shown in Scheme K.

## SCHEME K

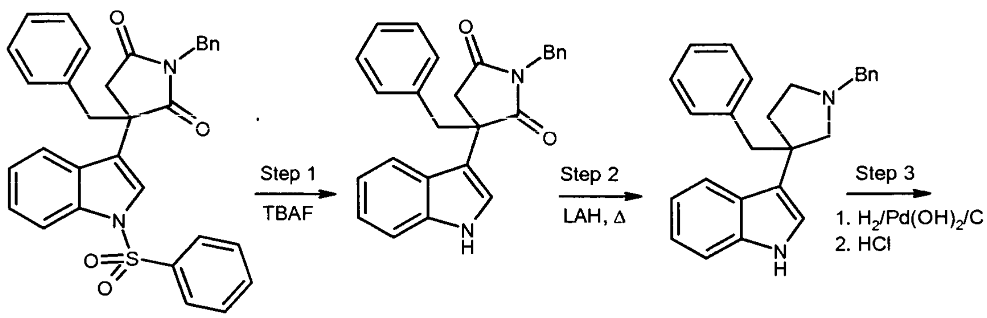


Step 1,3-Dibenzyl-3-(1*H*-indol-3-yl)-pyrrolidine-2,5-dione

A solution of tetrabutylammoniumfluoride (1.0 M in THF, 4.24 mL) was added, at room temperature, under nitrogen atmosphere, to a stirred solution of 3-(1-benzenesulfonyl-1*H*-indol-3-yl)-1,3-dibenzyl-pyrrolidine-2,5-dione (prepared following the procedure described in Example 1) (0.687 g, 1.29 mmol). The clear colorless solution became red in color immediately. After stirring for 6 hours, at room temperature, the reaction mixture was heated at 75° C. After 1.5 hour the resulting mixture was concentrated under reduced pressure, the residue was diluted with water (30 mL) and extracted 3 times with EtOAc (30 mL). The combined organic extracts were washed with a saturated aqueous solution of $NaHCO_3$ (20 mL) and with brine (20 mL), then dried over $MgSO_4$, filtered and evaporated under reduced pressure to give a brown oil. This crude residue was purified via flash chromatography (0% to 40% of EtOAc in hexane) to give 177 mg (35% yield) of 1,3-dibenzyl-3-(1*H*-indol-3-yl)-pyrrolidine-2,5-dione as a white foam. MS = 395$[M+H]^+$.

Step 2 3-(1,3-Dibenzyl-pyrrolidin-3-yl)-1*H*-indole

A solution of lithium aluminum hydride (1.0 M solution in THF, 1.29 mL) was added dropwise, under nitrogen atmosphere, at room temperature, to a stirred solution of 1,3-dibenzyl-3-(1H indol-3-yl)-pyrrolidine-2,5-dione (170 mg, 0.431 mmol) in THF (5 mL). The reaction mixture was heated at 70° C for 2 hours, it was then cooled at room temperature and quenched by addition of $Na_2SO_4.10H_2O$ (ca. 0.5 g). The mixture was diluted with EtOAc (20 mL) and filtered; the inorganic salts were washed 3 times with EtOAc (10 mL). The filtrate was concentrated under reduced pressure to give 3-(1,3-dibenzyl-pyrrolidin-3-yl)-1*H*-indole (166 mg, quantitative yield, colorless oil) which was used without further purifications.

Step 3 3-(3-Benzyl-pyrrolidin-3-yl)-1*H*-indole hydrochloride

A mixture of 3-(1,3-dibenzyl-pyrrolidin-3-yl)-1*H*-indole (ca. 0.431 mmol) in MeOH (20 mL) was shaken with Pd $(OH)_2$/C (20%, 100 mg) under hydrogen atmosphere (60 PSI) in a Parr apparatus for 4 hours. The resulting mixture was filtered and concentrated under reduced pressure to a light yellow residue (106 mg) that was purified via flash chromatography (0% to 10% of 9/1 MeOH/$NH_4OH$ in DCM) to give 51 mg (43% 2 steps yield) of 3-(3-benzyl-pyrrolidin-3-yl)-1*H*-indole as a yellow foam. This material was dissolved in DCM (0.5 mL) and a solution of HCl (1 M in $Et_2O$, 0.185 mL) was added; the mixture was concentrated under reduced pressure to give 59 mg (quantitative yield) of the hydrochloride salt of the title compound as a pale yellow foam. MS = 277 $[M+H]^+$.

Example 11 5-(3-Benzyl-pyrrolidin-3-yl)-1*H*-indole-2-carboxylic acid ethyl ester hydrochloride

The synthetic procedure described in this Example was carried out according to the process shown in Scheme L.

SCHEME L

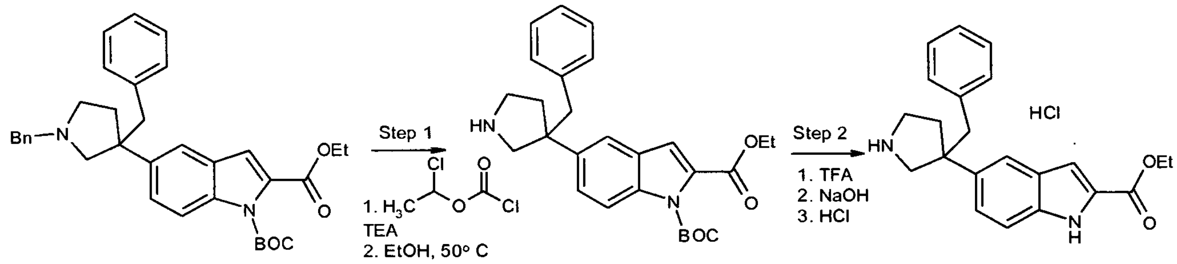


Step 1 5-(3-Benzyl-pyrrolidin-3-yl)-indole-1,2-dicarboxylic acid 1-*tert*-butyl ester 2-ethyl ester

1-Chloroethyl chloroformate (97 μL, 0.680 mmol) was added, at room temperature, under nitrogen atmosphere, to a stirred solution of 5-(1,3-dibenzyl-pyrrolidin-3-yl)-indole-1,2-dicarboxylic acid 1-*tert*-butyl ester 2-ethyl ester (183 mg, 0.340 mmol) in 1,2-dichloroethane (4 mL), followed by triethylamine (47 μL, 0.340 mmol). After stirring for 30 minutes the mixture was concentrated under reduced pressure, EtOH (4 mL) was then added and the resulting mixture was heated at 50° C, under nitrogen atmosphere, for 45 minutes. The reaction mixture was then evaporated under reduced pressure and the crude residue was purified via flash chromatography (5% to 20% of MeOH in DCM) to give 125 mg (82% yield) of 5-(3-benzyl-pyrrolidin-3-yl)-indole-1,2-dicarboxylic acid 1-*tert*-butyl ester 2-ethyl ester as a pale yellow oil. MS = 449 $[M+H]^+$.

Step 2 5-(3-Benzyl-pyrrolidin-3-yl)-1*H*-indole-2-carboxylic acid ethyl ester hydrochloride

Trifluoroacetic acid (1 mL) was added, at 0° C under nitrogen atmosphere, to a stirring solution of 5-(3-benzyl-pyrrolidin-3-yl)-indole-1,2-dicarboxylic acid 1-*tert*-butyl ester 2-ethyl ester (125 mg, 0.279 mmol) in DCM (5 mL). The color of the mixture turned form yellow to blue. After stirring for 15 minutes at 0° C it was warmed to room temperature and it was stirred for 45 minutes. An aqueous solution of NaOH (2 M, 20 mL) was then added and the resulting mixture was extracted 3 times with DCM (20 mL). The combined organic extracts were washed with brine (20 mL), dried over $MgSO_4$, filtered and evaporated under reduced pressure to give a pale yellow residue (150 mg). This crude material was purified via flash chromatography (0% to 20% of a mixture of 9/1 MeOH/$NH_4OH$ in DCM) to give 41 mg of 5-(3-benzyl-pyrrolidin-3-yl)-1*H*-indole-2-carboxylic acid ethyl ester. A portion of this product (20 mg) was dissolved in DCM (1 mL) and a solution of HCl (1 M in $Et_2O$, 0.09 mL) was added; the mixture was concentrated under reduced pressure to give 20 mg of the hydrochloride salt of the title compound as an off-white powder. MS = 349 $[M+H]^+$.

Example 12 5-(3-Benzyl-pyrrolidin-3-yl)-1*H*-indole-2-carboxylic acid methyl ester hydrochloride

The synthetic procedure described in this Example was carried out according to the process shown in Scheme M.

SCHEME M

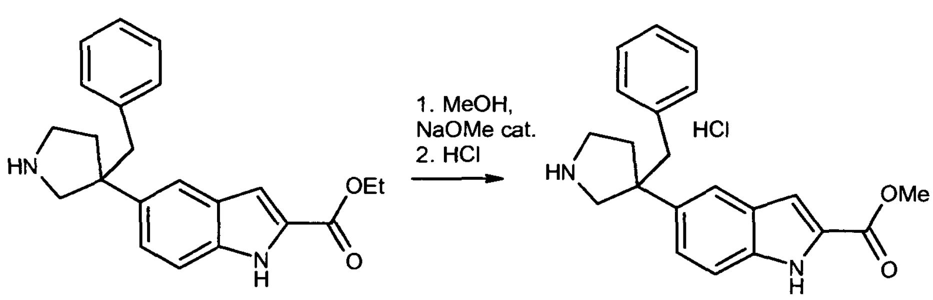


A stirring solution of 5-(3-benzyl-pyrrolidin-3-yl)-1*H*-indole-2-carboxylic acid ethyl ester (21 mg, 60.3 μmol) and NaOMe (2 mg) in MeOH (5 mL) was heated at 70° C for 48 hours. The mixture was then concentrated under reduced pressure to a colorless residue which was triturated with chloroform (5 mL); the supernatant was filtered and the filtrate

was evaporated under reduced pressure to an off-white foam (16 mg). This material was dissolved in DCM (0.5 mL) and a solution of HCl (1 M in $Et_2O$, 0.07 mL) was added; the mixture was concentrated under reduced pressure to give 16 mg (72% yield) of 5-(3-benzyl-pyrrolidin-3-yl)-1*H*-indole-2-carboxylic acid methyl ester hydrochloride as an off-white powder. MS = 335 $[M+H]^+$.

Example 13 1-[5-(3-Benzyl-pyrrolidin-3-yl)-1*H*-indol-2-yl]-2,2,2-trifluoro-ethanone

The synthetic procedure described in this Example was carried out according to the process shown in Scheme N.

**SCHEME N**

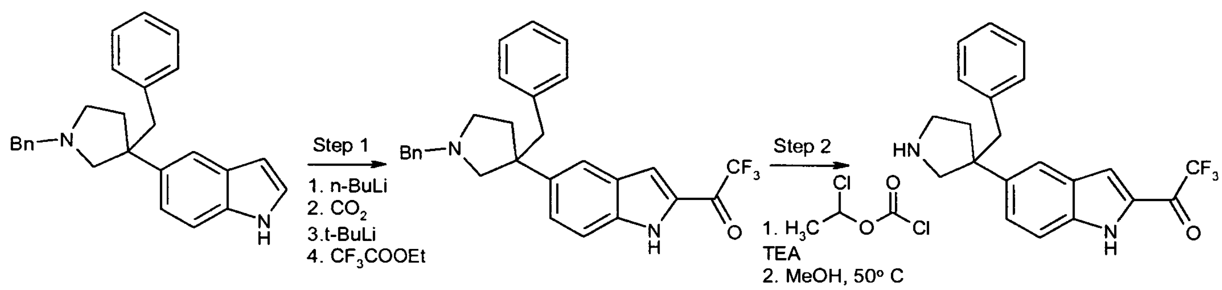


Step 1 1-[5-(1,3-Dibenzyl-pyrrolidin-3-yl-1*H*-indol-2-yl]-2,2,2-trifluoro-ethanone

n-Butyl lithium (2.21 M in hexane, 0.63 mL, 1.40 mmol) was added dropwise, at -70° C, under nitrogen atmosphere, to a stirring solution of 5-(1,3-dibenzyl-pyrrolidin-3-yl)-1*H*-indole (0.50 g, 1.37 mmol) in THF (5 mL). After stirring for 30 minutes $CO_2$ (gaseous) was bubbled for 10 minutes at -70° C and then the mixture was warmed to room temperature while continuing the $CO_2$ bubbling. To ensure complete quenching a small pellet of solid $CO_2$ was added. After bubbling had ceased the mixture was evaporated under a flow of nitrogen and then under reduced pressure. The resulting yellow foam was dissolved in THF (5 mL) and cooled to -70° C, *tert*-butyl lithium (1.44 M in pentane, 0.98 mL, 1.41 mmol) was then added dropwise over a period of 20 minutes. After stirring for 1 hour at -70° C, a solution of ethyl trifluoroacetate (0.18 mL, 1.51 mmol) in THF (1 mL) was added dropwise maintaining the temperature below -70° C. After stirring for 2 hours water (1 mL) was added and the resulting mixture was warmed to room temperature; it was then stirred overnight. The mixture was diluted with a saturated aqueous solution of $NH_4Cl$ and EtOAc. The organic layer was separated and the aqueous layer was extracted 3 times with EtOAc (20 mL). The combined organic extracts were washed with brine (20 mL), dried over $MgSO_4$, filtered and evaporated under reduced pressure to give a yellow residue (850 mg). This crude material was purified via flash chromatography (10% to 50% of EtOAc in hexane) to give 79 mg (12% yield) of 1-[5-(1,3-dibenzyl-pyrrolidin-3-yl)-1*H*-indol-2-yl]-2,2,2-trifluoro-ethanone as yellow oil.

Step 2 1-[5-(3-Benzyl-pyrrolidin-3-yl)-1*H*-indol-2-yl]-2,2,2-trifluoro-ethanone

1-[5-(1,3-Dibenzyl-pyrrolidin-3-yl)-1H indol-2-yl]-2,2,2-trifluoro-ethanone was deprotected in a similar manner as described in Example 6 Step 3. 1-[5-(3-Benzyl-pyrrolidin-3-yl)-1*H*-indol-2-yl]-2,2,2-trifluoro-ethanone was obtained as a yellow solid. MS = 373 $[M+H]^+$.

Example 14 5-(3-Benzyl-pyrrolidin-3-yl)-1-methyl-1*H*-indole

The synthetic procedure described in this Example was carried out according to the process shown in Scheme O.

## SCHEME O

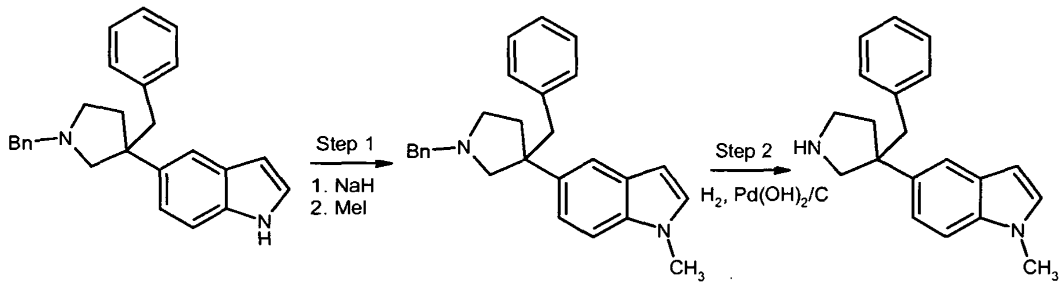


Step1 5-(1,3-Dibenzyl-pyrrolidin-3-yl)-1-methyl-1*H*-indole

A solution of 5-(1,3-dibenzyl-pyrrolidin-3-yl)-1*H*-indole (0.20 g, 0.546 mmol) in DMF (3 mL) was added, at room temperature, under nitrogen atmosphere, to a stirring suspension ofNaH (60% in mineral oil, 85.2 mg, 2.113 mmol) in DMF (2.0 mL). The resulting mixture was stirred at room temperature for 1 hour and then methyl iodide (41 μL, 0.655 mmol) was added to the cloudy suspension. After stirring for 3 hours water (10 mL) and EtOAc (20 mL) were added and the phases were separated. The aqueous phase was extracted twice with EtOAc (10 mL); the combined organic extracts were washed with brine (10 mL), dried over $MgSO_4$, filtered and evaporated under reduced pressure to give a colorless residue (220 mg). This crude material was purified via flash chromatography (10% to 60% of EtOAc in hexane) to give 129 mg (62% yield) of 5-(1,3-dibenzyl-pyrrolidin-3-yl)-1-methyl-1*H*-indole as a colorless film.

Similarly prepared using the appropriate alkylating agent, was: 1-Cyclopropylmethyl-5-(1,3-dibenzyl-pyrrolidin-3-yl)-1*H*-indole (137 mg, 60% yield) as a clear film; MS = 421 $[M+H]^+$; 5-(1-Benzyl-3-pyridin-2-ylmethyl-pyrrolidin-3-yl)-1-methyl-1*H*-indole (182 mg, 77% yield).

Step 2 5-(3-Benzyl-pyrrolidin-3-yl)-1-methyl-1*H*-indole

5-(1,3-Dibenzyl-pyrrolidin-3-yl)-1-methyl-1*H*-indole was deprotected in a similar manner as described in Example 9 Step 2. 5-(3-Benzyl-pyrrolidin-3-yl)-1-methyl-1*H*-indole was obtained as a colorless foam in 75% yield. MS = 291 $[M+H]^+$.

Similarly prepared using the appropriate starting material, were: 5-(3-Benzyl-pyrrolidin-3-yl)-1-cyclopropylmethyl-1*H*-indole as a yellow gum; the corresponding hydrochloride salt was generated by addition of a solution of HCl in $Et_2O$ (white powder, 56 mg, 47% yield); MS = 331 1 $[M+H]^+$; 1-Methyl-5-(3-pyridin-2-ylmethyl-pyrrolidin-3-yl)-1*H*-indole (pale yellow solid, 85 mg, 62% yield); MS = 292 $[M+H]^+$.

Example 15 5-(3-Benzyl-pyrrolidin-3-yl)-1-methanesulfonyl-1*H*-indole

The synthetic procedure described in this Example was carried out according to the process shown in Scheme P.

## SCHEME P

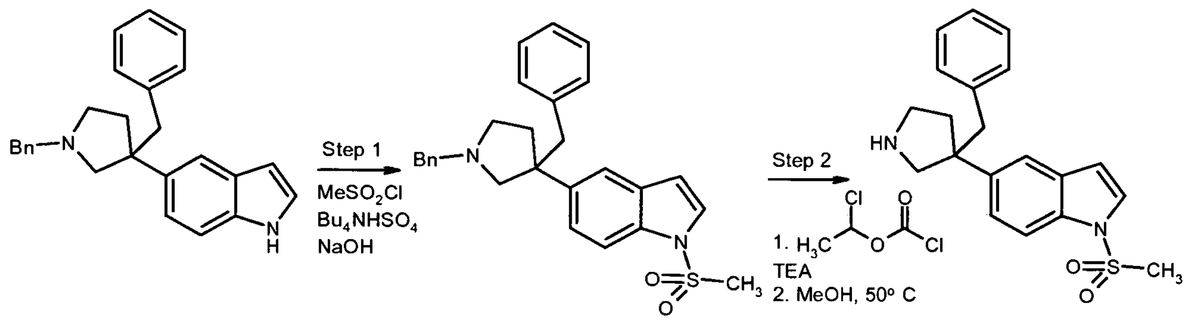

Step 1 5-(1,3-Dibenzyl-pyrrolidin-3-yl)-1-methanesulfonyl-1*H*-indole

To a vigorously stirred solution of 5-(1,3-dibenzyl-pyrrolidin-3-yl)-1H indole (230 mg, 0.628 mmol) in toluene (2 mL) and NaOH (50% in water, 2 mL) at 0° C was added $Bu_4NHSO_4$ (32 mg, 0.0943 mmol) followed by methanesulfonyl chloride (98 μL, 1.26 mmol). The reaction mixture was warmed to room temperature and was stirred for 2 hours; then a second aliquot of methanesulfonyl chloride (49 μL, 1.0 equivalent) was added. After stirring for an additional hour a third aliquot of methanesulfonyl chloride (49 μL, 1.0 equivalent) was added. The reaction mixture was stirred for 18 hours; it was then diluted with water (5 mL) and EtOAc (20 mL) and the phases were separated. The aqueous phase was extracted twice with EtOAc (20 mL); the combined organic extracts were washed twice with water (10 mL) and once with brine (10 mL), dried over $MgSO_4$, filtered and evaporated under reduced pressure to give a yellow liquid. This crude material was purified via flash chromatography (0% to 5% of MeOH in DCM) to give 160 mg (57% yield) of 5-(1,3-dibenzyl-pyrrolidin-3-yl)-1-methanesulfonyl-1*H*-indole as a colorless gum.

Step 2 5-(3-Benzyl-pyrrolidin-3-yl)-1-methanesulfonyl-1*H*-indole

5-(1,3-Dibenzyl-pyrrolidin-3-yl)-1-methanesulfonyl-1*H*-indole was deprotected in a similar manner as described in Example 13 Step 2. 5-(3-Benzyl-pyrrolidin-3-yl)-1-methanesulfonyl-1H-indole was obtained as a colorless foam in 89% yield. MS = 355 $[M+H]^+$.

Example 16 5-(3-Benzyl-pyrrolidin-3-yl)-indole-1-carboxylic acid dimethylamide

The synthetic procedure described in this Example was carried out according to the process shown in Scheme Q.

SCHEME Q

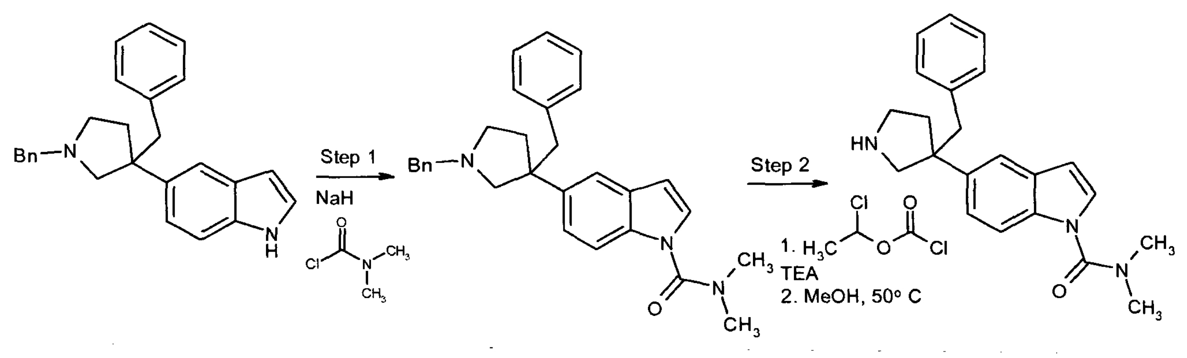


Step 1 5-(1,3-Dibenzyl-pyirolidin-3-yl)-indole-1-carboxylic acid dimethylamide

A solution of 5-(1,3-dibenzyl-pyrrolidin-3-yl)-1*H*-indole (0.230 g, 0.628 mmol) in THF (1 mL) was added, at 0° C under nitrogen atmosphere, to a stirring suspension ofNaH (60% in mineral oil, 30.1 mg, 0.754 mmol) in THF (1.0 mL). The resulting mixture was stirred at room temperature for 1 hour; it was then cooled to 0° C and N,N-dimethylcarbamoyl chloride (63 μL, 0.691 mmol) was added. The reaction mixture was stirred at room temperature for 6 hours; it was then quenched by addition of a saturated aqueous solution of $NH_4Cl$ (5 mL).The resulting mixture was extracted 3 times with EtOAc (10 mL); the combined organic extracts were washed with brine (5 mL), dried over $MgSO_4$, filtered and evaporated under reduced pressure to give a colorless gum (300 mg). This crude material was purified via flash chromatography (0% to 5% ofMeOH in DCM) to give 160 mg (58% yield) of 5-(1,3-dibenzyl-pyrrolidin-3-yl)-indole-1-carboxylic acid dimethylamide as a colorless gum.

Step 2 5-(3-Benzyl-pyrrolidin-3-yl)-indole-1-carboxylic acid dimethylamide

5-(1,3-dibenzyl-pyrrolidin-3-yl)-indole-1-carboxylic acid dimethylamide was deprotected in a similar manner as described in Example 13 Step 2. 5-(3-Benzyl-pyrrolidin-3-yl)-indole-1-carboxylic acid dimethylamide was obtained as an off-white powder in 94% yield (119 mg). MS = 348 $[M+H]^+$.

Example 17 5-(3-Benzyl-pyrrolidin-3-yl)-3-chloro-indole-1-sulfonic acid dimethylamide

The synthetic procedure described in this Example was carried out according to the process shown in Scheme R.

## SCHEME R

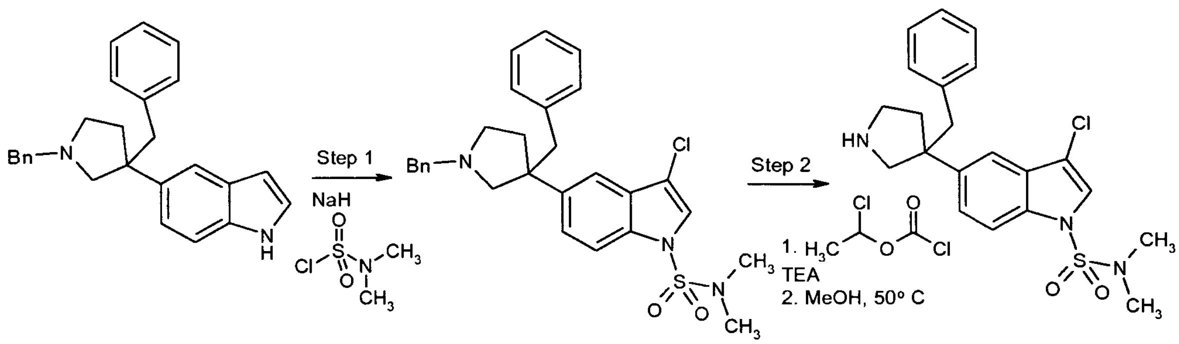


Step 1 3-Chloro-5-(1,3-dibenzyl-pyrrolidin-3-yl)-indole-1-sulfonic acid dimethylamide

NaH (60% in mineral oil, 31.5 mg, 0.788 mmol) was added, at 0° C under nitrogen atmosphere, to a stirring solution of 5-(1,3-dibenzyl-pyrrolidin-3-yl)-1*H*-indole (0.206 g, 0.563 mmol) in THF (2 mL). The resulting mixture was stirred at room temperature for 1 hour; it was then cooled to 0° C and N,N-dimethylsulfamoyl chloride (73 μL, 0.676 mmol) was added. The reaction mixture was warmed to room temperature and it was stirred for 20 hours; water (5 mL) and EtOAc (10 mL) were then added. The organic layer was separated and the aqueous layer was extracted twice with EtOAc (15 mL); the combined organic extracts were washed with brine (10 mL), dried over $MgSO_4$, filtered and evaporated under reduced pressure to give a yellow residue. This crude material was purified via flash chromatography (0% to 5% of MeOH in DCM) to give 50 mg of 3-chloro-5-(1,3-dibenzyl-pyrrolidin-3-yl)-indole-1-sulfonic acid dimethylamide as a colorless residue.

Step 2 5-(3-Benzyl-pyrrolidin-3-yl)-3-chloro-indole-1-sulfonic acid dimethylamide

3-Chloro-5-(1,3-dibenzyl-pyrrolidin-3-yl)-indole-1-sulfonic acid dimethylamide was deprotected in a similar manner as described in Example 13 Step 2. 5-(3-Benzyl-pyrrolidin-3-yl)-3-chloro-indole-1-sulfonic acid dimethylamide was obtained as a pale yellow solid in 42% yield (23 mg). MS = 418 $[M+H]^+$.

Example 18 5-(3-Benzyl-pyrrolidin-3-yl)-1*H*-indole-3-carbonitrile

The synthetic procedure described in this Example was carried out according to the process shown in Scheme S.

## SCHEME S

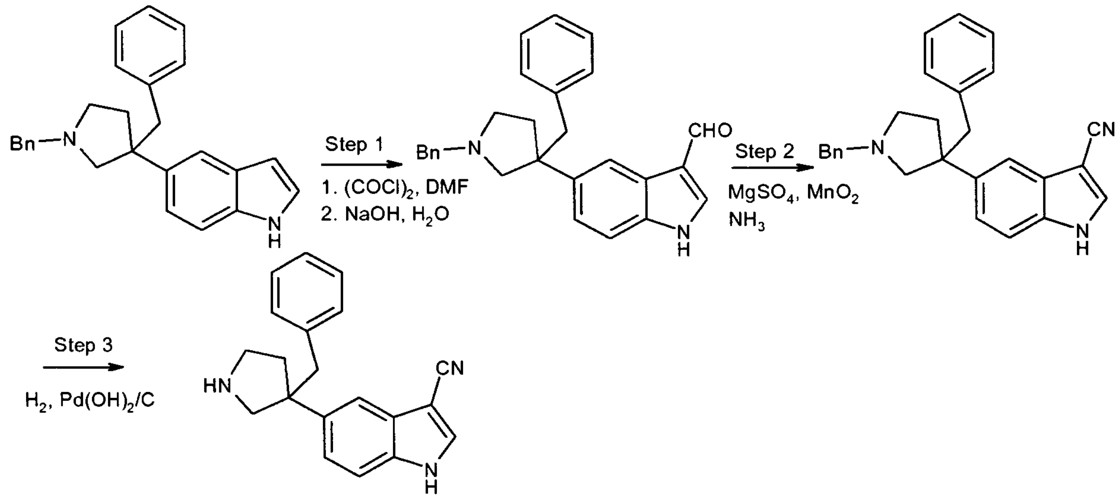


Step1 5-(1,3-Dibenzyl-pyrrolidin-3-yl)-1*H*-indole-3-carbaldehyde.

Oxalyl chloride (64 µL, 0.751 mmol) was added, at 0° C under nitrogen atmosphere, to a stirring solution of DMF (63 µL, 0.820 mmol) in 1,2-dichloroethane (4 mL); on addition of oxalyl chloride a vigorous effervescence was observed and a cloudy precipitate was formed. After stirring for 30 minutes a solution of 5-(1,3-dibenzyl-pyrrolidin-3-yl)-1*H*-indole (0.250 g, 0.683 1 mmol) in 1,2-dichloroethane (3 mL) was added dropwise; an orange colored gum immediately formed. After stirring for 1 hour in a second flask oxalyl chloride (64 µL, 0.751 mmol) was added, at 0° C under nitrogen atmosphere, to a stirring solution of DMF (63 µL, 0.820 mmol) in 1,2-dichloroethane (2 mL) and the mixture was stirred for 15 minutes; the thick precipitate formed was pipetted into the first reaction flask. A cloudy pink mixture formed that immediately cleared to leave an orange colored gum on the flask surface. After stirring for 10 minutes a solution of NaOH (2 M in water, 15 mL) was added and the resulting mixture was stirred for 30 minutes until dissolution of the orange gum. The organic layer was separated and the aqueous layer was extracted 3 times with EtOAc (20 mL). The combined organic extracts were washed with brine (20 mL), dried over $MgSO_4$, filtered and evaporated under reduced pressure to give a yellow oil (310 mg). This crude material was purified via flash chromatography (1% to 20% of a mixture 9/1 $MeOH/NH_4OH$ in DCM) to give 59 mg (22% yield) of 5-(1,3-dibenzyl-pyrrolidin-3-yl)-1*H*-indole-3-carbaldehyde as a white foam.

5-(1-Benzyl-3-pyridin-2-ylmethyl-pyrrolidin-3-yl)-1*H*-indole-3-carbaldehyde (255 mg, 93% yield) was prepared in a similar manner using the appropriate starting material. MS = 396 $[M+H]^+$.

Step 2 5-(1,3-Dibenzyl-pyrrolidin-3-yl)-1*H*-indole-3-carbonitrile

A solution of ammonia (2.0 M in isopropanol, 1.28 mL, 2.55 mmol) and anhydrous magnesium sulfate (307 mg, 2.55 mmol) were added to a solution of 5-(1,3-dibenzyl-pyrrolidin-3-yl)-1*H*-indole-3-carbaldehyde (67 mg, 0.170 mmol) in THF (0.6 mL). After stirring for 15 minutes $MnO_2$ (85%, 261 mg, 2.55 mmol) was added portionwise and the resulting mixture was stirred at room temperature for 28 hours. The same amount of ammonia, magnesium sulfate and $MnO_2$ was then added and stirring was continued using a rubber septum in order to slow the rate of ammonia loss. After stirring for additional 18 hours the mixture was warmed to 50° C for 6 hour; it was then cooled, filtered and concentrated under reduced pressure to give 62 mg (93% yield) of 5-(1,3-dibenzyl-pyrrolidin-3-yl)-1*H*-indole-3-carbonitrile as a pale yellow residue without further purifications.

5-(1-Benzyl-3-pyridin-2-ylmethyl-pyrrolidin-3-yl)-1*H*-indole-3-carbonitrile (yellow foam, 199 mg, 73% 2 steps yield) was prepared in a similar manner using the appropriate starting material.

Step 3 5-(3-Benzyl-pyrrolidin-3-yl)-1*H*-indole-3-carbonitrile

5-(1,3-Dibenzyl-pyrrolidin-3-yl)-1*H*-indole-3-carbonitrile was deprotected in a similar manner as described in Example 9 Step 2. 5-(3-Benzyl-pyrrolidin-3-yl)-1*H*-indole-3-carbonitrile was obtained in 62% yield (2 steps yield, 32 mg) as a white solid. MS = 302 $[M+H]^+$; Mp = 119.9-125.5° C. 5-(3-Pyridin-2-ylmethyl-pyrrolidin-3-yl)-1*H*-indole-3-carbonitrile (yellow foamy solid, 20 mg, 25% yield); MS = 303 $[M+H]^+$.

Example 19 5-(3-Benzyl-pyrrolidin-3-yl)-7-chloro-1*H*-indole

The synthetic procedure described in this Example was carried out according to the process shown in Scheme T.

## SCHEME T

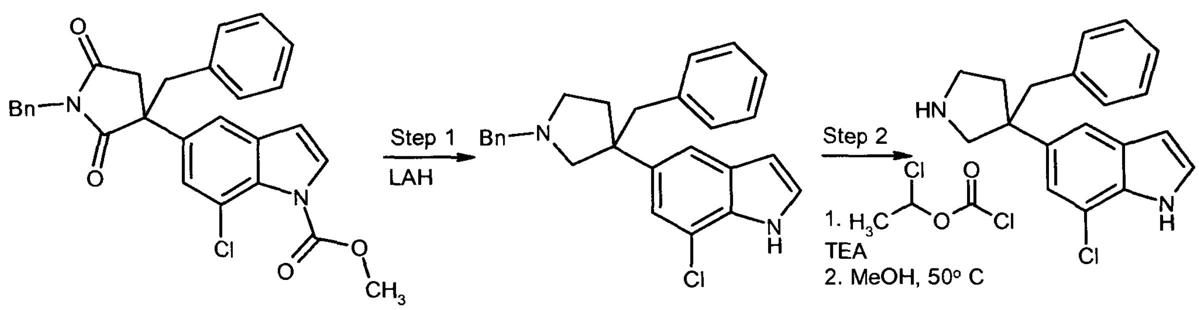


Step 1 7-Chloro-5-(1,3-dibenzyl-pyrrolidin-3-yl)-1*H*-indole.

A solution of lithium aluminum hydride (1.0 M in THF, 2.01 mL) was added dropwise, at room temperature, under nitrogen atmosphere, to a stirred solution of 7-chloro-5-(1,3-dibenzyl-2,5-dioxo-pyrrolidin-3-yl)-indole-1-carboxylic acid methyl ester (prepared in a similar manner as described in Example 1) (244 mg, 0.502 mmol) in THF (5 mL). The mixture was heated to 70° C for 45 minutes; it was then cooled and quenched by the addition of $Na_2SO_4.10H_2O$ (ca. 1 g). The mixture was diluted with EtOAc (40 mL) and filtered; the filtrate was concentrated under reduced pressure to give a colorless gum (230 mg). This crude material was purified via flash chromatography (30% to 80% EtOAc in hexane) to give 125 mg (62% yield) of 7-chloro-5-(1,3-dibenzyl-pyrrolidin-3-yl)-1*H*-indole as a colorless gum. MS = 401 $[M+H]^+$.

5-(3-Benzyl-1-methyl-pyrrolidin-3-yl)-1*H*-indole (354 mg, 90% yield, light green solid) was prepared in a similar manner. MS = 291 $[M+H]^+$.

Step 2 5-(3-Benzyl-pyrrolidin-3-yl)-7-chloro-1*H*-indole

7-Chloro-5-(1,3-dibenzyl-pyrrolidin-3-yl)-1*H*-indole was deprotected in a similar manner as described in Example 13 Step 2. 5-(3-Benzyl-pyrrolidin-3-yl)-7-chloro-1*H*-indole was obtained as an off-white foamy solid. MS = 311 $[M+H]^+$.

Example 20 3-Benzo*[b]*thiophen-5-yl-3-benzyl-1-methyl-pyrrolidine hydrochloride.

The synthetic procedure described in this Example was carried out according to the process shown in Scheme U.

## SCHEME U

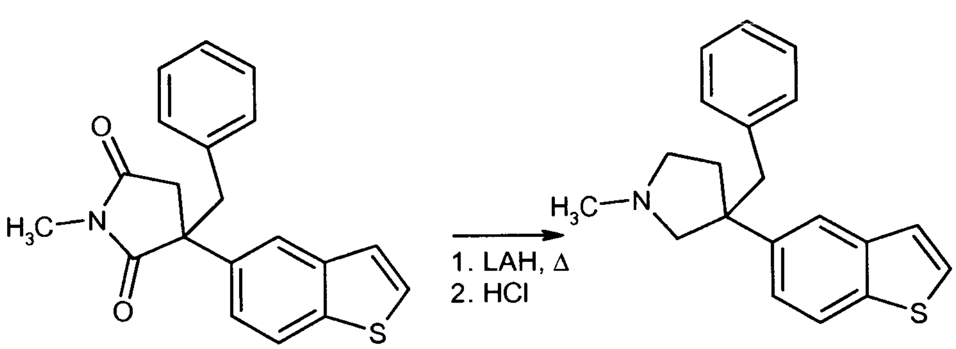


A solution of lithium aluminum hydride (1.0 M solution in THF, 11.2 mL) was added dropwise, under nitrogen atmosphere, at room temperature, to a stirred solution of 3-benzo*[b]*thiophen-5-yl-3-benzyl-1-methyl-pyrrolidine-2,5-dione (939 mg, 2.80 mmol) in THF (11.2 mL). The reaction mixture was heated at 80° C for 2 hours; it was then cooled at room temperature and quenched by addition of $Na_2SO_4.10H_2O$ (ca. 2 g). The mixture was diluted with EtOAc (20

mL) and filtered; the filtrate was concentrated under reduced pressure to give 0.89 g of a colorless oil. This crude material was purified by flash chromatography (0% to 10% of a mixture 9/1 1 MeOH/$NH_4OH$ in DCM) to give 707 mg (82% yield) of 3-benzo*[b]*thiophen-5-yl-3-benzyl-1-methyl-pyrrolidine as a clear gum. A portion of this material (67 mg) was dissolved in DCM (1 mL) and a solution of HCl (1 M in $Et_2O$, 0.218 mL) was added. The mixture was evaporated under reduced pressure to give 73 mg 3-benzo*[b]*thiophen-5-yl-3-benzyl-1-methyl-pyrrolidine hydrochloride as a white foam. MS = 308 $[M+H]^+$.

Similarly prepared using the appropriate starting material, were: 3-Benzo*[b]*thiophen-5-yl-1-methyl-pyrrolidine hydrochloride (white solid), MS = 218 $[M+H]^+$; 5-(1-Methyl-pyrrolidin-3-yl)-1H-indole (white solid, 189 mg, 865 yield), MS = 201 $[M+H]^+$.

Example 21 2-[3-(1*H*-Indol-5-yl)-pyrrolidin-3-yl]-1-phenyl-ethanol

The synthetic procedure described in this Example was carried out according to the process shown in Scheme V.

## SCHEME V

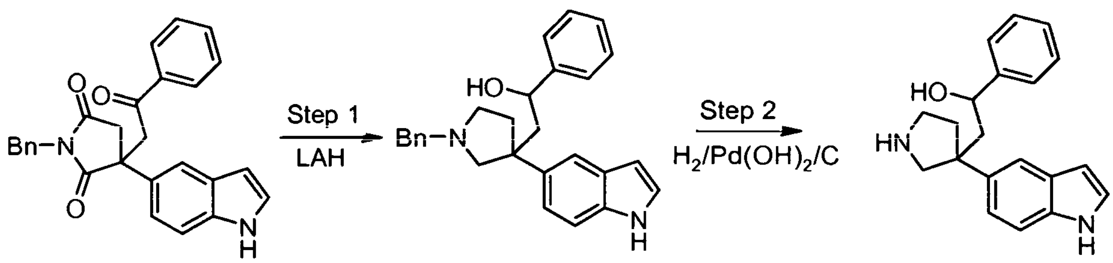


Step 1 2-[1-Benzyl-3-(1*H*-indol-5-yl)-pyrrolidin-3-yl]-1-phenyl-ethanol

A solution of lithium aluminum hydride (1.0 M solution in THF, 2.60 mL) was added, under nitrogen atmosphere at room temperature, to a stirred solution of 1-benzyl-3-(1*H*-indol-5-yl)-3-(2-oxo-2-phenyl-ethyl)-pyrrolidine-2,5-dione (274 mg, 0.645 mmol) in THF (5 mL). The reaction mixture was heated at 80° C for 3 hours; it was then cooled at room temperature and quenched by addition of $Na_2SO_4.10H_2O$ (ca. 1 g). The mixture was diluted with EtOAc (15 mL) and filtered; the filtrate was concentrated under reduced pressure to give 266 mg of a yellow residue. This crude material was purified by flash chromatography (0% to 5% of a mixture 9/1 MeOH/$NH_4OH$ in DCM) to give 92 mg of 2-[1-benzyl-3-(1*H*-indol-5-yl)-pyrrolidin-3-yl]-1-phenyl-ethanol as a single diastereomer.

Step 2 2-[3-(1*H*-indol-5-yl)-pyrrolidin-3-yl]-1-phenyl-ethanol

2-[1-Benzyl-3-(1*H*-indol-5-yl)-pyrrolidin-3-yl]-1-phenyl-ethanol was deprotected in a similar manner as described in Example 9 Step 2. 2-[3-(1*H*-Indol-5-yl)-pyrrolidin-3-yl]-1-phenyl-ethanol was obtained as a white foam. MS = 307 $[M+H]^+$.

Example 22 Formulations

Pharmaceutical preparations for delivery by various routes are formulated as shown in the following Tables. "Active ingredient" or "Active compound" as used in the Tables means one or more of the Compounds of Formula I.

Composition for Oral Administration

| Ingredient | % wt./wt. |
|---|---|
| Active ingredient | 20.0% |
| Lactose | 79.5% |
| Magnesium stearate | 0.5% |

The ingredients are mixed and dispensed into capsules containing about 100 mg each; one capsule would approximate a total daily dosage.

Composition for Oral Administration

| Ingredient | % wt./wt. |
|---|---|
| Active ingredient | 20.0% |
| Magnesium stearate | 0.5% |
| Crosscarmellose sodium | 2.0% |
| Lactose | 76.5% |
| PVP (polyvinylpyrrolidine) | 1.0% |

The ingredients are combined and granulated using a solvent such as methanol. The formulation is then dried and formed into tablets (containing about 20 mg of active compound) with an appropriate tablet machine.

Composition for Oral Administration

| Ingredient | Amount |
|---|---|
| Active compound | 1.0 g |
| Fumaric acid | 0.5 g |
| Sodium chloride | 2.0 g |
| Methyl paraben | 0.15 g |
| Propyl paraben | 0.05 g |
| Granulated sugar | 25.5 g |
| Sorbitol (70% solution) | 12.85 g |
| Veegum K (Vanderbilt Co.) | 1.0 g |
| Flavoring | 0.035 ml |
| Colorings | 0.5 mg |
| Distilled water | q.s. to 100 ml |

The ingredients are mixed to form a suspension for oral administration.

Parenteral Formulation

| Ingredient | % wt./wt. |
|---|---|
| Active ingredient | 0.25 g |
| Sodium Chloride | qs to make isotonic |
| Water for injection | 100 ml |

The active ingredient is dissolved in a portion of the water for injection. A sufficient quantity of sodium chloride is then added with stirring to make the solution isotonic. The solution is made up to weight with the remainder of the

water for injection, filtered through a 0.2 micron membrane filter and packaged under sterile conditions.

Suppository Formulation

| Ingredient | % wt./wt. |
|---|---|
| Active ingredient | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

Topical Formulation

| Ingredients | grams |
|---|---|
| Active compound | 0.2-2 |
| Span 60 | 2 |
| Tween 60 | 2 |
| Mineral oil | 5 |
| Petrolatum | 10 |
| Methyl paraben | 0.15 |
| Propyl paraben | 0.05 |
| BHA (butylated hydroxy anisole) | 0.01 |
| Water | q.s. 100 |

All of the ingredients, except water, are combined and heated to about 60˚C with stirring. A sufficient quantity of water at about 60˚C is then added with vigorous stirring to emulsify the ingredients, and water then added q.s. about 100 g.

Nasal Spray Formulations

Several aqueous suspensions containing from about 0.025-0.5 percent active compound are prepared as nasal spray formulations. The formulations optionally contain inactive ingredients such as, for example, microcrystalline cellulose, sodium carboxymethylcellulose, dextrose, and the like. Hydrochloric acid may be added to adjust pH. The nasal spray formulations may be delivered via a nasal spray metered pump typically delivering about 50-100 microliters of formulation per actuation. A typical dosing schedule is 2-4 sprays every 4-12 hours.

Example 23 Screening for Human Serotonin Transporter (hSERT) Antagonists Using a Scintillation Proximity Assay (SPA)

The screening assay of this example was used to determine the affinity of ligands at the hSERT transporter by competition with [$^3$H]-Citalopram.

Scintillation Proximity Assay (SPA) works by bringing radioligand within close proximity to the bead's scintillant to stimulate light emission. In this assay, the receptor-containing membranes were pre-coupled to the SPA beads and the binding of the appropriate radioligand to the transporter was measured. The light emission was proportional to the amount of bound radioligand. Unbound radioligand produced no signal as a result of distant proximity to scintillant (lack of energy transfer).

HEK-293 cells (Tatsumi et al., Eur. J. Pharmacol. 1997, 30, 249-258) stably expressing recombinant hSERT were maintained with media (DMEM high glucose with 10% FBS, 300 μg/ml G418 and 2 mM L-Glutamine) and incubated at 37 °C with 5% $CO_2$. Cells are released from culture flasks using PBS for 1-2 minutes. The cells were subsequently centrifuged at 1000g's for 5 minutes and resuspended in PBS prior to being used in the membrane preparation.

Cell membranes were prepared using a membrane preparation buffer of 50 mM TRIS (pH 7.4). Cell membranes were prepared from a single cube (7.5x$10^9$ cells total). Cells were homogenized using a Polytron (setting medium for a 4 second burst). The homogenate was then centrifuged at 48,000xg for 15 minutes, the supernatant subsequently removed and discarded, and the pellet resuspended with fresh buffer. After a second centrifugation, the pellet was re-homogenized and brought to a final volume determined during the assay. Typically, membrane portions were aliquoted in 3mg/ml (w:v). and stored at -80 °C.

For Scintillation Proximity Assay $IC_{50}/K_i$ determination, 50 mM Tris-HCl and 300 mM NaCl, (pH 7.4) buffers were utilized. Compounds of the invention were diluted from 10 mM to 0.1 nM FAC (10 point curves, whole log /half log dilutions) via a Beckman Biomek 2000 using a serial dilution protocol. The test compounds were then transferred (20 μl/well) and the [$^3$H]-Citalopram radioligand was added at 50 μl/well. Membrane and beads were prepared to a ratio of 10 μg : 0.7 mg, with 0.7 mg PVT-WGA Amersham beads (Cat# RPQ0282V) added per well. 130 μl of the membrane : bead mixture was added to the assay plate. The mixtures were allowed to stand at room temperature for one hour, and were then counted on a Packard TopCount LCS, a generic Scintillation Proximity Assay counting protocol settings (Energy Range: Low, Efficiency Mode: Normal, Region A: 1.50-35.00, Region B: 1.50-256.00, Count Time (min.): 0.40, Background Subtract: none, Half-Life Correction: no, Quench Indicator: tSIS, Platemap blank subtraction: No, Cross talk reduction: Off).

The % inhibition was calculated for each compound tested [(Compound counts per minute (CPM) at maximum concentration-Non-Specific CPM)/Total CPM * 100]. The concentration producing 50% inhibition ($IC_{50}$) was determined using an iterative non-linear curve fitting technique with Activity Base/Xlfit using the following equation:

$$y = \frac{\max - \min}{1 + (IC50 / x)^n} + \min$$

where max = total binding, min = non specific binding, x = concentration (M) of the tested compound and n = Hill slope. The inhibition dissociation constant (Ki) of each compound was determined according to the method of Cheng-Prusoff and then converted into negative logarithm (pKi) of the Ki.

Using the above procedure, compounds of the invention were found to have affinity for human serotonin transporter. For example, 5-(3-Benzyl-pyrrolidin-3-yl)-1*H*-indole-2-carbonitrile exhibited a pKi of approximately 9.5 using the above assay.

Example 24 Screening for compounds active at Human Norepinephrine Transporter (hNET) Using a Scintillation Proximity Assay (SPA)

This assay was used to determine the affinity of ligands for the hNET transporter by competition with [$^3$H]-Nisoxetine. As in the hSERT assay of the above example, receptor-containing membranes were pre-coupled to the SPA beads and the binding of the appropriate radioligand to the transporter was measured. The light emission was proportional to the amount of bound radioligand, with unbound radioligand producing no signal.

HEK-293 cells (Tatsumi et al., Eur. J. Pharmacol. 1997, 30, 249-258) stably expressing recombinant hNET (Clone: HEK-hNET #2) were maintained with media (DMEM hi glucose with 10% FBS, 300 μg/ml G418 and 2 mM L-Glutamine) and incubated at 37 °C with 5% $CO_2$. Cells were released from culture flasks using PBS for 1-2 minutes. The cells were subsequently centrifuged at 1000g's for 5 minutes and resuspended in PBS prior to being used in the membrane preparation.

Cell membranes were prepared using a membrane preparation buffer of 50 mM TRIS (pH 7.4). Cell membranes were prepared from a single cube (7.5x$10^9$ cells total). Cells were homogenized using a Polytron (setting medium for a 4 second burst). The homogenate was then centrifuged at 48,000xg for 15 minutes, the supernatant subsequently removed and discarded, and the pellet resuspended with fresh buffer. After a second centrifugation, the pellet was re-homogenized and brought to a final volume determined during the assay. Typically, membrane portions were aliquoted in 3-6 mg/ml (w:v). and stored at -80 °C.

$^3$[H] Nisoxetine radio ligand (Amersham Cat. # TRK942 or Perkin Elmer Cat. # NET1084, specific activity: 70-87 Ci/mmol, stock concentration: 1.22e-5 M, final concentration: 8.25e-9 M), and 50 mM Tris-HCl, 300 mM NaCl, (pH 7.4) buffers were used for Scintillation Proximity Assay $IC_{50}/K_i$ determination. Compounds of the invention were diluted from 10 mM to 0.1 nM FAC (10 point curves, whole log /half log dilutions) via a Beckman Biomek 2000 using a

serial dilution protocol. The test compounds were then transferred (20 μl/well) and the radioligand was added at 50 μl/well. Membrane and beads were prepared to a ratio of 10 μg : 0.7 mg, with 0.7 mg PVT-WGA Amersham beads (Cat# RPQ0282V) added per well. 130 μl of the membrane : bead mixture was added to the assay plate. The mixtures were allowed to stand at room temperature for one hour, and were then counted on a Packard TopCount LCS, a generic SPA counting protocol settings (Energy Range: Low, Efficiency Mode: Normal, Region A: 1.50-35.00, Region B: 1.50-256.00, Count Time (min.): 0.40, Background Subtract: none, Half-Life Correction: no, Quench Indicator: tSIS, Platemap blank subtraction: No, Cross talk reduction: Off).

The % inhibition was calculated for each compound tested [(Compound CPM at maximum concentration-Non-Specific CPM)/Total CPM * 100]. The concentration producing 50% inhibition ($IC_{50}$) was determined using an iterative non-linear curve fitting technique with Activity Base/Xlfit using the following equation:

$$y = \frac{\max - \min}{1 + (IC50/x)^n} + \min$$

where max = total binding, min = non specific binding, x = concentration (M) of the tested compound and n = Hill slope. The inhibition dissociation constant (Ki) of each compound was determined according to the method of Cheng-Prusoff and then converted into negative logarithm (pKi) of the Ki.

Using the above procedure, compounds of the invention were found to have affinity for the human norepinephrine transporter. For example, 6-((S)-3-Benzyl-pyrrolidin-3-yl)-1*H*-indole exhibited a pKi of approximately 9.2 using the above assay.

Example 25 Screening for compounds active at Human Dopamine Transporter Using a Scintillation Proximity Assay (SPA)

This assay was used to determine the affinity of ligands for the dopamine transporter by competition with [$^3$H]-Vanoxerine.

HEK-293 cells (Tatsumi et al., Eur. J. Pharmacol. 1997, 30, 249-258) stably expressing recombinant hDAT were maintained with media (DMEM hi glucose with 10% FBS, 300 μg/ml G418 and 2 mM L-Glutamine) and incubated at 37 °C with 5% $CO_2$. Cells were plated four hours prior to experiment by placing approximately 30,000 cells per well (in PBS) on white, opaque Cell-Tak coated 96 well plates. Extra buffer was apriated from the cell plates using an ELx405 plate washer.

$^3$[H] vanoxerine (GBR 12909) radio ligand, specific activity approximately 59 Ci/mmol, stock concentration, 400 nM, and 50 mM Tris-HCl, 300 mM NaCl, (pH 7.4) buffers were used for Scintillation Proximity Assay $IC_{50}/K_i$ determination. Compounds of the invention were diluted from 10 mM to 0.1 nM FAC (10 point curves, whole log /half log dilutions) via a Beckman Biomek 2000 using a 10-point dilution protocol. The mixtures were allowed to stand at room temperature for 30 minutes, and were then counted on a Packard TopCount LCS, a generic SPA counting protocol settings, Count Time (min.): 0.40, Background Subtract: none, Half-Life Correction: none, Quench Indicator: tSIS, Platemap blank subtraction: none, Cross talk reduction: Off).

The % inhibition was calculated for each compound tested [(Compound CPM at maximum concentration-Non-Specific CPM)/Total CPM * 100]. The concentration producing 50% inhibition ($IC_{50}$) was determined using an iterative non-linear curve fitting technique with Activity Base/Xlfit using the following equation:

$$y = \frac{\max - \min}{1 + (IC50/x)^n} + \min$$

where max = total binding, min = non specific binding, x = concentration (M) of the tested compound and n = Hill slope. The inhibition dissociation constant (Ki) of each compound was determined according to the method of Cheng-Prusoff and then converted into negative logarithm (pKi) of the Ki.

Using the above procedure, compounds of the invention were found to have affinity for the human dopamine transporter. For example, 5-((R)-3-Benzyl-pyrrolidin-3-yl)-1H-indole exhibited a pKi of approximately 8.0 using the above assay.

Example 26 Formalin Pain Assay

Male Sprague Dawley rats (180-220 g) are placed in individual Plexiglas cylinders and allowed to acclimate to the testing environment for 30 min. Vehicle, drug or positive control (morphine 2 mg/kg) is administered subcutaneously at 5 ml/kg. 15 min post dosing, formalin (5% in 50 μl) is injected into plantar surface of the right hind paw using a 26-gauge needle. Rats are immediately put back to the observation chamber. Mirrors placed around the chamber allow unhindered observation of the formalin-injected paw. The duration of nociphensive behavior of each animal is recorded by a blinded observer using an automated behavioral timer. Hindpaw licking and shaking / lifting are recorded separately in 5 min bin, for a total of 60 min. The sum of time spent licking or shaking in seconds from time 0 to 5 min is considered the early phase, whereas the late phase is taken as the sum of seconds spent licking or shaking from 15 to 40 min. A plasma sample is collected.

Example 27 Colon Pain Assay

Adult male Sprague-Dawley rats (350-425 g; Harlan, Indianapolis, IN) are housed 1-2 per cage in an animal care facility. Rats are deeply anesthetized with pentobarbital sodium (45 mg/kg) administered intraperitoneally. Electrodes are placed and secured into the external oblique musculature for electromyographic (EMG) recording. Electrode leads are tunneled subcutaneously and exteriorized at the nape of the neck for future access. After surgery, rats are housed separately and allowed to recuperate for 4-5 days prior to testing.

The descending colon and rectum are distended by pressure-controlled inflation of a 7-8 cm-long flexible latex balloon tied around a flexible tube. The balloon is lubricated, inserted into the colon via the anus, and anchored by taping the balloon catheter to the base of the tail. Colorectal distension (CRD) is achieved by opening a solenoid gate to a constant pressure air reservoir. Intracolonic pressure is controlled and continuously monitored by a pressure control device. Response is quantified as the visceromotor response (VMR), a contraction of the abdominal and hindlimb musculature. EMG activity produced by contraction of the external oblique musculature is quantified using Spike2 software (Cambridge Electronic Design). Each distension trial lasts 60 sec, and EMG activity is quantified for 20 sec before distension (baseline), during 20 sec distension, and 20 sec after distention. The increase in total number of recorded counts during distension above baseline is defined as the response. Stable baseline responses to CRD (10, 20, 40 and 80 mmHg, 20 seconds, 4 minutes apart) are obtained in conscious, unsedated rats before any treatment.

Compounds are evaluated for effects on responses to colon distension initially in a model of acute visceral nociception and a model of colon hypersensitivity produced by intracolonic treatment with zymosan (1 mL, 25 mg/mL) instilled into the colon with a gavage needle inserted to a depth of about 6 cm. Experimental groups will consist of 8 rats each.

Acute visceral nociception: For testing effects of drug on acute visceral nociception, 1 of 3 doses of drug, vehicle or positive control (morphine, 2.5 mg/kg) are administered after baseline responses are established; responses to distension are followed over the next 60-90 minutes.

Visceral hypersensitivity: For testing effects of drug or vehicle after intracolonic treatment with zymosan, intracolonic treatment is given after baseline responses are established. Prior to drug testing at 4 hours, responses to distension are assessed to establish the presence of hypersensitivity. In zymosan-treated rats, administration of 1 of 3 doses of drug, vehicle or positive control (morphine, 2.5 mg/kg) are given 4 hours after zymosan treatment and responses to distension followed over the next 60-90 minutes.

Example 28 Cold allodynia in Rats with a Chronic Constriction Injury of the Sciatic Nerve

The effects of compounds of this invention on cold allodynia are determined using the chronic constriction injury (CCI) model of neuropathic pain in rats, where cold allodynia is measured in a cold-water bath with a metal-plate floor and water at a depth of 1.5-2.0 cm and a temperature of 3-4 ˚C (Gogas, K.R. et al., Analgesia, 1997, 3, 1-8).

Specifically, CCI, rats are anesthetized; the trifurcation of the sciatic nerve is located and 4 ligatures (4-0, or 5-0 chromic gut) are placed circumferentially around the sciatic nerve proximal to the trifurcation. The rats are then allowed to recover from the surgery. On days 4-7 after surgery, the rats are initially assessed for cold -induced allodynia by individually placing the animals in the cold-water bath and recording the total lifts of the injured paw during a 1-min period of time: The injured paw is lifted out of the water. Paw lifts associated with locomotion or body repositioning are not recorded. Rats that displayed 5 lifts per min or more on day 4-7 following surgery are considered to exhibit cold allodynia and are used in subsequent studies. In the acute studies, vehicle, reference compound or compounds of this invention are administered subcutaneously (s.c.) 30 min before testing. The effects of repeated administration of the compounds of this invention on cold allodynia are determined 14, 20 or 38 h following the last oral dose of the following regimen: oral (p.o.) administration of vehicle, reference or a compound of this invention at ~12 h intervals (BID) for 7 days.

## Claims

**1.** A compound of Formula I:

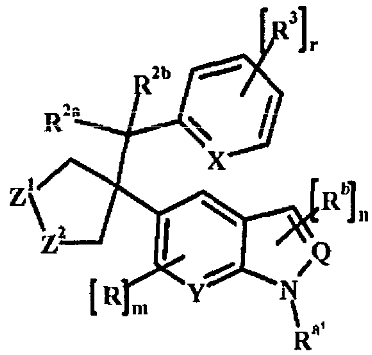

I

or an enantiomer, diastereomer, or pharmaceutically acceptable salt thereof, wherein:

either $Z^1$ or $Z^2$ is $N(R^a)$ and the other is $CH_2$;
X is CHorN;
Y is CH or N;
m is 0 or 1;
R is hydroxy, halogen, $C_1$-$C_6$alkyl, or $C_1$-$C_6$alkoxy;
Q is CH, $C(R^b)$, or N;
$R^a$ is H, $C_1$-$C_6$alkyl, or benzyl;
$R^{a'}$ is H, $C_1$-$C_6$alkyl, cycloalkyl alkyl, $-S(=O)_2R^c$, $-C(-O)N(R^c)_2$, or $S(-O)_2N(R^c)_2$;
n is 0 or 1;
each $R^b$ is independently $R^{b'}$ or $R^{b''}$;
$R^{b'}$ is hydroxy, halogen, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$, or $-S(=O)_2N(R^c)_2$;
$R^{b''}$ is $C_1$-$C_6$alkyl, $C_2$-$C_6$alkenyl, $C_2$-$C_6$alkynyl, $C_1$-$C_6$alkoxy, $C_1$-$C_6$haloalkyl, phenyl, cycloalkyl, cycloalkyl alkyl, heterocycloalkyl, heterocycloalkyl alkyl, optionally substituted with one or more $R^c$;
each $R^c$ is independently $R^d$ or $R^e$;
$R^d$ is H;
$R^e$ is $C_1$-$C_6$alkyl, $C_1$-$C_6$haloalkyl, cycloalkyl, phenyl, heterocycloalkyl, or heteroaryl, optionally substituted with one or more $R^{e'}$;
each $R^{e'}$ is independently hydroxy, halogen, amino, $C_1$-$C_6$alkyl, $C_2$-$C_6$alkenyl, $C_2$-$C_6$alkynyl, $C_1$-$C_6$alkoxy, $C_1$-$C_6$aloalkyl, or -CN;
$R^{2a}$ and $R^{2b}$ are each independently H, hydroxy, $C_1$-$C_6$akyl $C_1$-$C_6$haloalkyl, or $C_1$-$C_6$alkoxy;
r is 0, 1, or 2; and
$R^3$ is halogen, hydroxy, $C_1$-$C_6$alkyl, $C_1$-$C_6$haloalkyl, or $C_1$-$C_6$alkoxy.

**2.** The compound of claim 1, wherein $Z^1$ is $N(R^a)$ and $Z^2$ is $CH_2$.

**3.** The compound of claim 1, wherein $Z^1$ is $CH_2$ and $Z^2$ is $N(R^a)$.

**4.** The compound of claim 2, wherein $R^a$, $R^{2a}$, and $R^{2b}$ are H, m is 0, r is 0, X is CH, Y is CH, $R^{a'}$ is H, and n is 1.

**5.** The compound of claim 3, wherein $R^a$, $R^{2a}$, and $R^{2b}$ are H, m is 0, r is 0, X is CH, Y is CH, $R^{a'}$ is H, and n is 1.

**6.** The compound of claim 4, wherein Q is $C(R^b)$.

**7.** The compound of claim 5, wherein Q is $G(R^b)$.

**8.** The compound of claim 6, wherein $R^b$ is $R^{b'}$.

**9.** The compound of claim 6, wherein $R^b$ is $R^{b''}$,

**10.** The compound of claim 7, wherein $R^b$ is $R^{b'}$.

**11.** The compound of claim 7, wherein $R^b$ is $R^{b''}$.

**12.** The compound of claim 8, wherein $R^{b'}$ is -CN or halogen.

**13.** The compound of claim 8, wherein $R^{b'}$ is $-C(=O)N(R^{2c})_2$ or $-NHC(=O)(R^{2c})$.

**14.** The compound of claim 8, wherein $R^{b'}$ is $-S(=O)_2R^{2c}$ or $-S(=O)_2N(R^{2c})_2$.

**15.** The compound of claim 8, wherein $R^{b'}$ is $-C(=O)(R^{2c})$ or $-C(=O)O(R^{2c})$.

**16.** The compound of claim 9, wherein $R^{b''}$ is $C_1$-$C_6$alkyl or cycloalkyl alkyl.

**17.** The compound of claim 10, wherein $R^{b'}$ is -CN or halogen.

**18.** The compound of claim 10, wherein $R^{b'}$ is $-C(=O)N(R^{2c})_2$ or $-NHC(=O)(R^{2c})$.

**19.** The compound of claim 10, wherein $R^{b'}$ is $-C(=O)(R^{2c})$ or $-C(=O)O(R^{2c})$.

**20.** The compound of claim 11, wherein $R^{b''}$ is $C_1$-$C_6$alkyl or cycloalkyl alkyl.

**21.** A compound of Formula II:

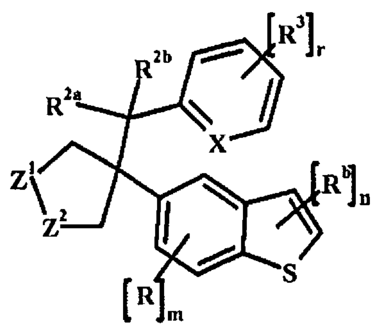

II

or an enantiomer, diastereomer, or pharmaceutically acceptable salt thereof, wherein:

either $Z^1$ or $Z^2$ is $N(R^a)$ and the other is $CH_2$;
X is CH or N;
m is 0 or 1;
R is hydroxy, halogen, $C_1$-$C_6$alkyl, or $C_1$-$C_6$alkoxy;
$R^a$ is H, $C_1$-$C_6$alkyl, or benzyl;
n is 0 or 1;
each $R^b$ is independently $R^{b'}$ or $R^{b''}$;
$R^{b'}$ is hydroxy, halogen, $-C(=O)(R^c)$, $-C(=O)O(R^c)$, $--OC(=O)(R^c)$, $-N(R^c)_2$, $-C(=O)N(R^c)_2$, $NHC(=O)(R^c)$, -CN, $-S(=O)_2R^c$, or $-S(=O)_2N(R^c)_2$;
$R^{b''}$ is $C_1$-$C_6$alkyl, $C_2$-$C_6$alkenyl, $C_2$-$C_6$alkynyl, $C_1$-$C_6$alkoxy, $C_1$-$C_6$haloalkyl, phenyl, cycloalkyl, cycloalkyl alkyl, heterocycloalkyl, heterocycloalkyl alkyl, optionally substituted with one or more $R^c$;
each $R^c$ is independently $R^d$ or $R^e$;
$R^d$ is H;
$R^e$ is $C_1$-$C_6$alkyl, $C_1$-$C_6$haloalkyl, cycloalkyl, phenyl, heterocycloalkyl, or heteroaryl, optionally substituted with

one or more $R^{e'}$;
each $R^{e'}$ is independently hydroxy, halogen, amino, $C_1$-$C_6$alkyl) $C_2$-$C_6$alkenyl, $C_2$-$C_6$alkynyl, $C_1$-$C_6$alkoxy, $C_1$-$C_6$haloalkyl, or-CN;
$R^{2a}$ and $R^{2b}$ are each independently H, hydroxy, $C_1$-$C_6$alkyl, $C_1$-$C_6$haloalkyl, or $C_1$-$C_6$alkoxy;
r is 0, 1, or 2; and
$R^3$ is halogen, hydroxy, $C_1$-$C_6$alkyl, $C_1$-$C_6$haloalkyl, or $C_1$-$C_6$alkoxy.

**22.** The compound of claim 21, wherein $R^{2a}$ and $R^{2b}$ are H, m is 0, and r is 0.

**23.** The compound of claim 22, wherein $Z^1$ is $CH_2$, $Z^2$ is $N(R^a)$, $R^a$ is H, and n is 1.

**24.** A compound of Formula **III**:

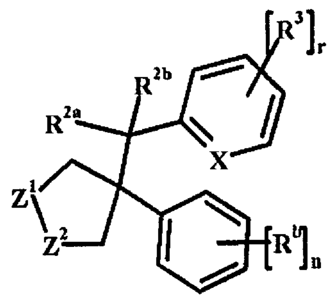

III

or an enantiomer, diastereomer, or pharmaceutically acceptable salt thereof, wherein:

either $Z^1$ or $Z^2$ is $N(R^a)$ and the other is $CH_2$;
XisCHorN;
$R^a$ is H, $C_1$-$C_6$alkyl, or benzyl;
n is 0, 1, or 2;
each $R^b$ is independently $R^{b'}$ or $R^{b''}$;
$R^{b'}$ is independently hydroxy, halogen, $-C(=O)(R^c)$, $-C(=O)O(R^c)$, $-OC(=O)(R^c)$, $-N(R^c)_2$, $-C(=O)N(R^c)_2$, -NHC(=O)($R^c$), -CN, $-S(=O)_2R^c$, or $-S(=O)_2N(R^c)_2$;
$R^{b''}$ is $C_1$-$C_6$alkyl, $C_2$-$C_6$alkenyl, $C_2$-$C_6$alkynyl, $C_1$-$C_6$alkoxy, $C_1$-$C_6$haloalkyl, phenyl, cycloalkyl, cycloalkyl alkyl, heterocycloalkyl, heterocycloalkyl alkyl, optionally substituted with one or more $R^c$;
each $R^c$ is independently $R^d$ or $R^c$;
$R^d$ is H;
$R^e$ is $C_1$-$C_6$alkyl, $C_1$-$C_6$haloalkyl, cycloalkyl, phenyl, heterocycloalkyl, or heteroaryl, optionally substituted with one or more $R^{e'}$; and
each $R^{e'}$ is independently hydroxy, halogen, amino, $C_1$-$C_6$alkyl, $C_2$-$C_6$alkenyl, $C_2$-$C_6$alkynyl, $C_1$-$C_6$alkoxy, $C_1$-$C_6$haloalkyl, or -CN;
$R^{2a}$ and $R^{2b}$ are each independently H hydroxy, $C_1$-$C_6$alkyl, $C_1$-$C_6$haloalkyl, or $C_1$-$C_6$alkoxy;
r is 0, 1, or 2;
$R^3$ is halogen, hydroxy, $C_1$-$C_6$alkyl, $C_1$-$C_6$haloalkyl, or $C_1$-$C_6$alkoxy;
with the proviso that when $Z^1$ is $N(R^a)$, $Z^2$ is $CH_2$, $R^8$ is H, and X is CH, then n is not 0;
and with the proviso that when $Z^1$ is $N(R^a)$, $Z^2$ is $CH_2$, $R^a$ is ethyl, X is CH, and either $R^{2a}$ or $R^{2b}$ is hydroxy, then n is not 0,

**25.** The compound of claim 24, wherein $R^{2a}$ and $R^{2b}$ are H and r is 0.

**26.** The compound of claim 25, wherein $Z^1$ is $CH_2$, $Z^2$ is $N(R^a)$, $R^a$ is H, and n is 1.

**27.** A compound of Formula **IV**

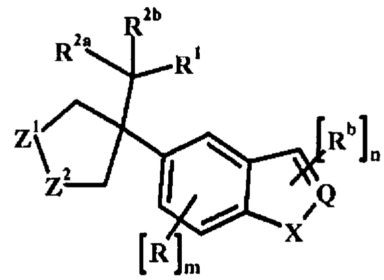

IV

or an enantiomer, diastereomer, or pharmaceutically acceptable salt thereof, wherein:

either $Z^1$ or $Z^2$ is $N(R^a)$ and the other is $CH_2$;
X is S or $N(R^{a'})$;
in is 0 or 1;
R is hydroxy, halogen, $C_1$-$C_6$alkyl, or $C_1$-$C_6$alkoxy;
Q is CH, $C(R^b)$, or N;
$R^a$ is H, $C_1$-$C_6$alkyl, or benzyl;
$R^{a'}$ is H, $C_1$-$C_6$alkyl, cycloalkyl alkyl, $-S(=O)_2R^c$, $-C(=O)N(R^c)_2$, or $S(=O)_2N(R^c)_2$;
$R^1$ is $R^{1a}$ or $R^{1b}$;
$R^{1a}$ is H;
$R^{1b}$ is $C_1$-$C_6$alkyl, $C_2$-$C_6$alkenyl, $C_2$-$C_6$alkynyl $C_1$-$C_6$alkoxy, or $C_1$-$C_6$haloalkyl, optionally substituted with one or more $R^{1b'}$;
each $R^{1b'}$ is independently hydroxy, halogen, amino, $C_1$-$C_6$alkyl, $C_1$-$C_6$alkoxy, $C_1$-$C_6$haloalkyl, or -CN;
n is 0 or 1;
each $R^b$ is independently $R^{b'}$ or $R^{b''}$;
$R^{b'}$ is hydroxy, halogen, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$, or $-S(=O)_2N(R^c)2$;
$R^{b''}$ is $C_1$-$C_6$alkyl, $C_2$-$C_6$alkenyl, $C_2$-$C_6$alkynyl, $C_1$-$C_6$alkoxy, $C_1$-$C_6$haloalkyl, phenyl, cycloalkyl, cycloalkyl alkyl, heterocycloalkyl, heterocycloalkyl alkyl, optionally substituted with one or more $R^c$;
each $R^c$ is independently $R^d$ or $R^c$;
$R^d$ is H;
$R^e$ is $C_1$-$C_6$alkyl, $C_1$-$C_6$haloalkyl, cycloalkyl, phenyl, heterocycloalkyl, or heteroaryl, optionally substituted with one or more $R^{e'}$;
each $R^{e'}$ is independently hydroxy, halogen, amino, $C_1$-$C_6$alkyl, $C_2$-$C_6$alkenyl, $C_2$-$C_6$alkynyl, $C_1$-$C_6$alkoxy, $C_1$-$C_6$haloalkyl, or -CN; and
$R^{2a}$ and $R^{2b}$ are each independently H, hydroxy, $C_1$-$C_6$alkyl, $C_1$-$C_6$haloalkyl, or $C_1$-$C_6$alkoxy.

**28.** The compound of claim 27, wherein $R^{2a}$ and $R^{2b}$ are H and m is 0.

**29.** The compound of claim 28, wherein $Z^1$ is $CH_2$, $Z^2$ is $N(R^a)$, $R^a$ is H, and n is 1.

**30.** A compound of Formula V:

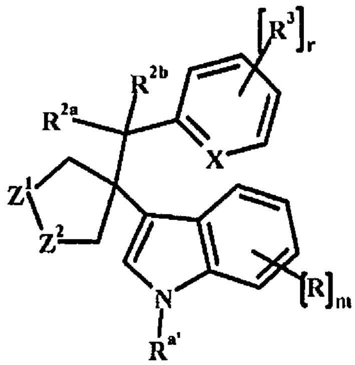

V

or an enantiomer, diastereomer, or pharmaceutically acceptable salt thereof, wherein:

either $Z^1$ or $Z^2$ is $N(R^a)$ and the other is $CH_2$;
$R^a$ is H, $C_1$-$C_6$alkyl, or benzyl;
X is CH or N;
m is 0 or 1;
R is hydroxy, halogen, $C_1$-$C_6$alkyl, or $C_1$-$C_6$alkoxy;
$R^{a'}$ is H, $C_1$-$C_6$alkyl, cycloalkyl alkyl, $-S(=O)_2R^c$, $-C(=O)N(R^c)_2$, or $S(=O)_2N(R^c)_2$;
each $R^c$ is independently $R^d$ or $R^e$;
$R^d$ is H;
$R^e$ is $C_1$-$C_6$alkyl, $C_1$-$C_6$haloalkyl, cycloalkyl, phenyl, heterocycloalkyl, or heteroaryl, optionally substituted with one or more $R^{e'}$;
each $R^{e'}$ is independently hydroxy, halogen, amino, $C_1$-$C_6$alkyl, $C_2$-$C_6$alkenyl, $C_2$-$C_6$alkynyl, $C_1$-$C_6$alkoxy, $C_1$-$C_6$ haloalkyl, or -CN;
$R^{2a}$ and $R^{2b}$ are each independently H, hydroxy, $C_1$-$C_6$alkyl, $C_1$-$C_6$haloalkyl, or $C_1$-$C_6$alkoxy;
r is 0, 1, or 2; and
$R^3$ is halogen, hydroxy, $C_1$-$C_6$alkyl, $C_1$-$C_6$haloalkyl, or $C_1$-$C_6$alkoxy,

**31.** A compound of Formula **VI**:

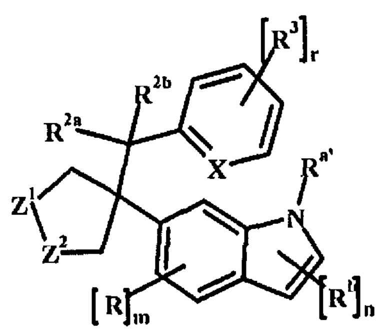

VI

or an enantiomer, diastereomer, or pharmaceutically acceptable salt thereof, wherein:

either $Z^1$ or $Z^2$ is $N(R^a)$ and the other is $CH_2$;
X is CH or N;
m is 0 or 1;
R is hydroxy, halogen, $C_1$-$C_6$alkyl, or $C_1$-$C_6$alkoxy;
$R^a$ is H, $C_1$-$C_6$alkyl, or benzyl;
$R^{a'}$ is H, $C_1$-$C_6$alkyl, cycloalkyl alkyl, $-S(=O)_2R^c$, $-C(=O)N(R^c)_2$, or $S(=O)_2N(R^c)_2$;

n is 0 or 1;

each $R^b$ is independently $R^{b'}$ or $R^{b''}$;

$R^{b'}$ is hydroxy, halogen, -C(=O)($R^c$), -C(=O)O($R^c$), -OC(=O)($R^c$), -N($R^c$)$_2$, -C(=O)N($R^c$)$_2$, -NHC(=O)($R^c$), -CN, -S(=O)$_2$$R^c$, or -S(=O)$_2$N($R^c$)$_2$;

$R^{b''}$ is $C_1$-$C_6$alkyl, $C_2$-$C_6$alkenyl, $C_2$-$C_6$alkynyl, $C_1$-$C_6$alkoxy, $C_1$-$C_6$haloalkyl, phenyl, cycloalkyl, cycloalkyl alkyl, heterocycloalkyl, heterocycloalkyl alkyl, optionally substituted with one or more $R^c$;

each $R^c$ is independently $R^d$ or $R^e$;

$R^d$ is H;

$R^e$ is $C_1$-$C_6$alkyl, $C_1$-$C_6$haloalkyl, cycloalkyl, phenyl, heterocycloalkyl, or heteroaryl, optionally substituted with one or more $R^{e'}$;

each $R^{e'}$ is independently hydroxy, halogen, amino, $C_1$-$C_6$alkyl, $C_2$-$C_6$alkenyl, $C_2$-$C_6$alkynyl, $C_1$-$C_6$alkoxy, $C_1$-$C_6$haloakyl, or -CN;

$R^{2a}$ and $R^{2b}$ are each independently H, hydroxy, $C_1$-$C_6$alkyl, $C_1$-$C_6$haloalkyl, or $C_1$-$C_6$alkoxy;

r is 0, 1, or 2; and

$R^3$ is halogen, hydroxy, $C_1$-$C_6$alkyl, $C_1$-$C_6$haloalkyl, or $C_1$-$C_6$alkoxy.

**32.** A compound selected from the group consisting of:

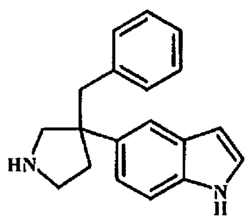
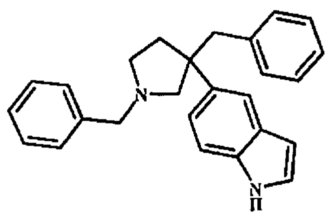
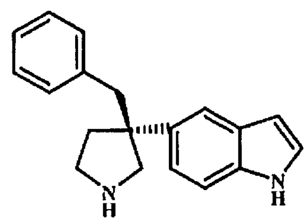
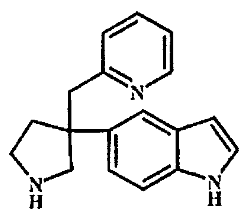
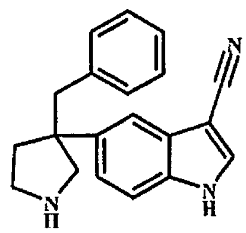
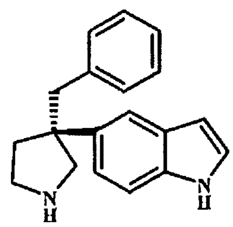

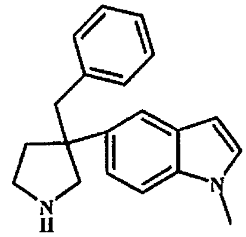
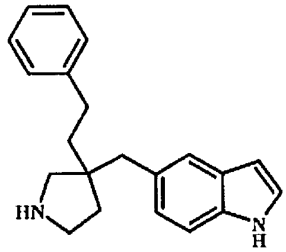
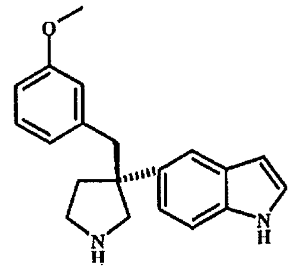
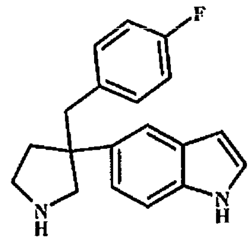
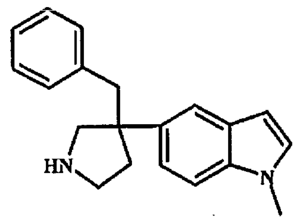
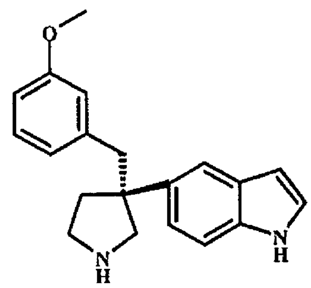
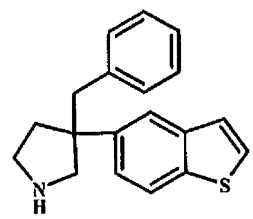
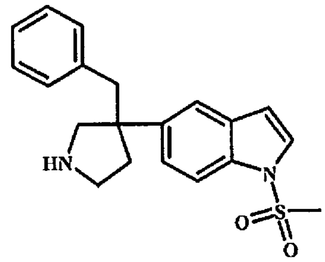
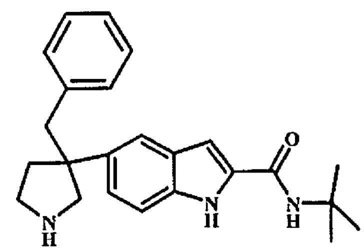
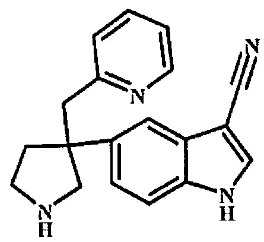
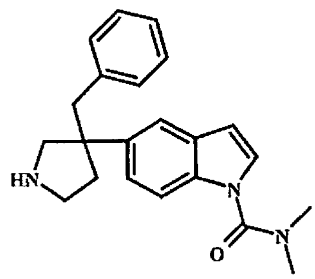
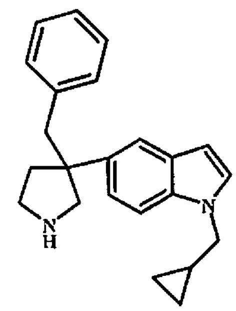
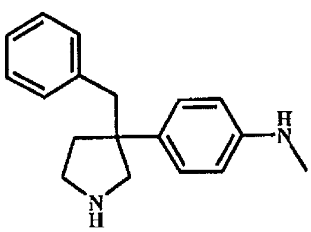
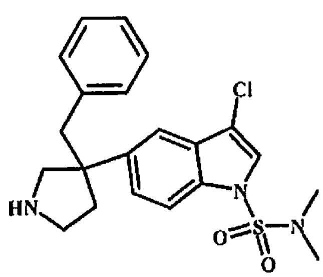
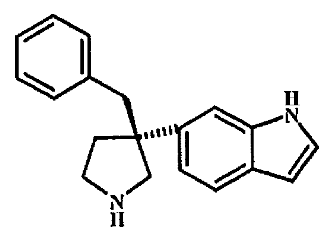
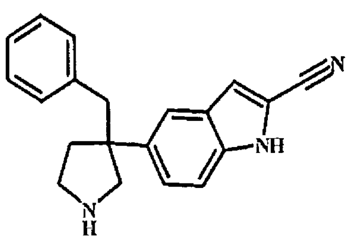
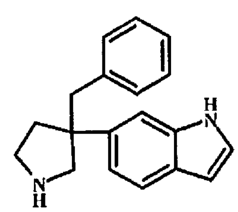
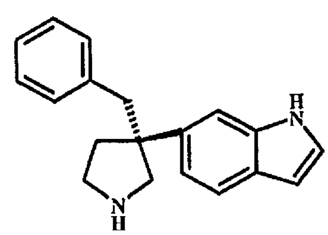
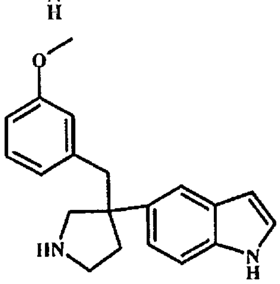

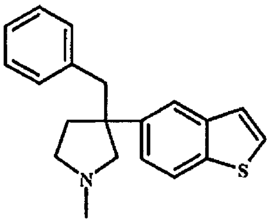
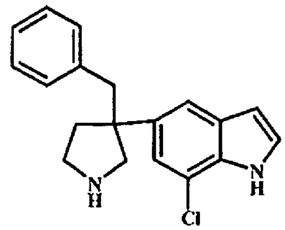
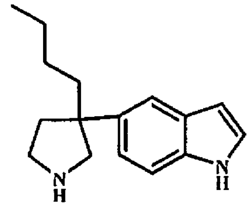
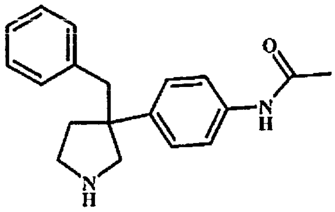
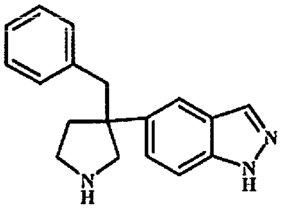
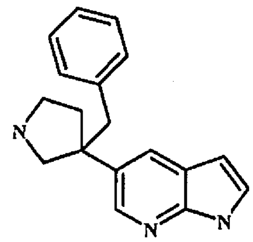
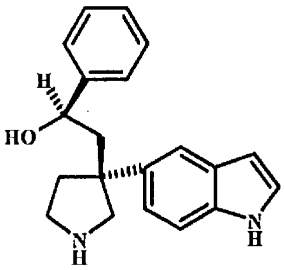
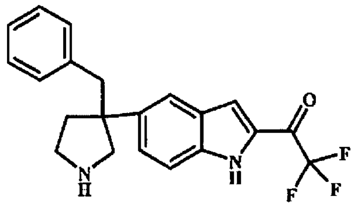
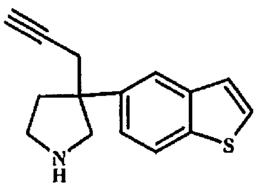
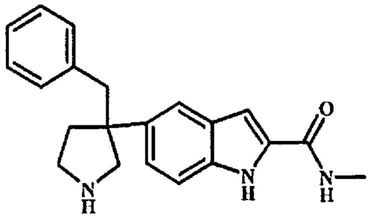
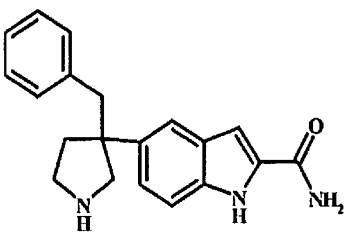
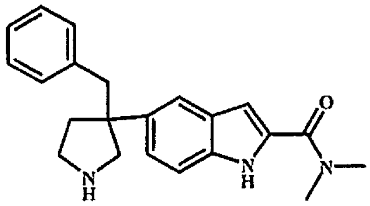
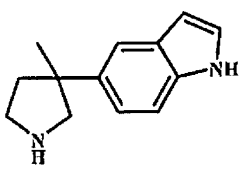
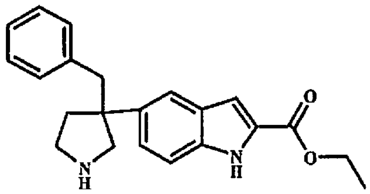
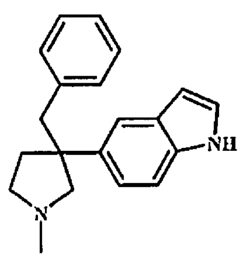
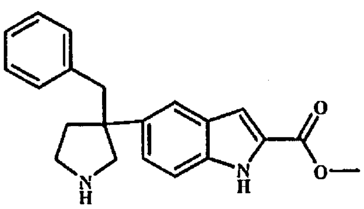
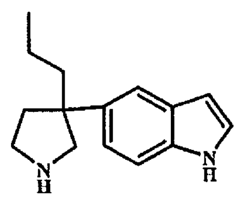

33. A pharmaceutical composition comprising the compound of any one of claims 1-32 and a pharmaceutically acceptable carrier.

34. A compound of any one of claims 1-32 for use in a method of treating anxiety, depression, or both, comprising administering to a subject in need thereof a pharmaceutically effective amount of the compound of any one of claims 1-32.

## Patentansprüche

1. Verbindung der Formel I:

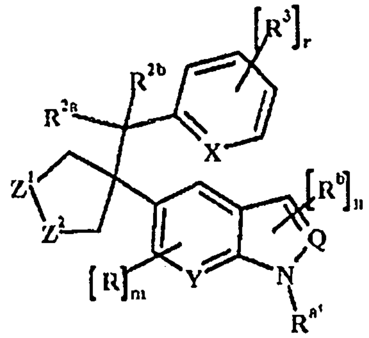

I

oder ein Enantiomer, Diastereomer oder pharmazeutisch akzeptables Salz davon, wobei:

entweder $Z^1$ oder $Z^2$ $N(R^a)$ ist und das andere $CH_2$ ist;
X CH oder N ist;
Y CH oder N ist;
m 0 oder 1 ist;
R Hydroxy, Halogen, $C_1$-$C_6$-Alkyl oder $C_1$-$C_6$-Alkoxy ist;
Q CH, $C(R^b)$ oder N ist;
$R^a$ H, $C_1$-$C_6$-Alkyl oder Benzyl ist;
$R^{a'}$ H, $C_1$-$C_6$-Alkyl, Cycloalkylalkyl, $-S(=O)_2R^c$, $-C(=O)N(R^c)_2$ oder $S(=O)_2N(R^c)_2$ ist;
n 0 oder 1 ist;
jeder $R^b$ unabhängig $R^{b'}$ oder $R^{b''}$ ist;
$R^{b'}$ Hydroxy, Halogen, $-C(=O)(R^c)$, $-C(=O)O(R^c)$, $-OC(=O)(R^c)$, $-N(R^c)_2$, $-C(=O)N(R^c)2$, $-NHC(=O)(R^c)$, -CN, $-S(=O)_2R^c$ oder $-S(=O)_2N(R^c)_2$ ist;
$R^{b''}$ $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl, Phenyl, Cycloalkyl, Cycloalkylalkyl, Heterocycloalkyl, Heterocycloalkylalkyl, gegebenenfalls substituiert mit einem oder mehreren $R^c$, ist;
jeder $R^c$ unabhängig $R^d$ oder $R^e$ ist;
$R^d$ H ist;
$R^e$ $C_1$-$C_6$-Alkyl, $C_1$-$C_6$-Halogenalkyl, Cycloalkyl, Phenyl, Heterocycloalkyl oder Heteroaryl, gegebenenfalls substituiert mit einem oder mehreren $R^{e'}$, ist;
jeder $R^{e'}$ unabhängig Hydroxy, Halogen, Amino, $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl oder -CN ist;
$R^{2a}$ und $R^{2b}$ jeweils unabhängig H, Hydroxy, $C_1$-$C_6$-Alkyl, $C_1$-$C_6$-Halogenalkyl oder $C_1$-$C_6$-Alkoxy sind;
r 0, 1 oder 2 ist und
$R^3$ Halogen, Hydroxy, $C_1$-$C_6$-Alkyl, $C_1$-$C_6$-Halogenalkyl oder $C_1$-$C_6$-Alkoxy ist.

2. Verbindung nach Anspruch 1, wobei $Z^1$ $N(R^a)$ ist und $Z^2$ $CH_2$ ist.

3. Verbindung nach Anspruch 1, wobei $Z^1$ $CH_2$ ist und $Z^2$ $N(R^a)$ ist.

4. Verbindung nach Anspruch 2, wobei $R^a$, $R^{2a}$ und $R^{2b}$ H sind, m 0 ist, r 0 ist, X CH ist, Y CH ist, $R^{a'}$ H ist und n 1 ist.

5. Verbindung nach Anspruch 3, wobei $R^a$, $R^{2a}$ und $R^{2b}$ H sind, m 0 ist, r 0 ist, X CH ist, Y CH ist, $R^{a'}$ H ist und n 1 ist.

**6.** Verbindung nach Anspruch 4, wobei Q $C(R^b)$ ist.

**7.** Verbindung nach Anspruch 5, wobei Q $C(R^b)$ ist.

**8.** Verbindung nach Anspruch 6, wobei $R^b$ $R^{b'}$ ist.

**9.** Verbindung nach Anspruch 6, wobei $R^b$ $R^{b''}$ ist.

**10.** Verbindung nach Anspruch 7, wobei $R^b$ $R^{b'}$ ist.

**11.** Verbindung nach Anspruch 7, wobei $R^b$ $R^{b''}$ ist.

**12.** Verbindung nach Anspruch 8, wobei $R^{b'}$ -CN oder Halogen ist.

**13.** Verbindung nach Anspruch 8, wobei $R^{b'}$ $-C(=O)N(R^{2c})_2$ oder $-NHC(=O)(R^{2c})$ ist.

**14.** Verbindung nach Anspruch 8, wobei $R^{b'}$ $-S(=O)_2R^{2c}$ oder $-S(=O)_2N(R^{2c})_2$ ist.

**15.** Verbindung nach Anspruch 8, wobei $R^{b'}$ $-C(=O)(R^{2c})$ oder $-C(=O)O(R^{2c})$ ist.

**16.** Verbindung nach Anspruch 9, wobei $R^{b''}$ $C_1$-$C_6$-Alkyl oder Cycloalkylalkyl ist.

**17.** Verbindung nach Anspruch 10, wobei $R^{b'}$ -CN oder Halogen ist.

**18.** Verbindung nach Anspruch 10, wobei $R^{b'}$ $-C(=O)N(R^{2c})_2$ oder $-NHC(=O)(R^{2c})$ ist.

**19.** Verbindung nach Anspruch 10, wobei $R^{b'}$ $-C(=O)(R^{2c})$ oder $-C(=O)O(R^{2c})$ ist.

**20.** Verbindung nach Anspruch 11, wobei $R^{b''}$ $C_1$-$C_6$-Alkyl oder Cycloalkylalkyl ist.

**21.** Verbindung der Formel II:

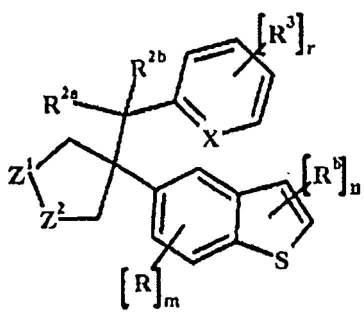

II

oder ein Enantiomer, Diastereomer oder pharmazeutisch akzeptables Salz davon, wobei:

entweder $Z^1$ oder $Z^2$ $N(R^a)$ ist und das andere $CH_2$ ist;
X CH oder N ist;
m 0 oder 1 ist;
R Hydroxy, Halogen, $C_1$-$C_6$-Alkyl oder $C_1$-$C_6$-Alkoxy ist;
$R^a$ H, $C_1$-$C_6$-Alkyl oder Benzyl ist;
n 0 oder 1 ist;
jeder $R^b$ unabhängig $R^{b'}$ oder $R^{b''}$ ist;
$R^{b'}$ Hydroxy, Halogen, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$ oder $-S(=O)_2N(R^c)_2$ ist;
$R^{b''}$ $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl, Phenyl, Cycloalkyl, Cycloalkylalkyl, Heterocycloalkyl, Heterocycloalkylalkyl, gegebenenfalls substituiert mit einem oder mehreren $R^c$, ist;
jeder $R^c$ unabhängig $R^d$ oder $R^e$ ist;

$R^d$ H ist;
$R^e$ $C_1$-$C_6$-Alkyl, $C_1$-$C_6$-Halogenalkyl, Cycloalkyl, Phenyl, Heterocycloalkyl oder Heteroaryl, gegebenenfalls substituiert mit einem oder mehreren $R^{e'}$, ist;
jeder $R^{e'}$ unabhängig Hydroxy, Halogen, Amino, $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl oder -CN ist;
$R^{2a}$ und $R^{2b}$ jeweils unabhängig H, Hydroxy, $C_1$-$C_6$-Alkyl, $C_1$-$C_6$-Halogenalkyl oder $C_1$-$C_6$-Alkoxy sind;
r 0, 1 oder 2 ist und
$R^3$ Halogen, Hydroxy, $C_1$-$C_6$-Alkyl, $C_1$-$C_6$-Halogenalkyl oder $C_1$-$C_6$-Alkoxy ist.

**22.** Verbindung nach Anspruch 21, wobei $R^{2a}$ und $R^{2b}$ H sind, m 0 ist und r 0 ist.

**23.** Verbindung nach Anspruch 22, wobei $Z^1$ $CH_2$ ist, $Z^2$ $N(R^a)$ ist, $R^a$ H ist und n 1 ist.

**24.** Verbindung der Formel III:

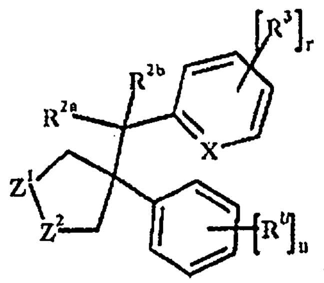

III

oder ein Enantiomer, Diastereomer oder pharmazeutisch akzeptables Salz davon, wobei:

entweder $Z^1$ oder $Z^2$ $N(R^a)$ ist und das andere $CH_2$ ist;
X CH oder N ist;
$R^a$ H, $C_1$-$C_6$-Alkyl oder Benzyl ist;
n 0, 1 oder 2 ist;
jeder $R^b$ unabhängig $R^{b'}$ oder $R^{b''}$ ist;
$R^{b'}$ unabhängig Hydroxy, Halogen, -C(=O)($R^c$), -C(=O)O($R^c$), -OC(=O)($R^c$), -N($R^c$)$_2$, -C(=O)N($R^c$)$_2$, -NHC(=O)($R^c$), -CN, -S(=O)$_2$$R^c$ oder -S(=O)$_2$N($R^c$)$_2$ ist;
$R^{b''}$ $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl, Phenyl, Cycloalkyl, Cycloalkylalkyl, Heterocycloalkyl, Heterocycloalkylalkyl, gegebenenfalls substituiert mit einem oder mehreren $R^c$, ist;
jeder $R^c$ unabhängig $R^d$ oder $R^e$ ist;
$R^d$ H ist;
$R^e$ $C_1$-$C_6$-Alkyl, $C_1$-$C_6$-Halogenalkyl, Cycloalkyl, Phenyl, Heterocycloalkyl oder Heteroaryl, gegebenenfalls substituiert mit einem oder mehreren $R^{e'}$, ist und
jeder $R^{e'}$ unabhängig Hydroxy, Halogen, Amino, $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl oder -CN ist;
$R^{2a}$ und $R^{2b}$ jeweils unabhängig H, Hydroxy, $C_1$-$C_6$-Alkyl, $C_1$-$C_6$-Halogenalkyl oder $C_1$-$C_6$-Alkoxy sind;
r 0, 1 oder 2 ist;
$R^3$ Halogen, Hydroxy, $C_1$-$C_6$-Alkyl, $C_1$-$C_6$-Halogenalkyl oder $C_1$-$C_6$-Alkoxy ist;
mit der Maßgabe, dass, wenn $Z^1$ $N(R^a)$ ist, $Z^2$ $CH_2$ ist, $R^a$ H ist und X CH ist, dann n nicht 0 ist; und mit der Maßgabe, dass, wenn $Z^1$ $N(R^a)$ ist, $Z^2$ $CH_2$ ist, $R^a$ Ethyl ist, X CH ist und entweder $R^{2a}$ oder $R^{2b}$ Hydroxy ist, dann n nicht 0 ist.

**25.** Verbindung nach Anspruch 24, wobei $R^{2a}$ und $R^{2b}$ H sind und r 0 ist.

**26.** Verbindung nach Anspruch 25, wobei $Z^1$ $CH_2$ ist, $Z^2$ $N(R^a)$ ist, $R^a$ H ist und n 1 ist.

**27.** Verbindung der Formel IV

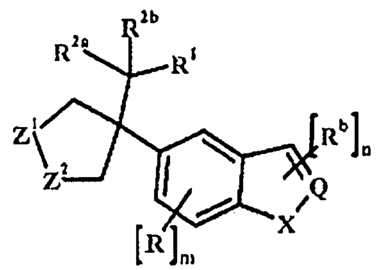

IV

oder ein Enantiomer, Diastereomer oder pharmazeutisch akzeptables Salz davon, wobei:

entweder $Z^1$ oder $Z^2$ $N(R^a)$ ist und das andere $CH_2$ ist;
X S oder $N(R^{a'})$ ist;
m 0 oder 1 ist;
R Hydroxy, Halogen, $C_1$-$C_6$-Alkyl oder $C_1$-$C_6$-Alkoxy ist;
Q CH, $C(R^b)$ oder N ist;
$R^a$ H, $C_1$-$C_6$-Alkyl oder Benzyl ist;
$R^{a'}$ H, $C_1$-$C_6$-Alkyl, Cycloalkylalkyl, $-S(=O)_2R^c$, $-C(=O)N(R^c)_2$ oder $S(=O)_2N(R^c)_2$ ist;
$R^1$ $R^{1a}$ oder $R^{1b}$ ist;
$R^{1a}$ H ist
$R^{1b}$ $C_1$-$C_6$-Alkyl, $C_2$-$C_6$-Alkenyl, $C_2$-$C_6$-Alkinyl, $C_1$-$C_6$-Alkoxy oder $C_1$-$C_6$-Halogenalkyl, gegebenenfalls substituiert mit einem oder mehreren $R^{1b'}$, ist;
jeder $R^{1b'}$ unabhängig Hydroxy, Halogen, Amino, $C_1$-$C_6$-Alkyl, $C_1$-$C_6$-Alkoxy, $C_1$-$C_6$-Halogenalkyl oder -CN ist;
n 0 oder 1 ist;
jeder $R^b$ unabhängig $R^{b'}$ oder $R^{b''}$ ist;
$R^{b'}$ Hydroxy, Halogen, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$ oder $-S(=O)_2N(R^c)_2$ ist;
$R^{b''}$ $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl, Phenyl, Cycloalkyl, Cycloalkylalkyl, Heterocycloalkyl, Heterocycloalkylalkyl, gegebenenfalls substituiert mit einem oder mehreren $R^c$, ist;
jeder $R^c$ unabhängig $R^d$ oder $R^e$ ist;
$R^d$ H ist;
$R^e$ $C_1$-$C_6$-Alkyl, $C_1$-$C_6$-Halogenalkyl, Cycloalkyl, Phenyl, Heterocycloalkyl oder Heteroaryl, gegebenenfalls substituiert mit einem oder mehreren $R^{e'}$, ist;
jeder $R^{e'}$ unabhängig Hydroxy, Halogen, Amino, $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl oder -CN ist und
$R^{2a}$ und $R^{2b}$ jeweils unabhängig H, Hydroxy, $C_1$-$C_6$-Alkyl, $C_1$-$C_6$-Halogenalkyl oder $C_1$-$C_6$-Alkoxy sind.

**28.** Verbindung nach Anspruch 27, wobei $R^{2a}$ und $R^{2b}$ H sind und m 0 ist.

**29.** Verbindung nach Anspruch 28, wobei $Z^1$ $CH_2$ ist, $Z^2$ $N(R^a)$ ist, $R^a$ H ist und n 1 ist.

**30.** Verbindung der Formel V:

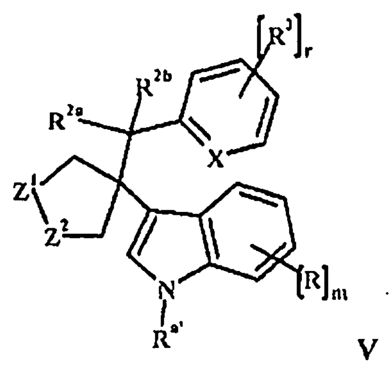

V

oder ein Enantiomer, Diastereomer oder pharmazeutisch akzeptables Salz davon, wobei:

entweder $Z^1$ oder $Z^2$ $N(R^a)$ ist und das andere $CH_2$ ist;
$R^a$ H, $C_1$-$C_6$-Alkyl oder Benzyl ist;
X CH oder N ist;
m 0 oder 1 ist;
R Hydroxy, Halogen, $C_1$-$C_6$-Alkyl oder $C_1$-$C_6$-Alkoxy ist;
$R^{a'}$ H, $C_1$-$C_6$-Alkyl, Cycloalkylalkyl, $-S(=O)_2R^c$, $-C(=O)N(R^c)_2$ oder $S(=O)_2N(R^c)_2$ ist;
jeder $R^c$ unabhängig $R^d$ oder $R^e$ ist;
$R^d$ H ist;
$R^e$ $C_1$-$C_6$-Alkyl, $C_1$-$C_6$-Halogenalkyl, Cycloalkyl, Phenyl, Heterocycloalkyl oder Heteroaryl, gegebenenfalls substituiert mit einem oder mehreren $R^{e'}$ ist;
jeder $R^{e'}$ unabhängig Hydroxy, Halogen, Amino, $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl oder -CN ist;
$R^{2a}$ und $R^{2b}$ jeweils unabhängig H, Hydroxy, $C_1$-$C_6$-Alkyl, $C_1$-$C_6$-Halogenalkyl oder $C_1$-$C_6$-Alkoxy sind;
r 0, 1 oder 2 ist und
$R^3$ Halogen, Hydroxy, $C_1$-$C_6$-Alkyl, $C_1$-$C_6$-Halogenalkyl oder $C_1$-$C_6$-Alkoxy ist.

**31.** Verbindung der Formel VI:

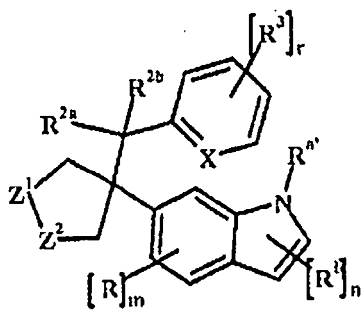

VI

oder ein Enantiomer, Diastereomer oder pharmazeutisch akzeptables Salz davon, wobei:

entweder $Z^1$ oder $Z^2$ $N(R^a)$ ist und das andere $CH_2$ ist;
X CH oder N ist;
m 0 oder 1 ist;
R Hydroxy, Halogen, $C_1$-$C_6$-Alkyl oder $C_1$-$C_6$-Alkoxy ist;
$R^a$ H, $C_1$-$C_6$-Alkyl oder Benzyl ist;
$R^{a'}$ H, $C_1$-$C_6$-Alkyl, Cycloalkylalkyl, $-S(=O)_2R^c$, $-C(=O)N(R^c)_2$ oder $S(=O)_2N(R^c)_2$ ist;
n 0 oder 1 ist;
jeder $R^b$ unabhängig $R^{b'}$ oder $R^{b''}$ ist;
$R^{b'}$ Hydroxy, Halogen, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$ oder $-S(=O)_2N(R^c)_2$ ist;
$R^{b''}$ $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl, Phenyl, Cycloalkyl, Cycloalkylalkyl, Heterocycloalkyl, Heterocycloalkylalkyl, gegebenenfalls substituiert mit einem oder mehreren $R^c$, ist;
jeder $R^c$ unabhängig $R^d$ oder $R^e$ ist;
$R^d$ H ist;
$R^e$ $C_1$-$C_6$-Alkyl, $C_1$-$C_6$-Halogenalkyl, Cycloalkyl, Phenyl, Heterocycloalkyl oder Heteroaryl, gegebenenfalls substituiert mit einem oder mehreren $R^{e'}$, ist;
jeder $R^{e'}$ unabhängig Hydroxy, Halogen, Amino, $C_1$-$C_6$-Alkyl, $C_2$-$C_6$-Alkenyl, $C_2$-$C_6$-Alkinyl, $C_1$-$C_6$-Alkoxy, $C_1$-$C_6$-Halogenalkyl oder -CN ist;
$R^{2a}$ und $R^{2b}$ jeweils unabhängig H, Hydroxy, $C_1$-$C_6$-Alkyl, $C_1$-$C_6$-Halogenalkyl oder $C_1$-$C_6$-Alkoxy sind;
r 0, 1 oder 2 ist und
$R^3$ Halogen, Hydroxy, $C_1$-$C_6$-Alkyl, $C_1$-$C_6$-Halogenalkyl oder $C_1$-$C_6$-Alkoxy ist.

**32.** Verbindung, ausgewählt aus der Gruppe, bestehend aus:

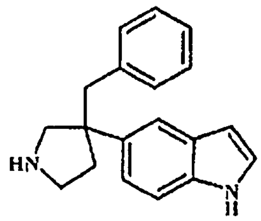

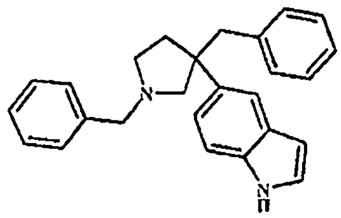

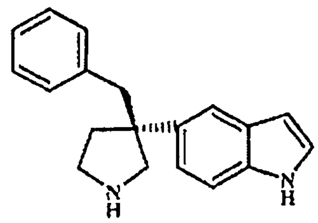

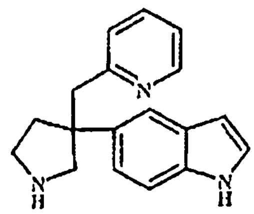

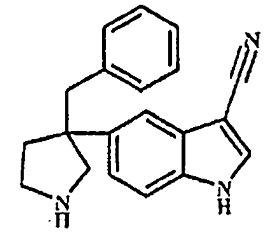

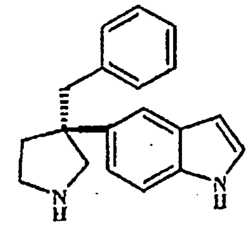

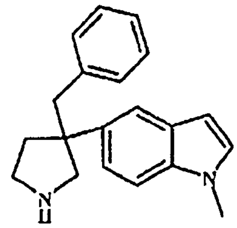
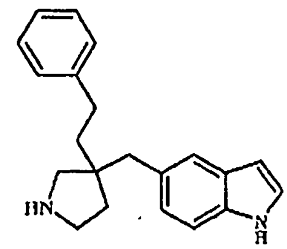
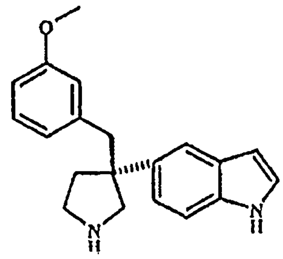
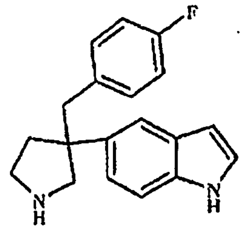
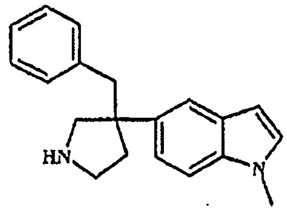
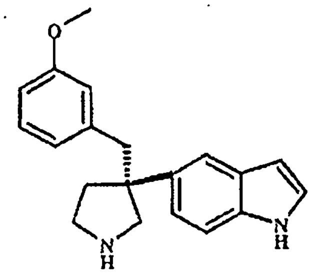
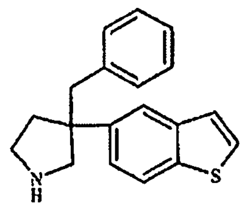
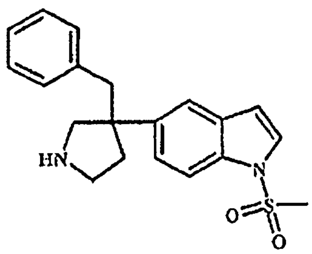
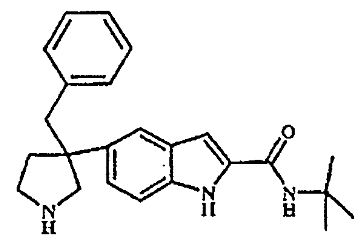
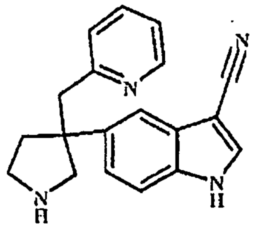
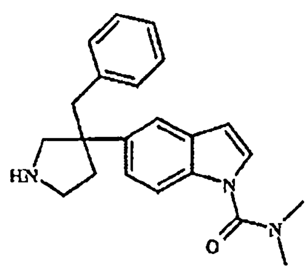
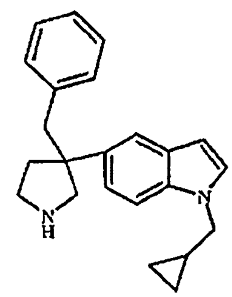
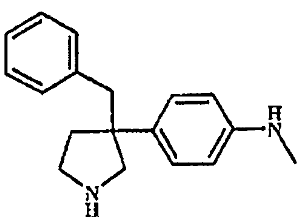
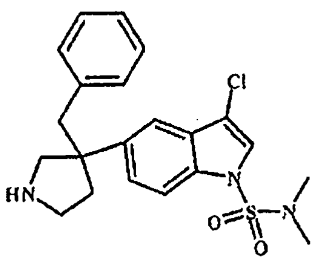
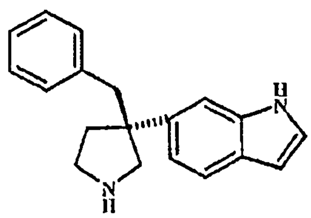
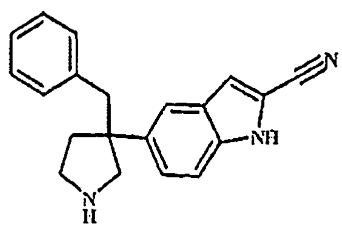
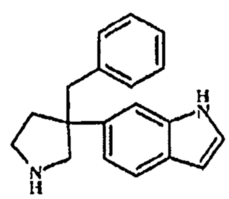
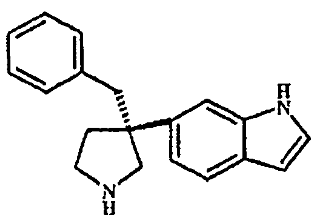
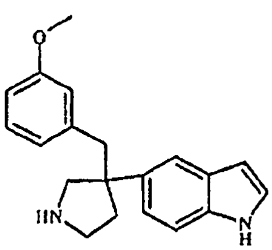

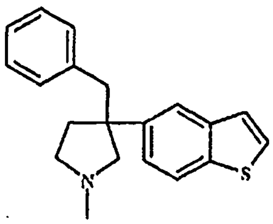

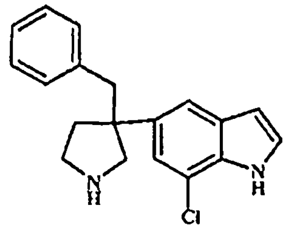

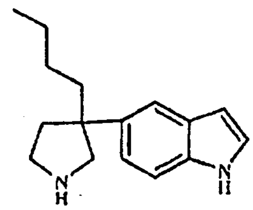

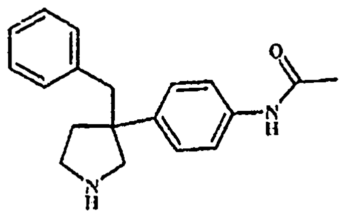

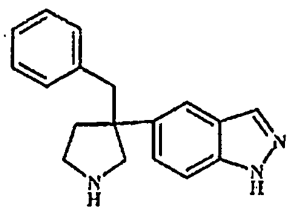

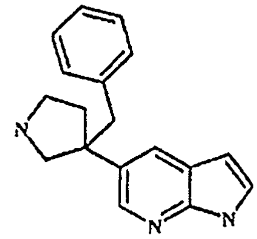

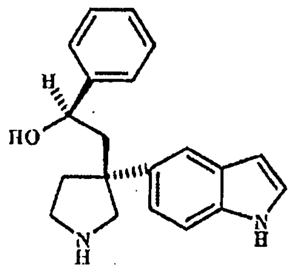

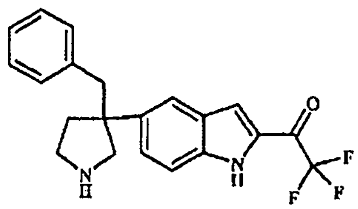

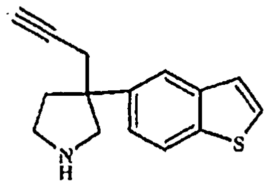

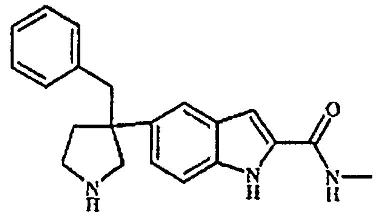

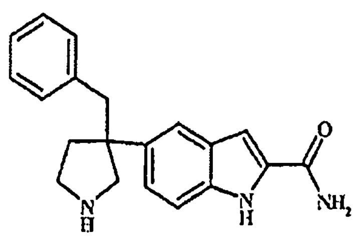

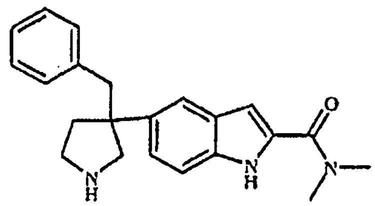

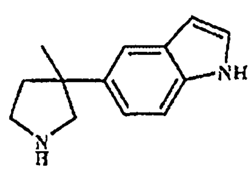

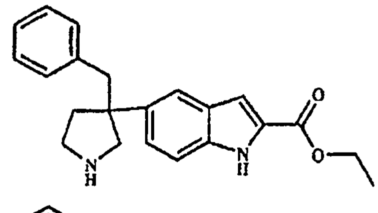

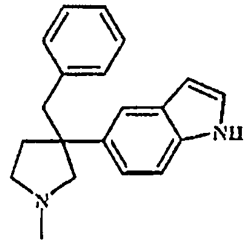

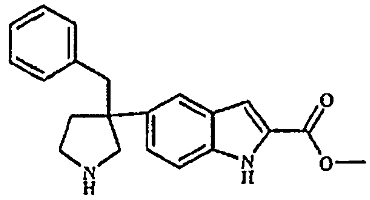

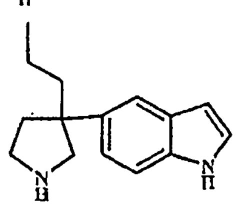

**33.** Pharmazeutische Zusammensetzung, umfassend die Verbindung nach einem der Ansprüche 1-32 und einen pharmazeutisch akzeptablen Träger.

**34.** Verbindung nach einem der Ansprüche 1 - 32 zur Verwendung in einem Verfahren der Behandlung von Angst, Depression oder beidem, umfassend das Verabreichen einer pharmazeutisch wirksamen Menge der Verbindung nach einem der Ansprüche 1 - 32 an einen Patienten, der derartigem bedarf.

## Revendications

**1.** Composé de formule I:

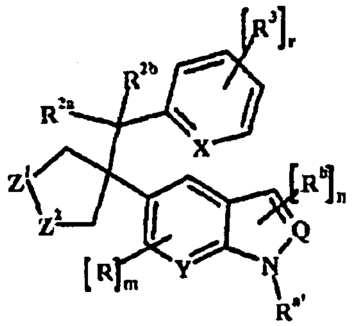

I

ou un de ses énantiomères, diastéréoisomères, ou sels pharmaceutiquement acceptables,
où
l'un des $Z^1$ et $Z^2$ est $N(R^a)$ et l'autre est $CH_2$;

X est CH ou N;
Y est CH ou N;
m est 0 ou 1;
R est hydroxy, halogène, alkyle en $C_1$-$C_6$ ou alcoxy en $C_1$-$C_6$;
Q est CH, $C(R^b)$ ou N;
$R^a$ est H, alkyle en $C_1$-$C_6$ ou benzyle;
$R^{a'}$ est H, alkyle en $C_1$-$C_6$, cycloalkylalkyle, $-S(=O)_2R^c$, $-C(=O)N(R^c)_2$, ou $S(=O)_2N(R^c)_2$;
n est 0 ou 1;

chaque $R^b$ est indépendamment $R^{b'}$ ou $R^{b''}$;

$R^{b'}$ est hydroxy, halogène, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$, ou $S(=O)_2N(R^c)_2$;
$R^{b''}$ est alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$, phényle, cycloalkyle, cycloalkylalkyle, hétérocycloalkyle, hétérocycloalkylalkyle, éventuellement substitués par un ou plusieurs $R^c$;

chaque $R^c$ est indépendamment $R^d$ ou $R^e$;

$R^d$ est H;
$R^e$ est alkyle en $C_1$-$C_6$, halogénoalkyle en $C_1$-$C_6$, cycloalkyle, phényle, hétérocycloalkyle, ou hétéroaryle, éventuellement substitués par un ou plusieurs $R^{c'}$;
chaque $R^{c'}$ est indépendamment hydroxy, halogène, amino, alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$ ou -CN;
$R^{2a}$ et $R^{2b}$ sont chacun indépendamment H, hydroxy, alkyle en $C_1$-$C_6$, halogénoalkyle en $C_1$-$C_6$, ou alcoxy en $C_1$-$C_6$;
r est 0, 1 ou 2; et
$R^3$ est halogène, hydroxy, alkyle en $C_1$-$C_6$, halogénoalkyle en $C_1$-$C_6$ ou alcoxy en $C_1$-$C_6$.

**2.** Composé selon la revendication 1, dans lequel $Z^1$ est $N(R^a)$ et $Z^2$ est $CH_2$.

**3.** Composé selon la revendication 1, dans lequel $Z^1$ est CH et $Z^2$ est $N(R^a)$.

**4.** Composé selon la revendication 2, dans lequel $R^a$, $R^{2a}$ et $R^{2b}$ sont H, m est 0, r est 0, X est CH, Y est CH, $R^{a'}$ est H et n est 1.

**5.** Composé selon la revendication 3, dans lequel $R^a$, $R^{2a}$ et $R^{2b}$ sont H, m est 0, r est 0, X est CH, Y est CH, $R^{a'}$ est H et n est 1.

**6.** Composé selon la revendication 4, dans lequel Q est $C(R^b)$.

**7.** Composé selon la revendication 5, dans lequel Q est $C(R^b)$.

**8.** Composé selon la revendication 6, dans lequel $R^b$ est $R^{b'}$.

**9.** Composé selon la revendication 6, dans lequel $R^b$ est $R^{b''}$.

**10.** Composé selon la revendication 7, dans lequel $R^b$ est $R^{b'}$.

**11.** Composé selon la revendication 7, dans lequel $R^b$ est $R^{b''}$.

**12.** Composé selon la revendication 8, dans lequel $R^{b'}$ est -CN ou halogène.

**13.** Composé selon la revendication 8, dans lequel $R^{b'}$est $-C(=O)N(R^{2c})_2$ ou $-NHC(=O)(R^{2c})$.

**14.** Composé selon la revendication 8, dans lequel $R^{b'}$ est $-S(=O)_2R^{2c}$ ou $S(=O)_2N(R^{2c})_2$.

**15.** Composé selon la revendication 8, dans lequel $R^{b'}$ est $-C(=O)(R^{2c})$ ou $-C(=O)O(R^{2c})$.

**16.** Composé selon la revendication 9, dans lequel $R^{b''}$ est alkyle en $C_1$-$C_6$ ou cycloalkylalkyle.

**17.** Composé selon la revendication 10, dans lequel $R^{b'}$ est -CN ou halogène.

**18.** Composé selon la revendication 10, dans lequel $R^{b'}$ est- $C(=O)N(R^{2c})_2$ ou $-NHC(=O)(R^{2c})$.

**19.** Composé selon la revendication 10, dans lequel $R^{b'}$ est $-C(=O)(R^{2c})$ ou $-C(=O)O(R^{2c})$.

**20.** Composé selon la revendication 11, dans lequel $R^{b''}$ est alkyle en Ci-$C_6$ ou cycloalkylalkyle.

**21.** Composé de formule II:

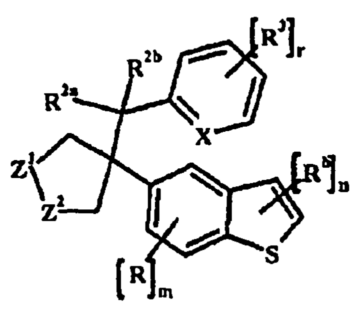

II

ou un de ses énantiomères, diastéréoisomères, ou sels pharmaceutiquement acceptables,
où
l'un des $Z^1$ et $Z^2$ est $N(R^a)$ et l'autre est $CH_2$;

X est CH ou N;
m est 0 ou 1;
R est hydroxy, halogène, alkyle en $C_1$-$C_6$ ou alcoxy en $C_1$-$C_6$;
$R^a$ est H, alkyle en $C_1$-$C_6$ ou benzyle;
n est 0 ou 1;

chaque $R^b$ est indépendamment $R^{b'}$ ou $R^{b''}$;

$R^{b'}$ est hydroxy, halogène, -C(=O)($R^c$), -C(=O)O($R^c$), -OC(=O)($R^c$), -N($R^c$)$_2$, -C(=O)N($R^c$)$_2$, -NHC(=O)($R^c$), -CN, -S(=O)$_2R^c$, ou S(=O)$_2$N($R^c$)$_2$;
$R^{b''}$ est alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$, phényle, cycloalkyle, cycloalkylalkyle, hétérocycloalkyle, hétérocycloalkylalkyle, éventuellement substitués par un ou plusieurs $R^c$;

chaque $R^c$ est indépendamment $R^d$ ou $R^e$;

$R^d$ est H;
$R^e$ est alkyle en $C_1$-$C_6$, halogénoalkyle en $C_1$-$C_6$, cycloalkyle, phényle, hétérocycloalkyle, ou hétéroaryle, éventuellement substitués par un ou plusieurs $R^{e'}$;
chaque $R^{e'}$ est indépendamment hydroxy, halogène, amino, alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$ ou -CN;
$R^{2a}$ et $R^{2b}$ sont chacun indépendamment H, hydroxy, alkyle en $C_1$-$C_6$, halogénoalkyle en $C_1$-$C_6$, ou alcoxy en $C_1$-$C_6$;
r est 0, 1 ou 2; et
$R^3$ est halogène, hydroxy, alkyle en $C_1$-$C_6$, halogénoalkyle en $C_1$-$C_6$ ou alcoxy en $C_1$-$C_6$.

**22.** Composé selon la revendication 21, dans lequel $R^{2a}$ et $R^{2b}$ sont H, m est 0 et r est 0.

**23.** Composé selon la revendication 22, dans lequel $Z^1$ est $CH_2$, $Z^2$ est N($R^a$), $R^a$ est H et n est 1.

**24.** Composé de formule III:

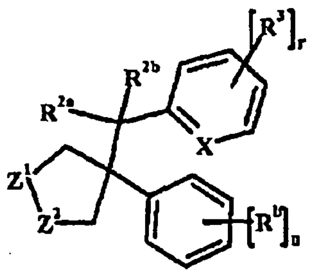

III

ou un de ses énantiomères, diastéréoisomères, ou sels pharmaceutiquement acceptables,
où
l'un des $Z^1$ et $Z^2$ est N($R^a$) et l'autre est $CH_2$;

X est CH ou N;
$R^a$ est H, alkyle en $C_1$-$C_6$ ou benzyle;
n est 0, 1 ou 2

chaque $R^b$ est indépendamment $R^{b'}$ ou $R^{b''}$;

$R^{b'}$ est hydroxy, halogène, -C(=O)($R^c$), -C(=O)O($R^c$), -OC(=O)($R^c$), -N($R^c$)$_2$, -C(=O)N($R^c$)$_2$, -NHC(=O)($R^c$), -CN, -S(=O)$_2R^c$, ou S(=O)$_2$N($R^c$)$_2$;
$R^{b''}$ est alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$, phényle, cycloalkyle, cycloalkylalkyle, hétérocycloalkyle, hétérocycloalkylalkyle, éventuellement substitués par

un ou plusieurs $R^c$;

chaque $R^c$ est indépendamment $R^d$ ou $R^e$;

$R^d$ est H;
$R^e$ est alkyle en $C_1$-$C_6$, halogénoalkyle en $C_1$-$C_6$, cycloalkyle, phényle, hétérocycloalkyle, ou hétéroaryle, éventuellement substitués par un ou plusieurs $R^{e'}$; et
chaque $R^{e'}$ est indépendamment hydroxy, halogène, amino, alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$ ou -CN;
$R^{2a}$ et $R^{2b}$ sont chacun indépendamment H, hydroxy, alkyle en $C_1$-$C_6$, halogénoalkyle en $C_1$-$C_6$, ou alcoxy en $C_1$-$C_6$;
r est 0, 1 ou 2;
$R^3$ est halogène, hydroxy, alkyle en $C_1$-$C_6$, halogénoalkyle en $C_1$-$C_6$ ou alcoxy en $C_1$-$C_6$;

à condition que n ne soit pas 0 lorsque $Z^1$ est $N(R^a)$, $Z^2$ est $CH_2$, $R^a$ est H et X est CH; et à condition que n ne soit pas 0 lorsque $Z^1$ est $N(R^a)$, $Z^2$ est $CH_2$, $R^a$ est éthyle, X est CH et l'un ou l'autre de $R^{2a}$ et $R^{2b}$ est hydroxy.

**25.** Composé selon la revendication 24, dans lequel $R^{2a}$ et $R^{2b}$ sont H et r est 0.

**26.** Composé selon la revendication 25, dans lequel $Z^1$ est $CH_2$, $Z^2$ est $N(R^a)$, $R^a$ est H et n est 1.

**27.** Composé de formule IV:

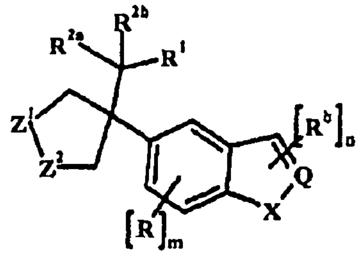

IV

ou un de ses énantiomères, diastéréoisomères, ou sels pharmaceutiquement acceptables,
où
l'un des $Z^1$ et $Z^2$ est $N(R^a)$ et l'autre est $CH_2$;

X est S ou $N(R^{a'})$;
m est 0 ou 1;
R est hydroxy, halogène, alkyle en $C_1$-$C_6$ ou alcoxy en $C_1$-$C_6$;
Q est CH, $C(R^b)$ ou N;
$R^a$ est H, alkyle en $C_1$-$C_6$ ou benzyle;
$R^{a'}$ est H, alkyle en $C_1$-$C_6$, cycloalkylalkyle, $-S(=O)_2R^c$, $-C(=O)N(R^c)_2$, ou $S(=O)_2N(R^c)_2$;
$R^1$ est $R^{1a}$ ou $R^{1b}$;
$R^{1a}$ est H;
$R^{1b}$ est alkyle en $C_1$-$C_6$, alcényle en $C_2$-$C_6$, alcynyle en $C_2$-$C_6$, alcoxy en $C_1$-$C_6$, ou halogénoalkyle en $C_1$-$C_6$, éventuellement substitués par un ou plusieurs $R^{1b'}$;
chaque $R^{1b'}$ est indépendamment hydroxy, halogène, amino, alkyle en $C_1$-$C_6$, alcoxy en $C_1$-$C_6$, halogénoalkyle en $C_1$-$C_6$ ou -CN;
n est 0 ou 1;
chaque $R^b$ est indépendamment $R^{b'}$ ou $R^{b''}$;
$R^{b'}$ est hydroxy, halogène, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$, ou $S(=O)_2N(R^c)_2$;
$R^{b''}$ est alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$, phényle, cycloalkyle, cycloalkylalkyle, hétérocycloalkyle, hétérocycloalkylalkyle, éventuellement substitués par un ou plusieurs $R^c$;

chaque $R^c$ est indépendamment $R^d$ ou $R^e$;

$R^d$ est H;
$R^e$ est alkyle en $C_1$-$C_6$, halogénoalkyle en $C_1$-$C_6$, cycloalkyle, phényle, hétérocycloalkyle, ou hétéroaryle, éventuellement substitués par un ou plusieurs $R^{e'}$;
chaque $R^{e'}$ est indépendamment hydroxy, halogène, amino, alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$ ou -CN; et
$R^{2a}$ et $R^{2b}$ sont chacun indépendamment H, hydroxy, alkyle en $C_1$-$C_6$, halogénoalkyle en $C_1$-$C_6$, ou alcoxy en $C_1$-$C_6$.

**28.** Composé selon la revendication 27, dans lequel $R^{2a}$ et $R^{2b}$ sont H et m est 0.

**29.** Composé selon la revendication 28, dans lequel $Z^1$ est $CH_2$, $Z^2$ est $N(R^a)$, $R^a$ est H et n est 1.

**30.** Composé de formule V:

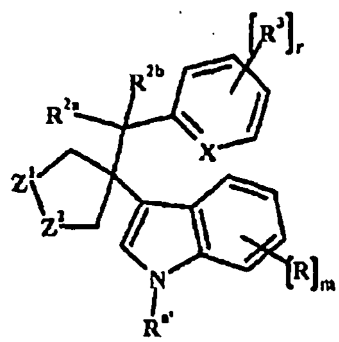

V

ou un de ses énantiomères, diastéréoisomères, ou sels pharmaceutiquement acceptables,
où
l'un des $Z^1$ et $Z^2$ est $N(R^a)$ et l'autre est $CH_2$;

$R^a$ est H, alkyle en $C_1$-$C_6$ ou benzyle;
X est CH ou N;
m est 0 ou 1;
R est hydroxy, halogène, alkyle en $C_1$-$C_6$ ou alcoxy en $C_1$-$C_6$;
$R^{a'}$ est H, alkyle en $C_1$-$C_6$, cycloalkylalkyle, $-S(=O)_2R^c$, $-C(=O)N(R^c)_2$, ou $S(=O)_2N(R^c)_2$;

chaque $R^c$ est indépendamment $R^d$ ou $R^e$;

$R^d$ est H;
$R^e$ est alkyle en $C_1$-$C_6$, halogénoalkyle en $C_1$-$C_6$, cycloalkyle, phényle, hétérocycloalkyle, ou hétéroaryle, éventuellement substitués par un ou plusieurs $R^{e'}$;
chaque $R^{e'}$ est indépendamment hydroxy, halogène, amino, alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$ ou -CN;
$R^{2a}$ et $R^{2b}$ sont chacun indépendamment H, hydroxy, alkyle en $C_1$-$C_6$, halogénoalkyle en C1-$C_6$, ou alcoxy en $C_1$-$C_6$;
r est 0, 1 ou 2; et
$R^3$ est halogène, hydroxy, alkyle en $C_1$-$C_6$, halogénoalkyle en $C_1$-$C_6$ ou alcoxy en $C_1$-$C_6$.

**31.** Composé de formule VI:

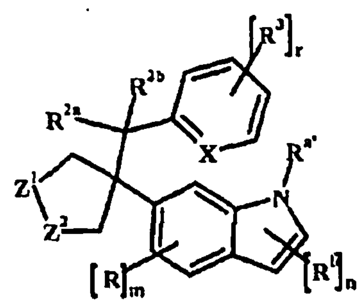

VI

ou un de ses énantiomères, diastéréoisomères, ou sels pharmaceutiquement acceptables, où
l'un des $Z^1$ et $Z^2$ est $N(R^a)$ et l'autre est $CH_2$;

X est CH ou N;
m est 0 ou 1;
R est hydroxy, halogène, alkyle en $C_1$-$C_6$ ou alcoxy en $C_1$-$C_6$;
$R^a$ est H, alkyle en $C_1$-$C_6$ ou benzyle;
$R^{a'}$ est H, alkyle en $C_1$-$C_6$, cycloalkylalkyle, $-S(=O)_2R^c$, $-C(=O)N(R^c)_2$, ou $S(=O)_2N(R^c)_2$;
n est 0 ou 1;

chaque $R^b$ est indépendamment $R^{b'}$ ou $R^{b''}$;

$R^{b'}$ est hydroxy, halogène, $-C(=O)(R^c)$, $-C(=O)O(R^c)$, $-OC(=O)(R^c)$, $-N(R^c)_2$, $-C(=O)N(R^c)_2$, $-NHC(=O)(R^c)$, -CN, $-S(=O)_2R^c$, ou $S(=O)_2N(R^c)_2$;
$R^{b''}$ est alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$, phényle, cycloalkyle, cycloalkylalkyle, hétérocycloalkyle, hétérocycloalkylalkyle, éventuellement substitués par un ou plusieurs $R^c$;

chaque $R^c$ est indépendamment $R^d$ ou $R^e$;

$R^d$ est H;
$R^e$ est alkyle en $C_1$-$C_6$, halogénoalkyle en $C_1$-$C_6$, cycloalkyle, phényle, hétérocycloalkyle, ou hétéroaryle, éventuellement substitués par un ou plusieurs $R^{e'}$;
chaque $R^{e'}$ est indépendamment hydroxy, halogène, amino, alkyle en $C_1$-$C_6$, alcényle en $C_2$-$C_6$, alcynyle en $C_2$-$C_6$, alcoxy en $C_1$-$C_6$, halogénoalkyle en $C_1$-$C_6$ ou -CN;
$R^{2a}$ et $R^{2b}$ sont chacun indépendamment H, hydroxy, alkyle en $C_1$-$C_6$, halogénoalkyle en $C_1$-$C_6$, ou alcoxy en $C_1$-$C_6$;
r est 0, 1 ou 2; et
$R^3$ est halogène, hydroxy, alkyle en $C_1$-$C_6$, halogénoalkyle en $C_1$-$C_6$ ou alcoxy en $C_1$-$C_6$.

**32.** Composé choisi dans le groupe constitué par:

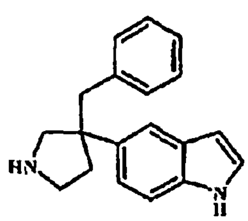

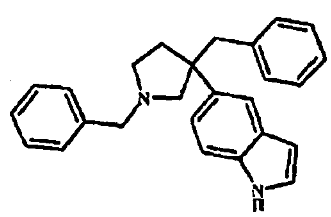

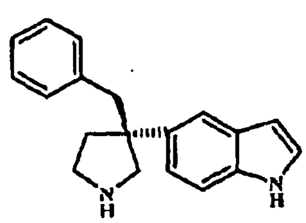

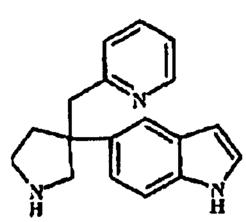

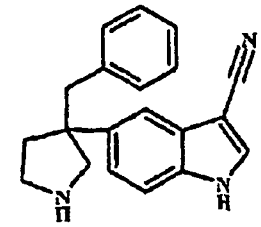

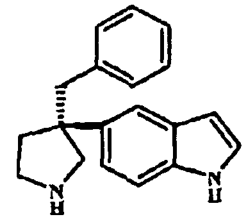

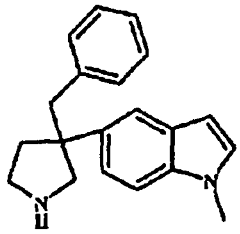

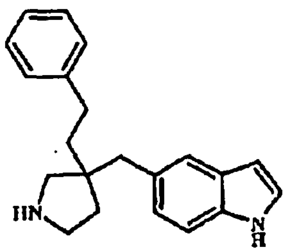

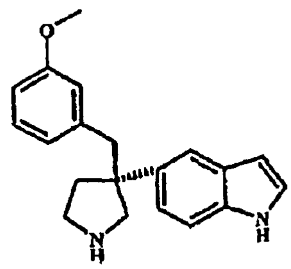

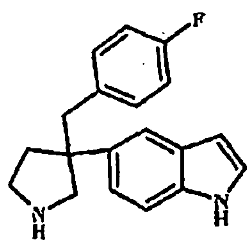

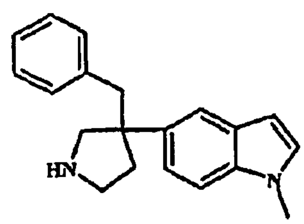

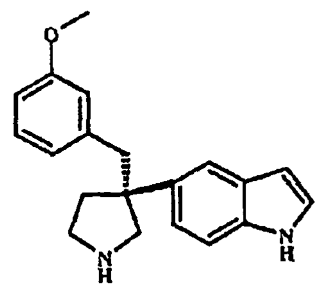

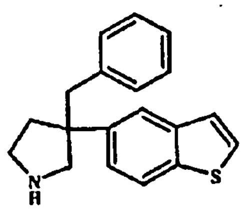

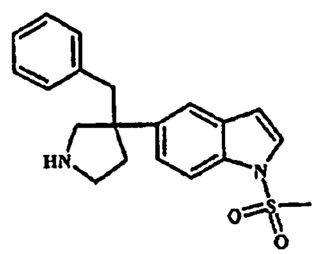

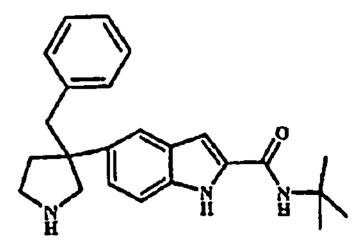

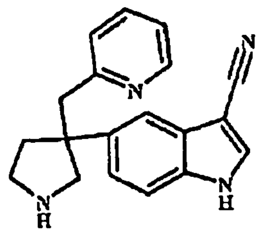

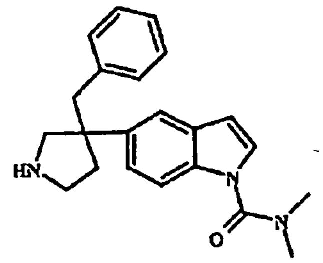

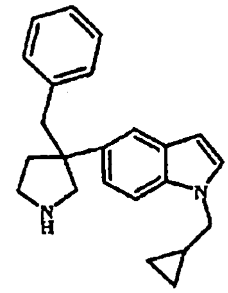

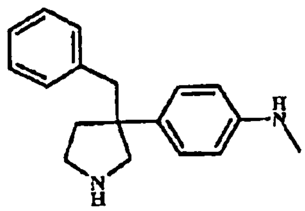

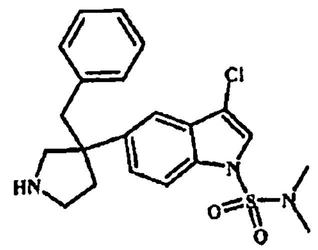

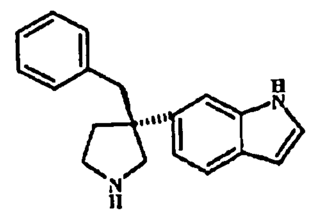

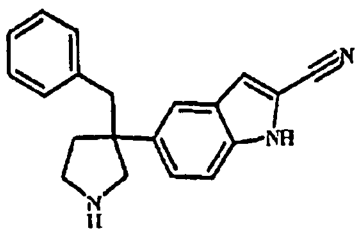

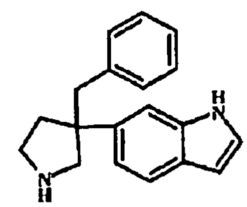

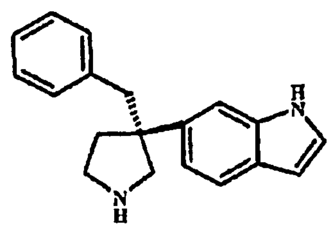

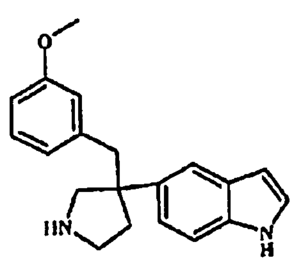

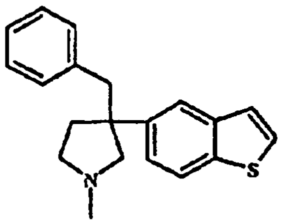
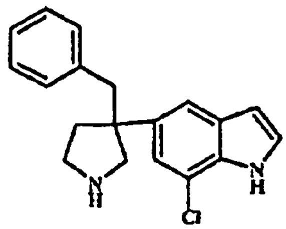
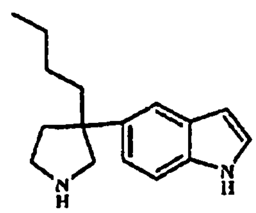

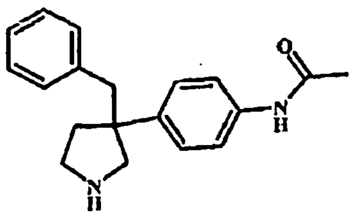
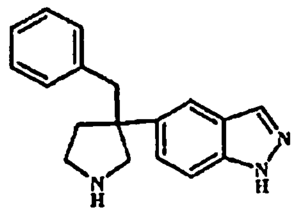
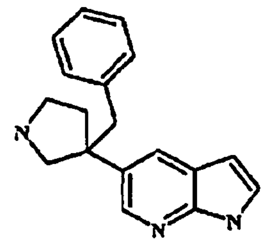

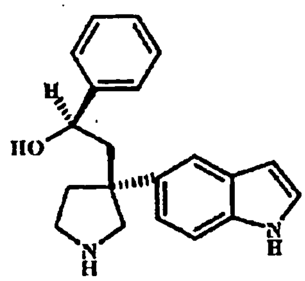
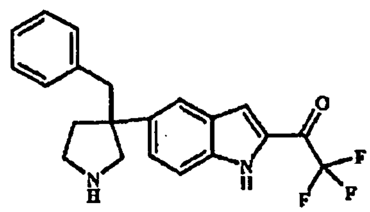

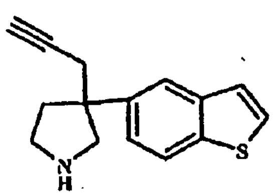
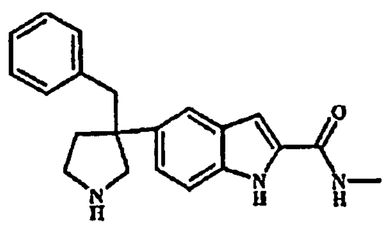

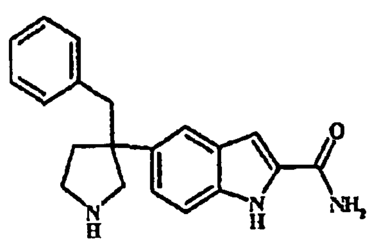
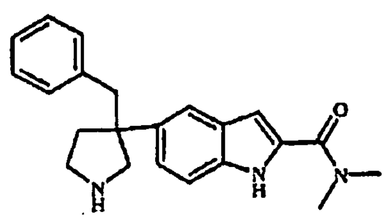

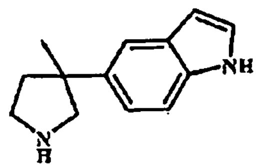

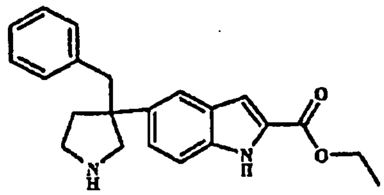

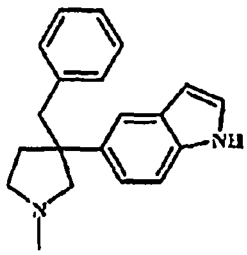

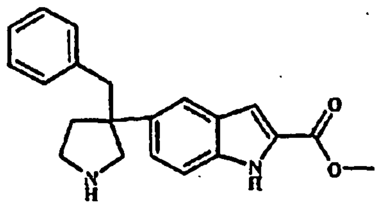

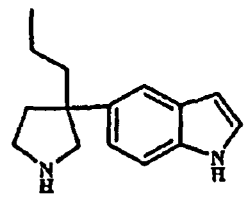

**33.** Composition pharmaceutique comprenant le composé selon l'une quelconque des revendications 1-32 et un support pharmaceutiquement acceptable.

**34.** Composé selon l'une quelconque des revendications 1-32, à utiliser dans un procédé de traitement de l'anxiété, de la dépression, ou des deux, comprenant l'administration à un sujet en ayant besoin d'une quantité pharmaceutiquement efficace du composé selon l'une quelconque des revendications 1-32.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006121218 A
- EP 1304324 A
- WO 2004078757 A2

### Non-patent literature cited in the description


- **Charney et al.** *J. Clin. Psychiatry,* 1998, vol. 59, 1-14
- **Delgado et al.** *J. Clin. Psychiatry,* 2000, vol. 67, 7-11
- **Resser et al.** *Depress. Anxiety,* 2000, vol. 12 (1), 2-19
- **Hirschfeld et al.** *J. Clin. Psychiatry,* 2000, vol. 61, 4-6
- **Mas et al.** *Harv. Rev. Psychiatry,* 1999, vol. 7, 69-84
- **Scates et al.** *Ann. Pharmacother.,* 2000, vol. 34, 1302-1312
- **Tatsumi et al.** *Eur-. J. Pharmacol.,* 1997, vol. 340, 249-258
- **Thase et al.** *Br. J. Psychiatry,* 2001, vol. 178, 234, 241
- **Tran et al.** *J. Clin. Psychopharmacology,* 2003, vol. 23, 78-86
- **Mallinckrodt et al.** *J. Clin. Psychiatry,* 2003, vol. 5 (1), 19-28
- **Bymaster et al.** *Expert Opin. Investig. Drugs,* 2003, vol. 12 (4), 531-543
- **Beer et al.** *J. Clinical Pharmacology,* 2004, vol. 44, 1360-1367
- **Skolnick et al.** *Eur J Pharmacol.,* 14 February 2003, vol. 461 (2-3), 99-104
- **Sommer et al.** *Molecular Neurobiology,* 2004, vol. 30 (2), 117-125
- **Iyengar et al.** *J. Pharm. Exper. Ther-apeutics,* 2004, vol. 311, 576-584
- Dorland's Illustrated Medical Dictionary. W. B. Saunders Company
- Fieser and Fieser's Reagents for Organic Synthesis. Wiley & Sons, 1991, vol. 1-15
- Rodd's Chemistry of Carbon Compounds. Elsevier Science Publishers, 1989, vol. 1-5
- Organic Reactions. Wiley & Sons, 1991, 1-40
- Remington: The Science and Practice of Pharmacy. Mack Publishing Company, 1995
- **Tatsumi et al.** *Eur. J. Pharmacol.,* 1997, vol. 30, 249-258
- **Gogas, K.R. et al.** *Analgesia,* 1997, vol. 3, 1-8